# EUROPEAN PATENT SPECIFICATION

(11) **EP 2 334 707 B1**
(45) Date of publication and mention of the grant of the patent: **01.02.2012**
(21) Application number: 09789248.3
(22) Date of filing: 03.09.2009
(51) Int. Cl.: C08F 36/22, C08L 47/00, C09J 147/00

(54) **ADHESIVE COMPOSITIONS COMPRISING A POLYFARNESENE**
KLEBSTOFFZUSAMMENSETZUNGEN, DIE EIN POLYFARNESEN UMFASSEN
COMPOSITIONS ADHÉSIVES COMPRENANT UN POLYFARNESÈNE

(30) Priority: 26.06.2009 US 220591 P; 04.09.2008 US 94059 P
(43) Date of publication of application: 22.06.2011
(73) Proprietor: Amyris, Inc., Emeryville CA 94608 (US)
(72) Inventor: MCPHEE, Derek, James, Fairfield CA 94534 (US); GRAHAM, Matthew, J., Akron, OH 44305 (US)
(74) Representative: Ritter, Thomas Kurt
(86) International application number: PCT/US2009/004958
(87) International publication number: WO 2010/027463

(56) References cited:
- JP-A- 11 335 327
- JP-A- 2008 201 994
- US-A- 5 668 207
- NEWMARK RICHARD A ET AL: "C-NMR SPECTRA OF cis-POLYMERCENE AND cis-POLYFARNESENE." JOURNAL OF POLYMER SCIENCE, PART A: POLYMER CHEMISTRY 1988 JAN, vol. 26, no. 1, January 1988 (1988-01), pages 71-77, XP002557250

## Description

This invention provides an adhesive composition comprising a polyfarnesene and a tackifier. The polyfarnesene can be a farnesene homopolymer derived from a farnesene or a farnesene interpolymer derived from a farnesene and at least a vinyl monomer. The adhesive composition disclosed herein can be used as a hot melt adhesive or a pressure sensitive adhesive.

An adhesive is a substance capable of holding materials (e.g.; adherends or substrates) together by surface attachment. Pressure sensitive adhesives (PSAs) generally are adhesive materials which bond to adherends when a required pressure is applied to effect an adhesion to the adherends. PSAs can be permanent or removable. Removable PSAs have been widely used in re-positionable applications, such as Post-it® notes. Pressure sensitive adhesives are generally based on a polymer, a tackifier and an oil. Some common PSAs are based on polymers such as natural rubbers, synthetic rubbers (e.g., styrene-butadiene rubber and styrene-isoprene-styrene copolymer), polyacrylates, polymethacrylates, and poly-alpha-olefins.

Hot-melt adhesives at ambient temperature are generally solid materials that can be heated to a melt to hold adherends or substrates together upon cooling and solidifying. In some applications, the bonded substrates can be detached by remelting the hot melt adhesive if the substrates can withstand the heat. The hot melt adhesives arc generally used in paper products, packaging materials, laminated wood panels, kitchen countertops, vehicles, tapes, labels, and a variety of disposable goods such as disposable diapers, hospital pads, feminine sanitary napkins, and surgical drapes. Generally, these hot melt adhesives are based on a polymer, tackifier, and a wax. Some common hot melt adhesives are based on semi-crystalline polymers such as ethylene homopolymers, ethylene copolymers and styrene block copolymers (e.g., styrene-isoprene-styrene copolymer or styrene-butadiene-styrene copolymer). One desirable property of hot melt adhesives is the absence of a liquid carrier, thereby eliminating a potential costly and hazardous process associated with solvent removal.

Polymers derived from terpenes or isoprenoid compounds are useful polymeric materials. For example, polyisoprene, polypinene and polylimonene have been used in various applications such as in the manufacture of paper coatings, rubber compounds, and other industrial products. However, adhesive compositions comprising polymers derived from terpenes or isoprenoid compounds are rare, even rarer are polymers derived from isoprenoid compounds having at least 15 carbon atoms.

Despite the availability of a variety of hot melt adhesives and pressure sensitive adhesives, there are still needs for new adhesive compositions having unique adhesive properties to meet new requirements. Further, there is a need for environmentally friendly or renewable polymers, for instance, polymers derived from isoprenoid compounds that can be obtained from natural sources.

The aforementioned needs are met by various aspects disclosed herein. In one aspect, provided herein is a composition comprising a polyfarnesene and a tackifier. In some embodiments, the composition disclosed herein is a hot melt adhesive composition. In other embodiments, the composition disclosed herein is a pressure sensitive adhesive composition.

In certain embodiments, the polyfarnesene has formula (X'): 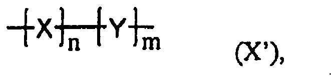 wherein n is an integer from 1 to 100,000; m is an integer from 0 to 100,000; X is derived from a farnesene; and Y is derived from a vinyl monomer, with the proviso that when m is 1 or greater, the mole percent ratio of X to Y is from 1:4 to 100:1, and (ii) when m is o, the polyfarnesene is a farnesene homopolymer.

In some embodiments, X of the polyfarnesene disclosed herein has one or more of formulae (I')-(VIII') and stereoisomers thereof: 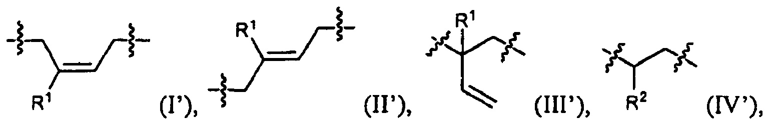 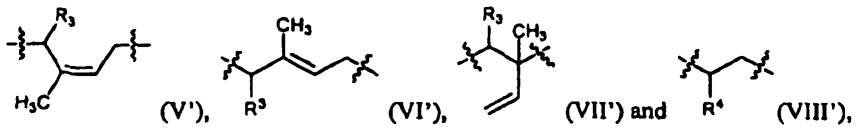 wherein R¹ has formula (XI): 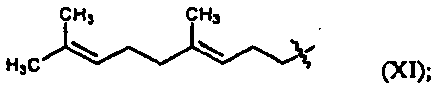 R² has formula (XII): 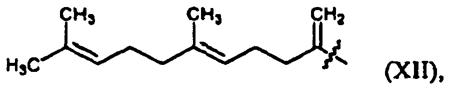 R³ has formula (XIII): 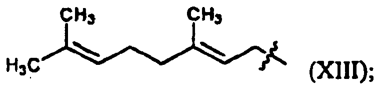 and R⁴ has formula (XIV):

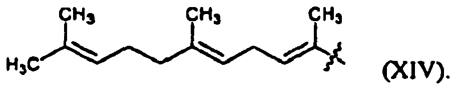

In some embodiments, the amount of formula (I'), *i*.*e*., the *cis*-1,4-microstructure, in the polyfarnesene disclosed herein is at most 80 wt.%. In other embodiments, the amount of formula (II'), *i.e*., the *trans*-1,4-microstructure, in the polyfarnesene disclosed herein is from 5 wt.% to 99 wt.%, based on the total weight of the polyfarnesene. In further embodiments, the amount of formula (III') is at least 70 wt.%, based on the total weight of the polyfarnesene. In still further embodiments, at least a portion of the double bonds in one or more of formulae (I')-(III'), (V')-(VII'), and (XI)-(XIV) and stereoisomers thereof is hydrogenated.

In certain embodiments, Y of the polyfarnesene disclosed herein has formula (IX') or a stereoisomer thereof: 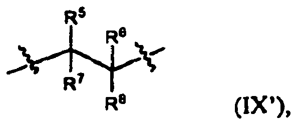 wherein each of R⁵, R⁶, R⁷ and R⁸ is independently H, alkyl, cycloalkyl, aryl, alkenyl, cycloalkenyl, alkynyl, heterocyclyl, alkoxy, aryloxy, carboxy, alkoxycarbonyl, aminocarbonyl, alkylaminocarbonyl, dialkylaminocarbonyl, acyloxy, nitrile or halo.

In some embodiments, R⁵ is aryl; and each of R⁶, R⁷ and R⁸ is H. In other embodiments, the aryl is phenyl.

In certain embodiments, the sum of m and n of the polyfarnesene disclosed herein is greater than 300. In other embodiments, m of the polyfarnesene disclosed herein is 0 and the polyfarnesene is a farnesene homopolymer. In further embodiments, m is from 1 to 100,000 and the polyfarnesene is a random farnesene interpolymer. In some embodiments, m is from 1 to 100,000 and the polyfarnesene is a block farnesene interpolymer. In further embodiments, the block farnesene interpolymer comprises one block comprising X and two blocks comprising Y and wherein the block comprising X is between the two blocks comprising Y.

In certain embodiments, the M_{w} of the polyfarnesene disclosed herein is greater than 60,000 daltons. In other embodiments, the T_{g} of the polyfarnesene disclosed herein is less than -60 °C.

In some embodiments, the tackifier is present from 5 wt.% to 70 wt.%, based on the total weight of the composition. In other embodiments, the tackifier has a ring and ball (R&B) softening point equal to or greater than 80°C, as measured in accordance with ASTM 28-67.

In certain embodiments, the composition disclosed herein further comprises an additive selected from the group consisting of plasticizers, oils, waxes, antioxidants. UV stabilizers, colorants or pigments, fillers, flow aids, coupling agents, crosslinking agents, surfactants, solvents and combinations thereof.

In one aspect, provided herein is a composition comprising a polyfarnesene and a tackifier, wherein the polyfarnesene is obtainable by polymerizing a farnesene and an optional vinyl monomer in the presence of a catalyst, with the proviso that when the vinyl monomer is present, the mole percent ratio of the farnesene to the vinyl monomer is from 1:4 to 100:1.

In certain embodiments, the farnesene is α-farnesene, β-farnesene or a combination thereof. In other embodiments, the farnesene is β-farnesene. In further embodiments, the amount of cis-1,4-microstructure in the polyfarnesene is at most 80 wt.%, based on the total weight of the polyfarnesene.

In some embodiments, the vinyl monomer is present and the polyfarnesene is a farnesene interpolymer. In other embodiments, the vinyl monomer is styrene. In further embodiments, the farnesene interpolymer is a block interpolymer.

In certain embodiments, the farnesene is prepared by a microorganism. In other embodiments, the farnesene is derived from a simple sugar.

In some embodiments, the catalyst comprises an organolithium reagent. In other embodiments, the catalyst further comprises 1,2-bis(dimethylamino)ethane. In further embodiments, the composition disclosed herein is a hot melt adhesive composition. In still further embodiments, the composition disclosed herein is a pressure sensitive adhesive composition.

In one aspect, provided herein is an article comprising a substrate partially or fully coated with the composition disclosed herein. In some embodiments, the article is a paper product, packaging material, laminated wood panel, kitchen countertop, vehicle, label, disposable diaper, hospital pad, feminine sanitary napkin, surgical drape, tape, case, carton, tray, medical device or bandage. In other embodiments, the article is a tape, case, carton, tray, medical device or bandage.

In one aspect, provided herein is a method of adhering a first adherend to a second adherend by using an adhesive comprising the composition disclosed herein. In some embodiments, the first adherend and the second adherend are the same. In other embodiments, the first adherend and the second adherend are different.

In certain embodiments, each of the first adherend and the second adherend independently comprises metal, wood, paper, plastic, rubber, glass, stone, granite, marble, masonry, porcelain, ceramic, tile, china, concrete, clay, sand, chalk, textile, cloth, non-woven fabric, leather or a combination thereof. In other embodiments, each of the first adherend and the second adherend is independently in the form of sheet, strip, tape, label, tag, web, disk, plate, film or any molded shape.

In one aspect, provided herein is an adhesive composition comprising a rubber and a tackifier, wherein the tackifier comprises a polyfarnesene disclosed herein. In some embodiments, the polyfarnesene is present in the range from 5 wt.% to 70 wt.%, based on the total weight of the adhesive composition. In other embodiments, the polyfarnesene has a R&B softening point equal to or greater than 80°C, as measured in accordance with ASTM 28-67. In further embodiments, the rubber has a T_{g} less than about 20 °C.

Additional aspects of the invention and characteristics and properties of various embodiments of the invention become apparent with the following description.

Figure 1 depicts Ultraviolet-Visible (UV-Vis) spectra of Example 1 and β-famesene.

Figure 2 depicts a Gel Permeation Chromatography (GPC) curve of Example 1.

Figure 3 depicts a C¹³ Nuclear Magnetic Resonance (NMR) spectrum of Example 1.

Figure 4 depicts a H¹ NMR spectrum of Example 1.

Figure 5 depicts a Differential Scanning Calorimetry (DSC) curve of Example 1.

Figure 6 depicts a thermal Gravimetric Analysis (TGA) curve of Example 1 measured in air.

Figure 7 depicts a TGA curve of Example 1 measured in nitrogen.

Figure 8 depicts lap test results of Example 1.

Figure 9 depicts a GPC curve of Example 2.

Figure 10 depicts a DSC curve of Example 2.

Figure 11 depicts tensile test results of Example 2.

Figure 12 depicts a GPC curve of Example 3.

Figure 13 depicts a C¹³ NMR spectrum of Example 3.

Figure 14 depicts a H¹ NMR spectrum of Example 3.

Figure 15 depicts a DSC curve of Example 3.

Figure 16 depicts a TGA curve of Example 3.

Figure 17 depicts lap test results of Example 3.

Figure 18 depicts a GPC curve of polystyrene formed.

Figure 19 depicts a GPC curve of polystyrene-1,4-polyfarnesene di-block copolymer formed.

Figure 20 depicts a GPC curve of Example 4.

Figure 21 depicts a ¹³C NMR spectrum of Example 4.

Figure 22 depicts a ¹H NMR spectrum of Example 4.

Figure 23 depicts a DSC curve of Example 4.

Figure 24 depicts a TGA curve of Example 4.

Figure 25 depicts tensile test results of Example 4.

Figure 26 depicts lap test results of Example 4.

Figure 27 depicts a GPC curve of polystyrene formed.

Figure 28 depicts a GPC curve of polystyrene-3,4-polyfarnesene di-block copolymer formed.

Figure 29 depicts a GPC curve of Example 5.

Figure 30 depicts a ¹³C NMR spectrum of Example 5.

Figure 31 depicts a ¹H NMR spectrum of Example 5.

Figure 32 depicts a DSC curve of Example 5.

Figure 33 depicts a TGA curve of Example 5.

Figure 34 depicts tensile test results of Example 5.

Figure 35 depicts a GPC curve of Example 5 after extraction with hexane.

Figure 36 depicts a GPC curve of hexane after extraction for Example 5.

Figure 37 depicts tensile test results of Example 5.

Figure 38 depicts lap test results of Example 5.

Figure 39 depicts tensile test results of Example 6.

Figure 40 depicts tensile test results of Example 7.

### General Definitions

"Polymer" refers to a polymeric compound prepared by polymerizing monomers, whether of the same or a different type. The generic term "polymer" embraces the terms "homopolymer," "copolymer," "terpolymer" as well as "interpolymer."

"Interpolymer" refers to a polymer prepared by the polymerization of at least two different types of monomers. The generic term "interpolymer_{,} includes the term "copolymer" (which generally refers to a polymer prepared from two different monomers) as well as the term "terpolymer" (which generally refers to a polymer prepared from three different types of monomers). It also encompasses polymers made by polymerizing four or more types of monomers.

"Organyl" refers to any organic substituent group, regardless of functional type, having one free valence at a carbon atom, *e.g*., CH₃CH₂-, CICH₂-, CH3C(=O)-, 4-pyridylmethyl.

"Hydrocarbyl" refers to any univalent group formed by removing a hydrogen atom from a hydrocarbon, such as alkyl (*e.g*., ethyl), cycloalkyl (*e.g*., cyclohexyl) and aryl (*e.g*., phenyl).

"Heterocyclyl" refers to any univalent group formed by removing a hydrogen atom from any ring atom of a heterocyclic compound.

"Alkyl" or "alkyl group" refers to a univalent group having the general formula CₙH₂ₙ₊₁ derived from removing a hydrogen atom from a saturated, unbranched or branched aliphatic hydrocarbon, where n is an integer, or an integer between 1 and 20, or between 1 and 8. Examples of alkyl groups include, but are not limited to, (C₁-C₈)alkyl groups, such as methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl and octyl. Longer alkyl groups include nonyl and decyl groups. An alkyl group can be unsubstituted or substituted with one or more suitable substituents. Furthermore, the alkyl group can be branched or unbranched. In some embodiments, the alkyl group contains at least 2, 3, 4, 5, 6, 7, or 8 carbon atoms.

"Cycloalkyl" or "cycloalkyl group" refers to a univalent group derived from a cycloalkane by removal of a hydrogen atom from a non-aromatic, monocyclic or polycyclic ring comprising carbon and hydrogen atoms. Examples of cycloalkyl groups include, but are not limited to, (C₃-C₇)cycloalkyl groups, such as cyclopropyl, cyclobutyl, cyclopentyl, cyclohexyl, and cycloheptyl, and saturated cyclic and bicyclic terpenes and (C₃-C₇)cycloalkenyl groups, such as cyclopropenyl, cyclebutenyl, cyclopentenyl, cyclohexenyl, and cycloheptenyl, and unsaturated cyclic and bicyclic terpenes. A cycloalkyl group can be unsubstituted or substituted by one or two suitable substituents. Furthermore, the cycloalkyl group can be monocyclic or polycyclic. In some embodiments, the cycloalkyl group contains at least 5, 6, 7, 8, 9, or 10 carbon atoms.

"Aryl" or "aryl group" refers to an organic radical derived from a monocyclic or polycyclic aromatic hydrocarbon by removing a hydrogen atom. Non-limiting examples of the aryl group include phenyl, naphthyl, benzyl, or tolanyl group, sexiphenylene, phenanthrenyl, anthracenyl, coronenyl, and tolanylphenyl. An aryl group can be unsubstituted or substituted with one or more suitable substituents. Furthermore, the aryl group can be monocyclic or polycyclic. In some embodiments, the aryl group contains at least 6, 7, 8, 9, or 10 carbon atoms.

"Isoprenoid" and "isoprenoid compound" are used interchangeably herein and refer to a compound derivable from isopentenyl diphosphate.

"Substituted" as used to describe a compound or chemical moiety refers to that at least one hydrogen atom of that compound or chemical moiety is replaced with a second chemical moiety. The second chemical moiety can be any desired substituent that does not adversely affect the desired activity of the compound. Examples of substituents are those found in the exemplary compounds and embodiments disclosed herein, as well as halogen; alkyl; heteroalkyl; alkenyl; alkynyl; aryl, heteroaryl, hydroxyl; alkoxyl; amino; nitro; thiol; thioether; imine; cyano; amido; phosphonato; phosphine; carboxyl; thiocarbonyl; sulfonyl; sulfonamide; carbonyl; formyl; carbonyloxy; oxo; haloalkyl (*e.g*., trifluoromethyl); carbocyclic cycloalkyl, which can be monocyclic or fused or non-fused polycyclic (*e.g*., cyclopropyl, cyclobutyl, cyclopentyl or cyclohexyl) or a heterocycloalkyl, which can be monocyclic or fused or non-fused polycyclic (*e.g*., pyrrolidinyl, piperidinyl, piperazinyl, morpholinyl or thiazinyl); carbocyclic or heterocyclic, monocyclic or fused or non-fused polycyclic aryl (*e.g*., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl or benzofuranyl); amino (primary, secondary or tertiary); o-lower alkyl; o-aryl, aryl; aryl-lower alkyl; -CO₂CH₃; -CONH₂; -OCH₂CONH₂; -NH₂; -SO₂NH₂; -OCHF₂; CF₃; -OCF₃; -NH(alkyl); -N(alkyl)₂; -NH(aryl); -N(alkyl)(aryl); -N(aryl)₂; -CHO; - CO(alkyl); -CO(aryl); -CO₂(alkyl); and -CO₂(aryl); and such moieties can also be optionally substituted by a fused-ring structure or bridge, for example -OCH₂O-. These substituents can optionally be further substituted with a substituent selected from such groups. All chemical groups disclosed herein can be substituted, unless it is specified otherwise.

"Organolithiurn reagent" refers to an organometallic compound with a direct bond between a carbon and a lithium atom. Some non-limiting examples of organolithium reagents include vinyllithium, aryllithium (e.g., phenyllithium), and alkyllithium (*e.g*., *n-*butyl lithium, *sec-* butyl lithium, *tert*- butyl lithium, methyllithium, isopropyllithium or other alkyllithium reagents having 1 to 20 carbon atoms).

A composition that is "substantially free" of a compound means that the composition contains less than 20 wt.%, less than 10 wt.%, less than 5 wt.%, less than 3 wt.%, less than 1 wt.%, less than 0.5 wt.%, less than 0.1 wt%, or less than 0.01 wt.% of the compound, based on the total volume of the composition.

A polymer that is "substantially linear" means that the polymer contains less than 20 wt.%, less than 10 wt.%, less than 5 wt.%, less than 3 wt.%, less than 1 wt.%, less than 0.5 wt.%, less than 0.1 wt.%, or less than 0.01 wt.% of the branched, star-shaped or other regular or irregular structures, based on the total volume of the composition.

A polymer that is "substantially branched" means that the polymer contains less than 20 wt.%, less than 10 wt.%, less than 5 wt.%, less than 3 wt.%, less than 1 wt.%, less than 0.5 wt.%, less tha 0.1 wt.%, or less than 0.01 wt.% of the linear, star-shaped or other regular or irregular structures, based on the total volume of the composition.

A polymer that is "substantially star-shaped" means that the polymer contains less than 20 wt.%, less than 10 wt.%, less than 5 wt.%, less than 3 wt.%, less than 1 wt.%, less than 0.5 wt.%, less than 0.1 wt.%,or less than 0.01 wt.% of the branched, linear or other regular or irregular structures, based on the total volume of the composition.

In the following description, all numbers disclosed herein are approximate values, regardless whether the word "about" or "approximate" is used in connection therewith. They may vary by percent, 2 percent, 5 percent, or, sometimes, 10 to 20 percent. Whenever a numerical range with a lower limit, R^{L}, and an upper limit, R^{U}, is disclosed, any number falling within the range is specifically disclosed. In particular, the following numbers within the range are specifically disclosed: R=R^{L}+k*(R^{U}-R^{L}). wherein k is a variable ranging from 1 percent to 100 percent with a 1 percent increment, *i.e*., k is 1 percent, 2 percent, 3 percent, 4 percent, 5 percent,..., 50 percent, 51 percent, 52 percent,..., 95 percent, 96 percent, 97 percent, 98 percent, 99 percent, or 100 percent. Moreover, any numerical range defined by two R numbers as defined in the above is also specifically disclosed.

The compositions disclosed herein generally comprise a polyfarnesene and a tackifier.

In some embodiments, the polyfarnesene is a farnesene homopolymer, a farnesene interpolymer or a combination thereof. In certain embodiments, the polyfarnesene is a farnesene homopolymer comprising units derived from at least one farnesene such as a-famesene, β-farnesene or a combination thereof. In other embodiments, the polyfarnesene is a farnesene interpolymer comprising units derived from at least one farnesene and units derived from at least one copolymerizable vinyl monomer. In further embodiments, the farnesene interpolymer is derived from styrene and at least one famesene. In still further embodiments, the farnesene interpolymer is a random, block or alternating interpolymer. In still further embodiments, the farnesene interpolymer is a di-block, tri-block or other multi-block interpolymer.

In some embodiments, the farnesene homopolymer is prepared by polymerizing β-farnesene in the presence of any catalyst suitable for polymerizing olefins such as ethylene, styrene or isoprene. In other embodiments, the farnesene homopolymer comprises one or more units having formula (I), (II), (III), (IV), a stereoisomer thereof or a combination thereof: 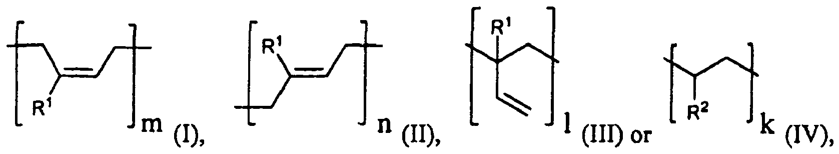 wherein R¹ has formula (XI): 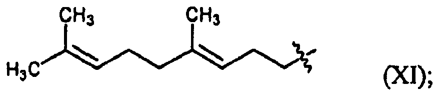 and R² has formula (XII): 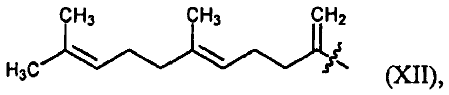 wherein each of m, n,1 and k is independently an integer from 1 to 5,000, from 1 to 10,000, from 1 to 50,000, from 1 to 100,000, from 2 to 10,000, from 2 to 50,000, from 2 to 100,000. In some embodiments, each of m, n, I and k is independently an integer from 1 to 100,000. In other embodiments, each of m, n, 1 and k is independently an integer from 2 to 100,000.

In certain embodiments, the farnesene homopolymer comprises at least one unit having formula (I) wherein m is greater than 300, greater than 500 or greater than 1000. In other embodiments, the farnesene homopolymer comprises at least one unit having formula (II) wherein n is greater than 300, greater than 500 or greater than 1000. In further embodiments, the farnesene homopolymer comprises at least one unit having formula (III) wherein 1 is greater than 300, greater than 500 or greater than 1000. In still further embodiments, the farnesene homopolymer comprises at least one unit having formula (IV) wherein k is greater than 300, greater than 500 or greater than 1000.

In some embodiments, the farnesene homopolymer comprises at least one unit having formula (I) and at least one unit having formula (II), wherein the sum of m and n is greater than 300, greater than 500 or greater than 1000. In other embodiments, the farnesene homopolymer comprises at least one unit having formula (I) and at least one unit having formula (III), wherein the sum of m and 1 is greater than 300, greater than 500 or greater than 1000. In other embodiments, the farnesene homopolymer comprises at least one unit having formula (II) and at least one unit having formula (III), wherein the sum of n and I is greater than 300, greater than 500 or greater than 1000. In still further embodiments, the farnesene homopolymer comprises at least one unit having formula (I), at least one unit having formula (II) and at least one unit having formula (III), wherein the sum of m, n and I is greater than 300, greater than 500 or greater 1000. In still further embodiments, the farnesene. homopolymer comprises at least one unit having formula (I), at least one unit having formula (II), at least one unit having formula (III) and at least one unit having formula (IV), wherein the sum of m, n, l and k is greater than 300, greater than 500 or greater than 1000. In still further embodiments, the one or more units having formula (I), (II), (III) or (IV) in the farnesene homopolymer disclosed herein can be in any order.

In certain embodiments, the farnesene homopolymer is prepared by polymerizing a-farnesene in the presence of any catalyst suitable for polymerizing olefins. In other embodiments, the farnesene homopolymer comprises one or more units having formula (V), (VI), (VII), (VIII), a stereoisomer thereof or a combination thereof: 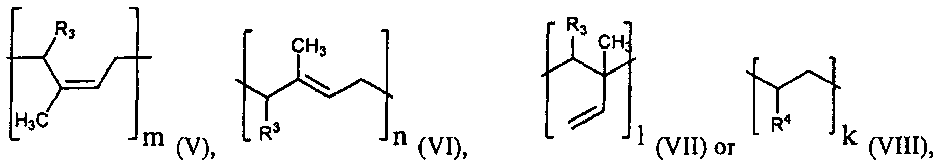 wherein R³ has formula (XIII): 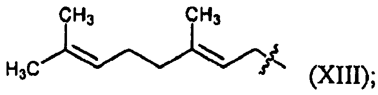 and R⁴ has formula (XIV): 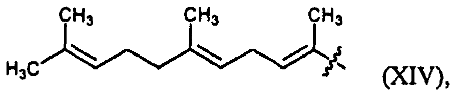 wherein each of m, n, I And k is independently an integer from 1 to 5,000, from 1 to 10,000, from 1 to 50,000, from 1 to 100,000, from 2 to 10,000, from 2 to 50,000, from 2 to 100,000. In some embodiments, each of m, n, I and k is independently an integer from 1 to 100,000. In other embodiments, each of m, n, I and k is independently an integer from 2 to 100,000.

In certain embodiments, the farnesene homopolymer comprises at least one unit having formula (V) wherein m is greater than 300, greater than 500 or greater than 1000. In other embodiments, the farnesene homopolymer comprises at least one unit having formula (VI) wherein n is greater than 300, greater than 500 or greater than 1000. In further embodiments, the farnesene homopolymer comprises at least one unit having formula (VII) wherein 1 is greater than 300, greater than 500 or greater than 1000. In still further embodiments, the farnesene homopolymer comprises at least one unit having formula (VIII) wherein k is greater than 300, greater than 500 or greater than 1000.

In some embodiments, the farnesene homopolymer comprises at least one unit having formula (V) and at least one unit having formula (VI), wherein the sum of m and n is greater than 300, greater than 500 or greater than 1000. In other embodiments, the farnesene homopolymer comprises-at-least one unit having formula (V) and at least one unit having formula (VII), wherein the sum of m and 1 is greater than 300, greater than 500 or greater than 1000. In other embodiments, the farnesene homopolymer comprises at least one unit having formula (VI) and at least one unit having formula (VII), wherein the sum of n and 1 is greater than 300, greater than 500 or greater than 1000. In still further embodiments, the farnesene homopolymer comprises at least one unit having formula (V), at least one unit having formula (VI) and at least one unit having formula (VII), wherein the sum of m, n and 1 is greater than 300, greater than 500 or greater than 1000. In still further embodiments, the farnesene homopolymer comprises at least one unit having formula (V), at least one unit having formula (VI), at least one unit having formula (VII) and at least one unit having formula (VIII), wherein the sum of n, 1 ann and k is greater than 300, greater than 500 or greater than 1000. In still further embodiments, the one or more units having formula (V), (VI), (VII) or (VIII) in the farnesene homopolymer disclosed herein can be in any order.

In some embodiments, the farnesene homopolymer is prepared by polymerizing a mixture of α-farnesene and β-farnesene in the presence of any catalyst suitable for polymerizing olefins. In other embodiments, the farnesene homopolymer comprises one or more units having-formula (I), (II), (III), (IV), (V), (VI), (VII) or (VIII) disclosed herein, a stereoisomer thereof or a combination thereof. In further embodiments, the one or more units having formula (I), (II), (III), (IV), (V), (VI), (VII) or (VIII) in the farnesene homopolymer disclosed herein can be in any order.

In some embodiments, the farnesene homopolymer comprises two or more units having two different formulae selected from formulae (I), (II), (III), (IV), (V), (VI), (VII), (VIII), stereoisomers thereof and combinations thereof. In other embodiments, such farnesene homopolymer can be represented by the following formula: AₓB_{y} wherein each of x and y is at least 1, and wherein each of A and B independently has formula (I), (II), (III), (IV), (V), (VI), (VII) or (VIII) and A and B are different. In further embodiment, each of x and y is independently greater than 1, such as 2, 3, 4, 5, 6, 7, 8, 9, 10, 15, 20, 25, 30, 35, 40, or higher. In some embodiment, the As and Bs are linked in a substantially linear fashion, as opposed to a substantially branched or substantially star-shaped fashion. In other embodiments, the As and Bs are randomly distributed along the farnesene homopolymer chain. In other embodiments, the As and Bs are in two "segments" to provide a farnesene homopolymer having a segmented structure, for example, AA--A-BB --- B. In other embodiments, the As and Bs are alternatively distributed along the farnesene homopolymer chain to provide a farnesene homopolymer having an alternative structure, for example, A-B, A-B-A, A-B-A-B, A-B-A-B-A or the like.

In some embodiments, the farnesene homopolymer comprises three or more units having three different formulae selected from formulae (I), (II), (III), (IV), (V), (VI), (VII), (VIII), stereoisomers thereof and combinations thereof. In other embodiments, such farnesene homopolymer can be represented by the following formula: AₓB_{y}C_{z} wherein each of x, y and z is at least 1, and wherein each of A, B and C independently has formula (I), (II), (III), (IV), (V), (VI), (VII) or (VIII) and A, B and C are different. In further embodiment, each of x, y and z is independently greater than 1, such as 2, 3, 4, 5, 6, 7, 8, 9, 10, 15, 20, 25, 30, 35, 40, or higher. In some embodiment, the As, Bs and Cs are linked in a substantially linear fashion, as opposed to a substantially branched or substantially star-shaped fashion. In other embodiments, the As, Bs and Cs are randomly distributed along the farnesene homopolymer chain. In other embodiments, the As, Bs and Cs are in three "segments" to provide a farnesene homopolymer having a segmented structure, for example, AA--A-BHB-CC--C. In other embodiments, the As, Bs and Cs are alternatively distributed along the farnesene homopolymer chain to provide a farnesene homopolymer having an alternative structure, for example, A-B-C-A-B, A-B-C-A-B-C or the like.

In certain embodiments, the polyfarnesene is a farnesene interpolymer. In other embodiments, the farnesene interpolymer is prepared by polymerizing at least one farnesene and at least one vinyl monomer in the presence of any catalyst suitable for polymerizing olefins and vinyl monomers. In further embodiments, the farnesene interpolymer disclosed herein comprises (a) one or more units having at least one of formulae (I), (II), (III) and (IV) disclosed herein; and (b) one or more units having formula (IX): 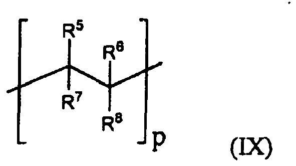 wherein p is an integer from 1 to 5,000, from 1 to 10,000, from 1 to 50,000, from 1 to 100,000, from 2 to 10,000, from 2 to 50,000, from 2 to 100,000 ; and each of R⁵, R⁶, R⁷ and R⁸ is independently H, an organyl group, or a functional group. In some embodiments, each of R⁵, R⁶ , R⁷ and R⁸ is not a monovalent hydrocarbon group containing 4-8 carbon atoms. In some embodiments, each of R⁵, R⁶, R⁷ and R⁸ is not an alkyl group containing 4-8 carbon atoms.

In some embodiments, the farnesene interpolymer disclosed herein comprises (a) one or more units having at least one of formulae (V), (VI), (VII) and (VIII) disclosed herein; and (b) one or more units having formula (IX) disclosed herein. In other embodiments, the farnesene interpolymer disclosed herein comprises (a) one or more units having at least one of formulae (I), (II), (III), (IV), (V), (VI), (VII) and (VIII) disclosed herein; and (b) one or more units having formula (IX) disclosed herein.

In some embodiments, the farnesene interpolymer disclosed herein is a random interpolymer. In other embodiments, the farnesene interpolymer disclosed herein is a random interpolymer wherein the vinyl monomer units and the farnesene units are randomly distributed. In further embodiments, the farnesene interpolymer disclosed herein is a random interpolymer wherein the vinyl monomer units and the farnesene units are randomly distributed and wherein two or more of formulae (I), (II), (III), (IV), (V), (VI), (VII), (VIII) and (XI) in the farnesene units are distributed randomly, alternatively or in blocks.

In some embodiments, the farnesene interpolymer disclosed herein is an alternating interpolymer. In other embodiments, the farnesene interpolymer disclosed herein is an alternating interpolymer wherein the vinyl monomer units and the farnesene units are alternatively distributed. In further embodiments, the farnesene interpolymer disclosed herein is an alternating interpolymer wherein the vinyl monomer units and the farnesene units are alternatively distributed and wherein two or more of formulae (I), (II), (III), (IV), (V), (VI), (VII), (VIII) and (XI) in the farnesene units are distributed randomly, alternatively or in blocks.

In certain embodiments, the farnesene interpolymer is a block interpolymer having one or more first blocks comprising the one or more units having formula (I), (II), (III), (IV) or a combination thereof and one or more second blocks comprising the one or more units having formula (IX). In further embodiments, the farnesene interpolymer is a block interpolymer having one or more first blocks comprising the one or more units having formula (V), (VI), (VII), (VIII) or a combination thereof and one or more second blocks comprising the one or more units having formula (IX). In still further embodiments, there are one first block and more second blocks and wherein the first block is between the two second blocks. In still further embodiments, each of the second blocks comprises units derived from styrene. In some embodiments, the farnesene block interpolymer is a polystyrene-polyfarnesene di-block polyfarnesene, polystyrene-polyfarnesene-polystyrene tri-block polyfarnesene or a combination thereof.

In some embodiments, the farnesene interpolymer can be represented by the following formula: PₓQ_{y} wherein each of x and y is at least 1, and wherein P has formula (IX) and Q has formula (I), (II), (III), (IV), (V), (VI), (VII) or (VIII). In further embodiment, each of x and y is independently greater than 1, such as 2, 3, 4, 5, 6, 7, 8, 9, 10, 15, 20, 25, 30, 35, 40, or higher. In some embodiment, the Ps and Qs are linked in a substantially linear fashion, as opposed to a substantially branched or substantially star-shaped fashion. In other embodiments, the Ps and Qs are randomly distributed along the farnesene interpolymer chain. In other embodiments, the Ps and Qs are in two or more blocks or segments to provide a farnesene interpolymer having a block structure, for example, PP--P-QQ---Q or PP--P-QQ-Q-P---PP. In other embodiments, the Ps and Qs are alternatively distributed along the farnesene interpolymer chain to provide a farnesene interpolymer having an alternative structure, for example, P-Q, P-Q-P, P-Q-P-Q, P-Q-P-Q-P or the like. In some embodiments, each Q has formula AₓB_{y} or AₓB_{y}Cₓ as disclosed herein.

In certain embodiments, the amount of formula (I) in the polyfarnesene disclosed herein is at most 85 wt.%, at most 80 wt.%, at most 70 wt%, at most 60 wt%, or at most 50 wt.%, based on the total weight of the polyfarnesene. In other embodiments, the amount of formula (III) in the polyfarnesene disclosed herein is at least 10 wt.%, at least 15 wt.%, at least 20 wt.%, at least 25 wt.%, at least 30 wt.%, at least 40 wt%, at least 50 wt.%, at least 60 wt.%, at least 70 wt.%, at least 80 wt.%, at least 90 wt.%, at least 95 wt.%, or at least 99 wt.%, based on the total weight of the polyfarnesene. In further embodiments, the amount of formula (II) in the polyfarnesene disclosed herein is from 1 wt.% to 99 wt.%, from 5 wt.% to 99 wt.%, from 10 wt.% to 99 wt.%, or from 15 wt.% to 99 wt.%, based on the total weight of the polyfarnesene. In still further embodiments, the amount of formula (IV) in the polyfarnesene disclosed herein is at most 0.1 wt.%, at most 0.5 wt.%, at most wt%, at most 2 wt.%, or at most 3 wt.%, based on the total weight of the polyfarnesene. In some embodiments, the polyfarnesene disclosed herein is substantially free of formula (I), (II), (III) or (IV).

In certain embodiments, the amount of formula (V), (VI), (VII) or (VIII) in the polyfarnesene disclosed herein is at most 1 wt.%, at most 5 wt.%, at most 10 wt.%, at most 20 wt.%, at most 30 wt.%, at most 40 wt.%, at most 50 wt.%, at most 60 wt.%, at most 70 wt.%, at most 80 wt.%, or at most 90 wt.%, based on the total weight of the polyfarnesene. In other embodiments, the amount of formula (V), (VI), (VII) or (VIII) in the polyfarnesene disclosed herein is at least 1 wt.%, at least 2 wt.%, at least 3 wt.%, at least 5 wt.%, at least 10 wt.%, at least 20 wt.%, at least 30 wt.%, at least 40 wt.%, at least 50 wt.%, at least 60 wt.%, based on the total weight of the polyfarnesene. In further embodiments, the amount of formula (V), (VI), (VII) or (VIII) in the polyfarnesene disclosed herein is from 1 wt.% to 99 wt.%, from 5 wt.% to 99 wt.%, from 10 wt.% to 99 wt.%, or from 15 wt.% to 99 wt.%, based on the total weight of the polyfarnesene. In some embodiments, the polyfarnesene disclosed herein is substantially free of formula (V), (VI), (VII) or (VIII).

In other embodiments, the sum of m and n disclosed herein is greater than 250, greater than 300, greater than 500, greater than 750, greater thank 1000, or greater than 2000. In further embodiments, the sum of m and 1 disclosed herein is greater than 250, greater than 300, greater than 500, greater than 750, greater than 1000, or greater than 2000. In certain embodiments, the sum of m, n and I disclosed herein is greater than 250, greater than 300, greater than 500, greater than 750, greater than 1000, or greater than 2000. In some embodiments, the sum of m, n, l and k disclosed herein is greater than 250, greater than 300, greater than 500, greater than 750, greater than 1000, or greater than 2000.

In certain embodiments, the number-average molecular weight (Mₙ), weight-average molecular weight (M_{w}), or viscosity-average molecular weight (M_{z}) of the polyfarnesene disclosed herein is greater than 60,000 daltons, greater than 100,000 daltons, greater than 200,000 daltons, greater than 300,000 daltons, greater than 500,000 daltons, greater than 750,000 daltons, greater than 1,000,000 daltons, greater than 1,500,000 daltons, or greater than 2,000,000 daltons. In other embodiments, the Mₙ, M_{w} or M_{z} of the polyfarnesene disclosed herein is less than 10,000,000 daltons, less than 5,000,000 daltons, less than 1,000,000 daltons, less than 750,000 daltons, or less than 500,000 daltons.

In some embodiments, the polyfarnesene has at least a glass transition temperature (T_{g}) of less than -55 °C, less than -60 °C, less than -65 °C, less than -70 °C, or less than -75 °C, as measured according to ASTM D7426-08 titled "*Standard Test Method for Assignment of the DSC Procedure for Determining T_{g} ala Polymer or an Elastomeric Compound*," which is incorporated herein by reference.

In some embodiments, the amount of formula (I) is at most 80 wt.%, based on the total weight of the polyfarnesene. In other embodiments, the sum of m, n and I is greater than 300. In further embodiments, at least a portion of the double bonds in one or more of formulae (I), (II), (III), (IV), (IX), (XI), (XII) and stereoisomers thereof is hydrogenated.

In some embodiments, the polyfarnesene is a farnesene interpolymer. In further embodiments, the farnesene interpolymer disclosed herein comprises one or more units derived from a farnesene in an amount of at least 5 mole percent, at least 10 mole percent, at least 15 mole percent, at least 20 mole percent, at least 30 mole percent, at least 40 mole percent, at least 50 mole percent, at least 60 mole percent, at least 70 mole percent, at least 80 mole percent, or at least 90 mole percent of the whole farnesene interpolymer. In still further embodiments, the farnesene interpolymer disclosed herein comprises one or more units derived from the vinyl monomer in an amount of at least 5 mole percent, at least 10 mole percent, at least 15 mole percent, at least 20 mole percent, at least 30 mole percent, at least 40 mole percent, at least 50 mole percent, at least 60 mole percent, at least 70 mole percent, at least 80 mole percent, or at least 90 mole percent of the whole farnesene interpolymer.

In certain embodiments, the polyfarnesene comprises one or more polymer molecules having formula (X'): 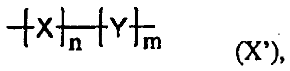 wherein n is an integer from 1 to 5,000, from 1 to 10,000, from 1 to 50,000, from 1 to 100,000; m is an integer from 0 to 5,000, from 0 to 10,000, from 0 to 50,000, from 0 to 100,000 ; X is derived from a farnesene; and Y is derived from a vinyl monomer.

In some embodiments, X has one or more of formulae (I')-(VIII'):

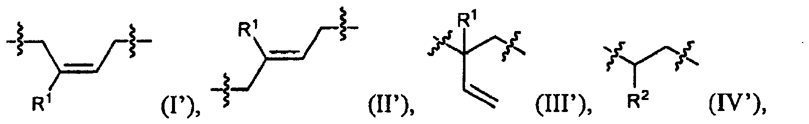

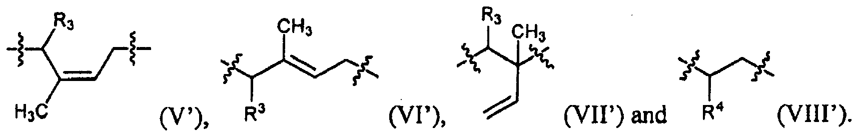

In certain embodiments, Y has formula (IX'): 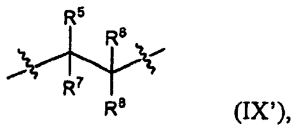 where R¹, R², R³, R⁴ are as defined herein and each of R⁵, R⁶, R⁷ and R⁸ is independently H, an organyl group or a functional group.

In general, the polyfarnesene comprising a mixture of polymer molecules, each of which has formula (X') wherein each of n and m independently has a specific value. The average and distribution of the n or m values disclosed herein depend on various factors such as the molar ratio of the starting materials, the reaction time and temperature, the presence or absence of a chain terminating agent, the amount of an initiator if there is any, and the polymerization conditions. The farnesene interpolymer of Formula (X') may include unreacted comonomers, although the concentrations of the comonomer would generally be small if not extremely small or undetectable. The extent of polymerization, as specified with n and m values, can affect the properties of the resulting polymer. In some embodiments, n is an integer from 1 to 5,000, from 1 to 10,000, from 1 to 50,000, from 1 to 100,000 ; and m is an integer from 0 to 5,000, from 0 to 10,000, from 0 to 50,000, from 0 to 100,000. In other embodiments, n is independently from 1 to 5000, from 1 to 2500, from 1 to abee 1000, from 1 to 500, from 1 to 100 or from 1 to 50; and m is from 0 to 5000, from 0 to 2500, from 0 to 1000, from 0 to 500, from 0 to 100 or from 0 to 50. A person of ordinary skill in the art will recognize that additional ranges of average n and m values are contemplated and are within the present disclosure.

In some embodiments, formula (X') comprises two end groups as shown by the following formula: 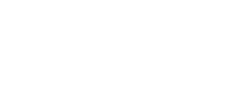 where each of the asterisks (*) in the formula represents an end group which may or may not vary between different polymer molecules of the polyfarnesene depending on many factors such as the molar ratio of the starting materials, the presence or absence of a chain terminating agent, and the state of the particular polymerization process at the end of the polymerization step.

In some embodiments, Xs and Ys of formula (X') are linked in a substantially linear fashion. In other embodiments, Xs and Ys of formula (X') are linked in substantially branched fashion. In further embodiments, Xs and Ys of formula (X') are linked in substantially star-shaped fashion. In still further embodiments, each of Xs and Ys independently forms at least a block along the polymer chain so as to provide a di-block, tri-block or multi-block farnesene interpolymer having at least one X block and at least one Y block. In still further embodiments, Xs and Ys are randomly distributed along the polymer chain so as to provide a random farnesene interpolymer. In still further embodiments, Xs and Ys are alternatively distributed along the polymer chain so as to provide an alternating farnesene interpolymer.

In some embodiments, the amount of the farnesene in the farnesene interpolymer disclosed herein is greater than 1.5 mole %, greater than 2.0 mole %, greater than 2.5 mole %, greater than 5 mole %, greater than 10 mole %, greater than 5 mole %, or greater than 20 mole %, based on the total amount of the farnesene interpolymer. In other embodiments, the amount of the farnesene in the farnesene interpolymer disclosed herein is less than 90 mole %, less than 80 mole %, less than 70 mole %, less than 60 mole %, less than 50 mole %, less than 40 mole %, or less than 30 mole %, based on the total amount of the farnesene interpolymer.

In some embodiments, the amount of the vinyl monomer in the farnesene interpolymer disclosed herein is greater than 1.5 mole %, greater than 2.0 mole %, greater than 2.5 mole %, greater than 5 mole %, greater than 10 mole %, greater than 15 mole %, or greater than 20 mole %, based on the total amount of the farnesene interpolymer. In other embodiments, the amount of the vinyl monomer in the farnesene interpolymer disclosed herein is less than 90 mole %, less than 80 mole %, less than 70 mole %, less than 60 mole %, less than 50 mole %, less than 40 mole %, or less than 30 mole %, based on the total amount of the farnesene interpolymer.

In certain embodiments, the mole percent ratio of the farnesene to the vinyl monomer (*i.e.*, the mole percent ratio of X to Y) in the farnesene interpolymer disclosed herein is from 1:5 to 100:1. In other embodiments, the mole percent ratio of X to Y is from 1:4 to 100:1; from 1:3.5 to ebewt 100:1, 1:3 to 100:1, from 1:2.5 to 100:1, or from 1:2 to 100:1. In some embodiments, m is 1 or greater, the mole percent ratio of X to Y is from 1:4 to J 100:1

In certain embodiments, the amount of formula (I') in the polyfarnesene disclosed herein is at most 85 wt.%, at most 80 wt.%, at most 70 wt.%, at most 60 wt.%, or at most 50 wt.%, based on the total weight of the polyfarnesene. In other embodiments, the amount of formula (III') in the polyfarnesene disclosed herein is at least wt.%, at least 15 wt.%, at least 10 wt.%, at least 25 wt.%, at least 30 wt.%, at least 40 wt.%, at least 50 wt.%, at least 60 wt.%, at least 70 wt%, at least 75 wt.%, at least 80 wt.%, at least 85 wt.%, at least 90 wt%, at least 95 wt.%, or at least 99 wt.%, based on the total weight of the polyfarnesene. In further embodiments, the amount of formula (II') in the polyfarnesene disclosed herein is from 1 wt.% to 99 wt%, from 5 wt.% to 99 wt%, from 0 wt.% to 99 wt.%, or from 15 wt.% to 99 wt.%, based on the total weight of the polyfarnesene. In still further embodiments, the amount of formula (IV') in the polyfarnesene disclosed herein is at most 0.1 wt.%, at most 0.5 wt.%, at most 1 wt%, at most 2 wt.%, or at most 3 wt.%, based on the total weight of the polyfarnesene. In some embodiments, the polyfarnesene disclosed herein is substantially free of formula (I'), (II'), (III') or (IV').

In certain embodiments, the amount of formula (V'), (VI'), (VII') or (VIII') in the polyfarnesene disclosed herein is at most 1 wt.%, at most 5 wt.%, at most 10 wt.%, at most 20 wt.%, at most 30 wt.%, at most 40 wt.%, at most 50 wt.%, at most 60 wt.%, at most 70 wt.%, at most 80 wt.%, or at most 90 wt.%, based on the total weight of the polyfarnesene. In other embodiments, the amount of formula (V'), (VI'), (VII') or (VIII') in the polyfarnesene disclosed herein is at least 1 wt.%, at least 2 wt.%, at least 3 wt.%, at least 5 wt.%, at least 10 wt.%, at least 20 wt.%, at least 30 wt.%, at least 40 wt.%, at least 50 wt.%, at least 60 wt.%, based on the total weight of the polyfarnesene. In further embodiments, the amount of formula (V'), (VI'), (VII') or (VIII') in the polyfarnesene disclosed herein is from 1 wt.% to 99 wt.%, from 5 wt.% to 9 wt.%, from 10 wt.% to 99 wt%, or from 15 wt.% to 99 wt.%, based on the total weight of the polyfarnesene. In some embodiments, the polyfarnesene disclosed herein is substantially free of formula (V'), (VI'), (VII') or (VIII').

Any compound containing a vinyl group, i.e., -CH=CH₂, that is copolymerizable with farnesene can be used as a vinyl monomer for making the farnesene interpolymer disclosed herein. Useful vinyl monomers disclosed herein include ethylene, i.e., CH₂=CH₂. In certain embodiments, the vinyl monomer has formula (XV): 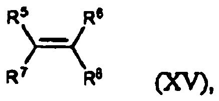 where each of R⁵, R⁶, R⁷ and R⁸ is independently H, an organyl group or a functional group.

In some embodiments, at least one of R⁵, R⁶, R⁷ and R⁸ of formula (IX), (IX') or (XV) is an organyl group. In further embodiments, the organyl group is hydrocarbyl, substituted hydrocarbyl, heterocyclyl or substituted heterocyclyl. In certain embodiments, each of R⁵, R⁶, R⁷ and R⁸ of formula (IX), (IX') or (XV) is independently H, alkyl, cycloalkyl, aryl, alkenyl, cycloalkenyl, alkynyl, heterocyclyl, alkoxy, aryloxy, carboxy, alkoxycarbonyl, aminocarbonyl, alkylaminocarbonyl, dialkylaminocarbonyl, acyloxy, nitrile or halo. In other embodiments, each of R⁵, R⁶, R⁷ and R⁸ of formula (IX), (IX') or (XV) is independently H, alkyl, cycloalkyl, aryl, cycloalkenyl, alkynyl, heterocyclyl, alkoxy, aryloxy, carboxy, alkoxycarbonyl, aminocarbonyl, alkylaminocarbonyl, dialkylaminocarbonyl, acyloxy, nitrile or halo. In certain embodiments, R⁵ of formula (IX), (IX') or (XV) is aryl; and each of R⁶, R⁷ and R⁸ is H. In further embodiments, R⁵ of formula (IX), (IX') or (XV) is phenyl; and each of R⁶, R⁷ and R⁸ is H.

In certain embodiments, at least one of R⁵, R⁶, R⁷ and R⁸ of formula (IX), (IX') or (XV) is H. In other embodiments, each of R⁵, R⁶, R⁷ and R⁸ of formula (IX), (IX') or (XV) is H. In further embodiments, R⁵ of formula (IX), (IX') or (XV) is hydrocarbyl; and each of R⁶, R⁷ and R⁸ is H. In still further embodiments, the hydrocarbyl is alkyl, cycloalkyl or aryl. In still further embodiments, none of R⁵, R⁶, R⁷ and R⁸ of formula (IX), (IX') or (XV) is or comprises alkenyl, cycloalkenyl or alkynyl. In still further embodiments, none of R⁵, R⁶, R⁷ and R⁸ of formula (IX), (IX') or (XV) is or comprises a hydrocarbyl, substituted hydrocarbyl, heterocyclyl or substituted heterocyclyl.

In certain embodiments, at least one of R⁵, R⁶, R⁷ and R⁸ of formula (IX), (IX') or (XV) is a functional group containing halo, O, N, S, P or a combination thereof. Some non-limiting examples of suitable functional groups include hydroxy, alkoxy, aryloxy, amino, nitro, thiol, thioether, imine, cyano, amido, phosphonato (-P(=O)(O-alkyl)₂, -P(=O)(O-aryl)₂, or -P(=O)(O-alkyl) )O-aryl), phosphinato (-P(=O)(O-alkyl)alkyl, -P(=O)(O-aryl)alkyl, - P(=O)(O-alkyl)aryl, or -P(=O)(O-aryl)aryl), carboxyl, thiocarbonyl, sulfonyl (-S(=O)₂alkyl, or -S(=O)₂aryl), sulfonamide (-SO₂NH₂, -SO₂NH(alkyl), -SO₂NH(aryl), -SO₂N(alkyl)₂, - SO₂N(aryl)₂, or -SO₂N(aryl)(alkyl)), ketone, aldehyde, ester, oxo, amino (primary, secondary or tertiary), -CO₂CH₃, -CONH₂, -OCH₂CONH₂, -NH₂, -OCHF₂, -OCF₃, -NH(alkyl), - N(alkyl)₂, -NH(aryl), -N(alkyl)(aryl), -N(aryl)₂, -CHO, -CO(alkyl), -CO(aryl), -CO₂(alkyl), or -CO₂(aryl). In some embodiments, the functional group is or comprises alkoxy, aryloxy, carboxy, alkoxycarbonyl, aminocarbonyl, alkylaminocarbonyl, dialkylaminocarbonyl, acyloxy, nitrile or halo. In other embodiments, none of R⁵, R⁶, R⁷ and R⁸ of formula (IX), (IX') or (XV) is or comprises a functional group. In other embodiments, none of R⁵, R⁶, R⁷ and R⁸ of formula (IX), (IX') or (XV) is or comprises alkoxy, aryloxy, carboxy, alkoxycarbonyl, aminocarbonyl, alkylaminocarbonyl, dialkylaminocarbonyl, acyloxy, nitrile or halo.

In some embodiments, the vinyl monomer is a substituted or unsubstituted olefin such as ethylene or styrene, vinyl halide, vinyl ether, acrylonitrile, acrylic ester, methacrylic ester, acrylamide, methacrylamide or a combination thereof. In other embodiments, the vinyl monomer is ethylene, an α-olefin or a combination thereof. Some non-limiting examples of suitable α-olefins include styrene, propylene, 1-butene, 1-hexane, 1-octene, 4-methyl-1-pentene, norbornene, 1-decene, 1,5-hexadiene and combinations thereof.

In some embodiments, the vinyl monomer is an aryl such as styrene, α-methyl styrene, or di-vinyl benzene. Additional examples include the functionalized vinyl aryls such as those disclosed by U.S. Patent No. 7,041,761.

In some embodiments, the farnesene interpolymers disclosed herein are derived from at least one farnesene and at least one olefin monomer. An olefin refers to an unsaturated hydrocarbon-based compound with at least one carbon-carbon double bond. In certain embodiments, the olefin is a conjugated diene. Depending on the selection of catalysts, any olefin may be used in embodiments of the invention. Some non-limiting examples of suitable olefins include C₂₋₂₀ aliphatic and C₈₋₂₀ aromatic compounds containing vinylic unsaturation, as well as cyclic compounds, such as cyclobutene, cyclopentene, dicyclopentadiene, and norbornene, including but not limited to, norbornene substituted in the 5 and 6 position with C₁₋₂₀ hydrocarbyl or cyclohydrocarbyl groups. Other non-limiting examples of suitable olefins include mixtures of such olefins as well as mixtures of such olefins with C₄₋₄₀ diolefin compounds.

Some non-limiting examples of suitable olefin or α-olefin monomers include styrene, ethylene, propylene, isobutylene, 1-butene, 1-pentene, 1-hexene, 1-heptene, 1-octene, 1-nonene, 1-decene, and 1-dodecene, 1-tetradecene, 1-hexadecene, 1-octadecene, 1-eicosene, 3-methyl-1-butene, 3-methyl-1-pentene, 4-methyl-1-pentene, 4,6-dimethyl-1-heptene, 4-vinylcyclohexene, vinylcyclohexane, norbornadiene, ethylidene norbornene, cyclopentene, cyclohexene, dicyclopentadiene, cyclooctene, C₄₋₄₀ dienes, including but not limited to 1,3-butadiene, 1,3-pentadiene, 1,4-hexadiene, 1,5-hexadiene, 1,7-octadiene, 1,9-decadiene, other C₄₋₄₀ α-olefins, and the like. In certain embodiments, the olefin monomer is propylene, 1-butene, 1-pentene, 1-hexane, 1-octane or a combination thereof.

The farnesene interpolymers disclosed herein may derived from a farnesene and styrene. The farnesene interpolymers may further comprise at least one C₂₋₂₀ olefin, at least one C₄₋₁₈ diolefin, at least one alkenylbenzene or a combination thereof. Suitable unsaturated comonomers useful for polymerizing with farnesene include, for example, ethylenically unsaturated monomers, polyenes such as conjugated or nonconjugated dienes, alkenylbenzenes, and the like. Examples of such comonomers include ethylene, C₂₋₂₀ olefins such as propylene, isobutylene, 1-butene, 1-hexene, 1-pentene, 4-methyl-1-pentene, 1-heptene, 1-octene, 1-nonene 1-decene, and the like. Other suitable monomers include styrene, halo- or alkyl-substituted styrenes, vinylbenzocyclobutane, 1,4-hexadiene, 1,7-octadiene, and cycloalkenes such as cyclopentene, cyclohexane and cyclooctene.

Some suitable non-conjugated diene monomers can be a straight chain, branched chain or cyclic hydrocarbon diene having from 6 to 15 carbon atoms. Some non-limiting examples of suitable non-conjugated dienes include straight chain acyclic dienes, such as 1,4-hexadiene, 1,6-octadiene, 1,7-octadiene, 1,9-decadiene, branched chain acyclic dienes, such as 5-methyl-1,4-hexadiene; 3,7-dimethyl-1,6-octadiene; 3,7-dimethyl-1,7-octadiene and mixed isomers of dihydromyricene and dihydroocinene, single ring alicyclic dienes, such as 1,3-cyclopentadiene; 1,4-cyclohexadiene; 1,5-cyclooctadiene and 1,5-cyclododecadiene, and multi-ring alicyclic fused and bridged ring dienes, such as tetrahydroindene, methyl tetrahydroindene, dicyclopentadiene, bicyclo-(2,2,1)-hepta-2,5-diene; alkenyl, alkylidene, cycloalkenyl and cycloalkylidene norbornenes, such as 5-methylene-2-norbomene (MNB); 5-propenyl-2-norbomene, 5-isopropylidene-2-norbomene, 5-(4-cyclopentenyl)-2-norbomene, 5-cyclohexylidene-2-norbornene, 5-vinyl-2-norbomene, and norbornadiene. Of the dienes typically used to prepare EPDMs, the particularly preferred dienes are 1,4-hexadiene (HD), 5-ethylidene-2-norbomene (ENB), 5-vinylidene-2-norbornene (VNB), 5-methylene-2-norbornene (MNB), and dicyclopentadiene (DCPD). In certain embodiments, the diene is 5-ethylidene-2-norbornene (ENB) or 1,4-hexadiene (HD). In other embodiments, the farnesene interpolymers are not derived from a polyene such as dienes, trienes, tetraenes and the like.

In some embodiments, the farnesene interpolymers are interpolymers of famesene, styrene, and a C₂₋₂₀ olefin. Some non-limiting examples of suitable olefins include ethylene, propylene, isobutylene, 1-butene, 1-pentene, 1-hexene, 4-methyl-1-pentene, and 1-octane. In some embodiments, the farnesene interpolymers disclosed herein are not derived from ethylene. In some embodiments, the farnesene interpolymers disclosed herein are not derived from one or more C₂₋₂₀ olefins.

In certain embodiments, the vinyl monomer does not comprise a terpene. In other embodiments, the vinyl monomer does not comprise a terpene selected from isoprene, dipentene, α-pinene, β-pinene, terpinolene, limonene (dipentene), terpinene, thujene, sabinene, 3-carene, camphene, cadinene, caryophyllene, myrcene, ocimene, cedrene, bisalbone, bisalbone, bisalbone, zingiberene, humulene, citronellol, linalool, geraniol, nerol, ipsenol, terpineol, D-terpineol-(4), dihydrocarveol, nerolidol, farnesol, eudesmol, citral, D-citronellal, carvone, D-pulegone, piperitone, carvenone, bisabolene, selinene, santalene, vitamin A, abietic acid or a combination thereof. In further embodiments, the vinyl monomer does not comprise an isoprene.

The farnesene interpolymers can be functionalized by incorporating at least one functional group in their polymer structure. Exemplary functional groups may includes, for example, ethylenically unsaturated mono- and di-functional carboxylic acids, ethylenically unsaturated mono- and di-functional carboxylic acid anhydrides, salts thereof and esters thereof. Such functional groups may be grafted to the farnesene interpolymers, or they may be copolymerized farnesene with an optional additional comonomer to form an interpolymer of farnesene, the functional comonomer and optionally other comonomer(s). Any means for grafting functional groups known to a skilled artisan can be used. One particularly useful functional group is maleic anhydride.

The amount of the functional group present in the functionalized farnesene interpolymer may vary. In some embodiments, the functional group is present in an amount of at least 1.0 wt.%, at least 2.5 wt.%, at least 5 wt.%, at least 7.5 wt%, or at least 10 wt.%, based on the total weight of the farnesene interpolymer. In other embodiments, the functional group is present in an amount of less than 40 wt.%, less than 30 wt.%, less than 25 wt.%, less than 20 wt.%, or less than 15 wt.%, based on the total weight of the farnesene interpolymer.

Any catalyst that can polymerize or copolymerize farnesene can be used for making the polyfarnesenes disclosed herein. Some non-limiting examples of suitable catalysts include organolithium reagents, Ziegler-Natta catalysts, Kaminsky catalysts and other metallocene catalysts. In some embodiments, the catalyst is a Ziegler-Natta catalyst, a Kaminsky catalyst, a metallocene catalyst or a combination thereof.

In some embodiments, the catalyst further comprises a cocatalyst. In further embodiments, the cocatalyst is a hydride, alkyl or aryl of a metal or a combination thereof. In still further embodiments, the metal is aluminum, lithium, zinc, tin, cadmium, beryllium or magnesium.

In some embodiments, the catalyst is an organolithium reagent. Any organolithium reagent that can act as a catalyst to polymerize olefins can be used herein. Some non-limiting examples of suitable organolithium reagents include n-butyllithium, *sec-*butyllithium or tert-butyllithium. Some non-limiting examples of suitable Lewis bases include TMEDA, PMDTA or sparteine. Some organolithium reagents are disclosed in Zvi Rappoport et al., "The Chemistry of Organolithium Compounds," Part 1 (2004) and Vol. 2 (2006).

In some embodiments, the catalyst is a mixture of an organolithium reagent and a Lewis base. Any Lewis base that can deaggregate organolithium reagents, making them more soluble and more reactive, can be used herein. An aggregated organolithium reagent generally has one lithium coordinating to more than one carbon atom and one carbon coordinating to more than one lithium atom. Some non-limiting examples of suitable Lewis bases include 1,2-bis(dimethylamino)ethane (also known as tetramethylethylenediamine or TMEDA), *N,N,N',N',N"*-pentamethyldiethylenetriamine (PMDTA), sparteine and combinations thereof.

In some embodiments, the catalyst is a Ziegler-Natta catalyst. Generally, Ziegler-Natta catalysts can be heterogeneous or homogeneous. In some embodiments, the Ziegler-Natta catalyst used for polymerizing the polyfarnesenes disclosed herein is a heterogeneous Ziegler-Natta catalyst. Some useful Ziegler-Natta catalysts are disclosed in J. Boor, "Ziegler-Natta Catalysts and Polymerizations," Saunders College Publishing, pp. 1-687 (1979); and Malcolm P. Stevens, "Polymer Chemistry, an Introduction," Third Edition, Oxford University Press, pp. 236-245 (1999).

Heterogeneous Ziegler-Natta catalysts generally comprise (1) a transition metal compound comprising an element from groups IV to VIII; and (2) an organometallic compound comprising a metal from groups I to III of the periodic table. The transition metal compound is referred as the catalyst while the organometallic compound is regarded as the cocatalyst or activator. The transition metal compound generally comprises a metal and one or more anions and ligands. Some non-limiting examples of suitable metals include titanium, vanadium, chromium, molybdenum, zirconium, iron and cobalt. Some non-limiting examples of suitable anions or ligands include halides, oxyhalides, alkoxy, acetylacetonyl, cyclopentadienyl, and phenyl.

Any cocatalyst or activator that can ionize the organometallic complex to produce an active olefin polymerization catalyst can be used herein. Generally, the organometallic cocatalysts are hydrides, alkyls, or aryls of metals, such as aluminum, lithium, zinc, tin, cadmium, beryllium, and magnesium. Some non-limiting examples of suitable cocatalysts include alumoxanes (methyl alumoxane (MAO), PMAO, ethyl alumoxane, diisobutyl alumoxane), alkylaluminum compounds (trimethylaluminum, triethylaluminum, diethyl aluminum chloride, trimethylaluminum, triisobutylaluminum, trioctylaluminum), diethylzinc, di(i-butyl)zinc, di(n-hexyl)zinc, and ethylzinc (t-butoxide) and the like. Other suitable cocatalysts include acid salts that contain non-nucleophilic anions. These compounds generally consist of bulky ligands attached to boron or aluminum. Some non-limiting examples of such compounds include lithium tetrakis(pentafluorophenyl)borate, lithium tetrakis(pentafluorophenyl)aluminate, anilinium tetrakis(pentalluorophenyl)borate, and the like. Some non-limiting examples of suitable cocatalysts also include organoboranes, which include boron and one or more alkyl, aryl, or aralkyl groups. Other non-limiting examples of suitable cocatalysts include substituted and unsubstituted trialkyl and triarylboranes such as tris(pentafluorophenyl)borane, triphenylborane, tri-n-octylborane, and the like. These and other suitable boron-containing cocatalysts or activators are described in U.S. Pat. Nos. 5,153,157, 5,198,401, and 5,241,025.

In certain embodiments, the Ziegler-Natta catalyst can be impregnated on a support material. Some suitable support materials are disclosed in Malcolm P. Stevens, "Polymer Chemistry, an Introduction," Third Edition, Oxford University Press, p. 251 (1999).

The support material is generally a material inert or substantially inert to olefin polymerization reactions. Non-limiting examples of suitable support materials include MgCl₂, MgO, alumina such as activated alumina and microgel alumina, silica, magnesia, kieselguhr, fuller's earth, clays, alumina silicates, porous rare earth halides and oxylalides, and combinations thereof. The support material can have a surface area between 5 m²/g and 450 m²/g, as determined by the BET (Brunauer-Emmet-Teller) methods of measuring surface area, as described by S. Brunauer, P. H. Emmett, and E. Teller, Journal of the American Chemical Society, 60, 309 (1938). In some embodiments, the surface area of the support material is between 10 m²/g and 350 m²/g. In further embodiments, the surface area of the support material is between 25 m²/g and 300 m²/g.

The support material can have an average particle size ranging from 20 to 300 microns, from 20 to 250 microns, from 20 to 200 microns, from 20 to 150 microns, from 20 to 120 microns, from 30 to 100 microns, or from 30 to 90 microns. The compacted or tamped bulk density of the support material can vary between 0.6 and 1.6 g/cc, between 0.7 and 1.5 g/cc, between 0.8 and 1.4 g/cc, or between 0.9 and 1.3 g/cc.

In certain embodiments, the catalyst used herein is or comprises a Kaminsky catalyst, also known as homogeneous Ziegler-Natta catalyst. The Kaminsky catalyst can be used to produce polyolefins such as the polyfarnesenes disclosed herein with unique structures and physical properties. Some Kaminsky catalysts or homogeneous Ziegler-Natta catalysts are disclosed in Malcolm P. Stevens, "Polymer Chemistry, an Introduction," Third Edition, Oxford University Press, pp. 245-251 (1999); and John Scheirs and Walter Kaminsky, "Metallocene-Based Polyolefins: Preparation, Properties, and Technology," Volume 1, Wiley (2000).

In some embodiments, the Kaminsky catalyst suitable for making the polyfarnesene disclosed herein comprises a transition-metal atom sandwiched between ferrocene ring structures. In other embodiments, the Kaminsky catalyst can be represented by the formula Cp₂MX₂, where M is a transition metal (*e.g.*, Zr, Ti or Hf); X is halogen (*e.g.*, Cl), alkyl or a combination thereof; and Cp is a ferrocenyl group. In further embodiments, the Kaminsky catalyst has formula (XVI): 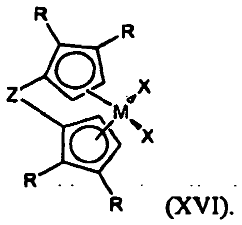 wherein Z is an optional divalent bridging group, usually C(CH₃)₂, Si(CH₃)₂, or CH₂CH₂; R is H or alkyl; M is a transition metal (*e.g.*, Zr, Ti or Hf); X is halogen (*e.g.*, Cl), alkyl or a combination thereof. Some non-limiting examples of Kaminsky catalysts have formulae (XVII) to (XIX): 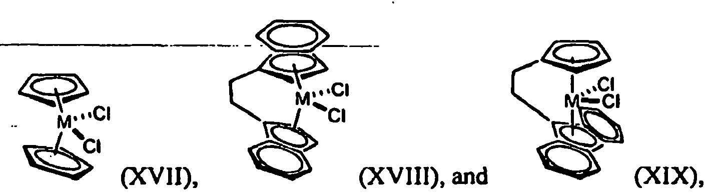 wherein M is Zr, Hf or Ti.

In some embodiments, a cocatalyst is used with the Kaminsky catalyst. The cocatalyst may be any of the cocatalyst disclosed herein. In certain embodiments, the cocatalyst is methylaminoxane (MAO). MAO is an oligomeric compound having a general formula (CH₃AlO)ₙ, where n is from 1 to 10. MAO may play several roles: it alkylates the metallocene precursor by replacing chlorine atoms with methyl groups; it produces the catalytic active ion pair Cp₂MCH₃⁺/MAO⁻, where the cationic moiety is considered responsible for polymerization and MAO⁻ acts as weakly coordinating anion. Some non-limiting examples of MAO include formulae (XX) to (XXI):

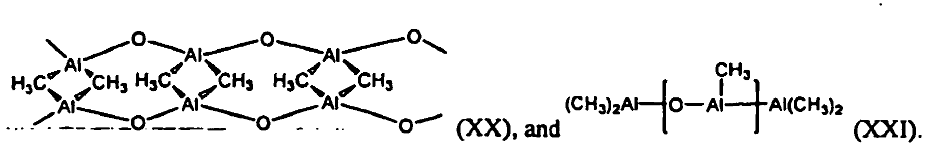

In certain embodiments, the catalyst for making the farnesene interpolymer disclosed herein is or comprises a metallocene catalyst. Some metallocene catalysts are disclosed in Tae Oan Ahn et al., "Modification of a Ziegler-Natta catalyst with a metallocene catalyst and its olefin polymerization behavior," Polymer Engineering and Science, 39(7), p. 1257 (1999); and John Scheirs and Walter Kaminsky, "Metallocene-Based Polyolefins: Preparation, Properties, and Technology," Volume 1, Wiley (2000).

In other embodiments, the metallocene catalyst comprises complexes with a transition metal centre comprising a transition metal, such as Ni and Pd, and bulky, neutral ligands comprising alpha-diimine or diketimine. In further embodiments, the metallocene catalyst has formula (XXII): 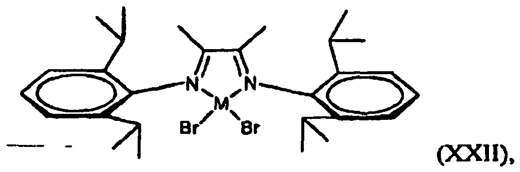 wherein M is Ni or Pd.

In some embodiments, the catalyst used herein is or comprises a metallocene catalyst bearing mono-anionic bidentate ligands. A non-limiting example of such a metallocene catalyst has structure (XXIII):

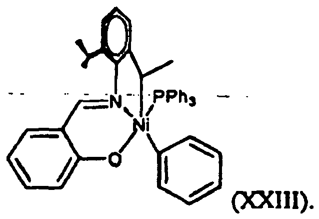

In other embodiments, the catalyst used herein is or comprises a metallocene catalyst comprising iron and a pyridyl is incorporated between two imine groups giving a tridentate ligand. A non-limiting example of such a metallocene catalyst has structure (XXIV):

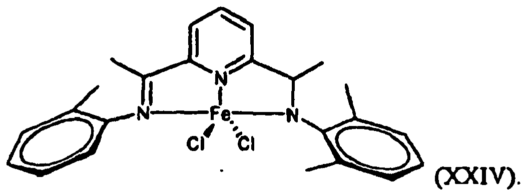

In some embodiments, the catalyst used herein is or comprises a metallocene catalyst comprising a salicylimine catalyst system based on zirconium. A non-limiting example of such a metallocene catalyst has structure (XXV):

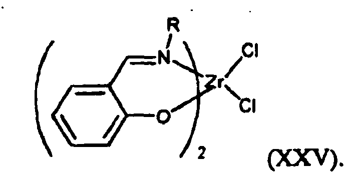

In some embodiments, the farnesene homopolymer disclosed herein is prepared by a process comprising the steps of:
(a) making a farnesene from a simple sugar or non-fermentable carbon source by using a microorganism; and
(b) polymerizing the farnesene in the presence of a catalyst disclosed herein.

In certain embodiments, the farnesene interpolymer disclosed herein is prepared by a process comprising the steps of:
(a) making a farnesene from a simple sugar or non-fermentable carbon source by using a microorganism; and
(b) copolymerizing the farnesene and at least one vinyl monomer in the presence of a catalyst disclosed herein.

In some embodiments, the polyfarnesene disclosed herein is prepared by polymerizing β-farnesene in the presence of a catalyst, wherein the amount of the *cis*-1,4-microstructure in the polyfarnesene is at most 80 wt.%, at most 75 wt.%, at most 70 wt.%, at most 65 wt.%, or at most 60 wt%, based on the total weight of the polyfamesene. In some embodiments, the β-farnesene is copolymerized with a vinyl monomer to form a farnesene copolymer. In other embodiments, the vinyl monomer is styrene. In further embodiments, the farnesene copolymer is a block copolymer.

In certain embodiments, the polyfarnesene disclosed herein is prepared by polymerizing an α-farnesene in the presence of a catalyst, wherein the amount of the *cis*-1,4-microstructure in the polyfarnesene is from 1 wt% to 99 wt.%, from 10 wt.% to 99 wt%, from 20 wt% to 99 wt.%, from 30 wt.% to 99 wt.% from 40 wt.% to 99 wt.%, from 50 wt.% to 99 wt.%, from 1 wt% to 99 wt.%, from 1 wt.% to 90 wt.%, from 1 wt.% to 80 wt.%, from 1 wt.% to 70 wt.%, or from 1 wt.% to 60 wt.%, based on the total weight of the polyfarnesene. In some embodiments, the α-farnesene is copolymerized with a vinyl monomer to form a farnesene copolymer. In other embodiments, the vinyl monomer is styrene. In further embodiments, the farnesene copolymer is a block copolymer.

In some embodiments, the polyfarnesene disclosed herein can be hydrogenated partially or completely by any hydrogenating agent known to a skilled artisan. For example, a saturated polyfarnesene can be prepared by (a) polymerizing a farnesene disclosed herein in the presence of a catalyst disclosed herein to form a polyfarnesene; and (b) hydrogenating at least a portion of the double bonds in the polyfarnesene in the presence of a hydrogenation reagent. In some embodiments, the farnesene is copolymerized with a vinyl monomer disclosed herein to form a farnesene copolymer. In other embodiments, the vinyl monomer is styrene. In further embodiments, the farnesene copolymer is a block copolymer. In still further embodiments, the farnesene is α-farnesene or β-farnesene or a combination thereof.

In certain embodiments, the hydrogenation reagent is hydrogen in the presence of a hydrogenation catalyst. In some embodiments, the hydrogenation catalyst is Pd, Pd/C, Pt, PtO₂, Ru(PPh₃)₂Cl₂, Raney nickel or a combination thereof. In one embodiment, the catalyst is a Pd catalyst. In another embodiment, the catalyst is 5% Pd/C. In a further embodiment, the catalyst is 10% Pd/C in a high pressure reaction vessel and the hydrogenation reaction is allowed to proceed until completion. Generally, after completion, the reaction mixture can be washed, concentrated, and dried to yield the corresponding hydrogenated product. Alternatively, any reducing agent that can reduce a C=C bond to a C-C bond can also be used. For example, the polyfarnesene can be hydrogenated by treatment with hydrazine in the presence of a catalyst, such as 5-ethyl-3-methyllumiflavinium perchlorate, under an oxygen atmosphere to give the corresponding hydrogenated products. The reduction reaction with hydrazine is disclosed in Imada et al., J. Am. Chem. Soc., 127, 14544-14545 (2005).

In some embodiments, at least a portion of the C=C bonds of the polyfarnesene disclosed herein is reduced to the corresponding C-C bonds by hydrogenation in the presence of a catalyst and hydrogen at room temperature. In other embodiments, at least a portion of the C=C bonds of one or more of formulae (1')-(111'). (V')-(VII'), and (XI)-(XIV) and stereoisomers thereof is reduced to the corresponding C-C bonds by hydrogenation in the presence of a catalyst and hydrogen at room temperature. In further embodiments, the hydrogenation catalyst is 10% Pd/C.

In certain embodiments, the vinyl monomer is styrene. In some embodiments, the farnesene is α-farnesene or β-farnesene or a combination thereof. In other embodiments, the farnesene is prepared by using a microorganism. In further embodiments, the farnesene is derived from a simple sugar or non-fermentable carbon source.

### Farnesene

The farnesene can be derived from any source or prepared by any method known to a skilled artisan. In some embodiments, the farnesene is derived from a chemical source (*e.g*., petroleum or coal) or obtained by a chemical synthetic method. In other embodiments, the farnesene is prepared by fractional distillation of petroleum or coal tar. In further embodiments, the farnesene is prepared by any known chemical synthetic method. One non-limiting example of suitable chemical synthetic method includes dehydrating nerolidol with phosphoryl chloride in pyridine as described in the article by Anet E. F. L. J., "Synthesis of (EZ)-α-,(ZZ)-α-, and (Z)-β-farnesene," Aust. J. Chem., 23(10), 2101-2108 (1970).

In some embodiments, the farnesene can be obtained or derived from naturally occurring terpenes that can be produced by a wide variety of plants, such as *Copaifera langsdorfii,* conifers, and spurges; insects, such as swallowtail butterflies, leaf beetles, termites, and pine sawflies; and marine organisms, such as algae, sponges, corals, mollusks, and fish.

*Copaifera longsdorfii* or Copaifera tree is also known as the diesel tree and kerosene tree. It has many names in local languages, including *kupa'y, cabismo,* and *copaúva.* Copaifera tree may produce a large amount of terpene hydrocarbons in its wood and leaves. Generally, one Copaifera tree can produce from 30 to 40 liters of terpene oil per year.

Terpene oils can also be obtained from conifers and spurges. Conifers belong to the plant division Pinophyta or Coniferae and are generally cone-bearing seed plants with vascular tissue. The majority of conifers are trees, but some conifers can be shrubs. Some non-limiting examples of suitable conifers include cedars, cypresses, douglas-firs, firs, junipers, kauris, larches, pines, redwoods, spruces, and yews. Spurges, also known as Euphorbia, are a very diverse worldwide genus of plants, belonging to the spurge family (Euphorbiaceae). Consisting of about 2160 species, spurges are one of the largest genera in the plant kingdom.

The farnesene is a sesquiterpene which are part of a larger class of compound called terpenes. A large and varied class of hydrocarbons, terpenes include hemiterpenes, monoterpenes, sesquiterpenes, diterpenes, sestenerpenes, triterpenes, tetraterpenes, and polyterpenes. As a result, the farnesene can be isolated or derived from terpene oils for use in the present invention.

In certain embodiments, the farnesene is derived from a biological source. In other embodiments, the farnesene can be obtained from a readily available, renewable carbon source. In further embodiments, the farnesene is prepared by contacting a cell capable of making a farnesene with a carbon source under conditions suitable for making the farnesene.

Any carbon source that can be converted into one or more isoprenoid compounds can be used herein. In some embodiments, the carbon source is a sugar or a non-fermentable carbon source. The sugar can be any sugar known to those of skill in the art. In certain embodiments, the sugar is a monosaccharide, disaccharide, polysaccharide or a combination thereof. In other embodiments, the sugar is a simple sugar (a monosaccharide or a disaccharide). Some non-limiting examples of suitable monosaccharides include glucose, galactose, mannose, fructose, ribose and combinations thereof. Some non-limiting examples of suitable disaccharides include sucrose, lactose, maltose, trehalose, cellobiose and combinations thereof. In still other embodiments, the simple sugar is sucrose. In certain embodiments, the bioengineered fuel component can be obtained from a polysaccharide. Some non-limiting examples of suitable polysaccharides include starch, glycogen, cellulose, chitin and combinations thereof.

The sugar suitable for making the farnesene can be found in a wide variety of crops or sources. Some non-limiting examples of suitable crops or sources include sugar cane, bagasse, miscanthus, sugar beet, sorghum, grain sorghum, switchgrass, barley, hemp, kenaf, potatoes, sweet potatoes, cassava, sunflower, fruit, molasses, whey or skim milk, corn, stover, grain, wheat, wood, paper, straw, cotton, many types of cellulose waste, and other biomass. In certain embodiments, the suitable crops or sources include sugar cane, sugar beet and corn. In other embodiments, the sugar source is cane juice or molasses.

A non-fermentable carbon source is a carbon source that cannot be converted by the organism into ethanol. Some non-limiting examples of suitable non-fermentable carbon sources include acetate and glycerol.

In certain embodiments, the farnesene can be prepared in a facility capable of biological manufacture of C₁₅ isoprenoids. The facility can comprise any structure useful for preparing the C₁₅ isoprenoids, such as α-farnesene, β-farnesene, nerolidol or farnesol, using a microorganism. In some embodiments, the biological facility comprises one or more of the cells disclosed herein. In other embodiments, the biological facility comprises a cell culture comprising at least a C₁₅ isoprenoid in an amount of at least 1 wt.%, at least 5 wt.%, at least 10 wt.%, at least 20 wt.%, or at least 30 wit.%, based on the total weight of the cell culture. In further embodiments, the biological facility comprises a fermentor comprising one or more cells described herein.

Any fermentor that can provide cells or bacteria a stable and optimal environment in which they can grow or reproduce can be used herein. In some embodiments, the fermentor comprises a culture comprising one or more of the cells disclosed herein. In other embodiments, the fermentor comprises a cell culture capable of biologically manufacturing farnesyl pyrophosphate (FPP). In further embodiments, the fermentor comprises a cell culture capable of biologically manufacturing isopentenyl diphosphate (IPP). In certain embodiments, the fermentor comprises a cell culture comprising at least a C₁₅ isoprenoid in an amount of at least 1 wt.%, at 5 wt.%, at least 10 wt.%, at least 20 wt.%, or at least 30 wt.%, based on the total weight of the cell culture.

The facility can further comprise any structure capable of manufacturing the fuel component or fuel additive from the C₁₅ isoprenoid, such as α-farnesene, β-farnesene, nerolidol or farnesol. The structure may comprise a reactor for dehydrating the nerolidol or farnesol to α-farnesene or β-farnesene. Any reactor that can be used to convert an alcohol into an alkene under conditions known to skilled artisans may be used herein. The reactor may comprise a dehydrating catalyst disclosed herein. In some embodiments, the structure further comprises a mixer, a container, and a mixture of the dehydrating products from the dehydrating step.

The biosynthetic process of making C₁₅ isoprenoid compounds are disclosed in U.S. Patent No. 7,399,323; U.S. Application Number US 2008/0274523; and PCT Publication Numbers WO 2007/140339 and WO 2007/139924. .

### α-Farnesene

α-Farnesene, whose structure is 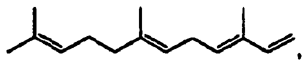 is found in various biological sources including, but not limited to, the Dufour's gland in ants and in the coating of apple and pear peels. Biochemically, α-farnesene is made from FPP by a-farnesene synthase. Some non-limiting examples of suitable nucleotide sequences that encode such an enzyme include (DQ309034; *Pyrus communis cultivar d'Anjou*) and (AY182241; *Malus domestica*)*.* See Pechouus et al., Planta 219(1):84-94 (2004).

### β-Farnesene

β-Farnesene, whose structure is 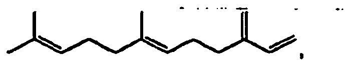 is found in various biological sources including, but not limited to, aphids and essential oils such as peppermint oil. In some plants such as wild potato, β-farnesene is synthesized as a natural insect repellent. Biochemically, β-farnesene is made from FPP by β-farnesene synthase. Some non-limiting examples of suitable nucleotide sequences that encode such an enzyme include (AF024615; *Mentha x piperita*) and (AY835398; *Artemisia annua*)*.* See Picaud et al., Phytochemistry 66(9): 961-967 (2005).

### Farnesol

Farnesol, whose structure is 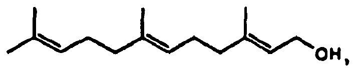 is found in various biological sources including insects and essential oils from cintronella, neroli, cyclamen, lemon grass, tuberose, and rose. Biochemically, farnesol is made from FPP by a hydroxylase such as farnesol synthase. Some non-limiting examples of suitable nucleotide sequences that encode such an enzyme include (AF529266; *Zea mays*) and (YDR481C; *Saccharomyces cerevisiae*)*.* See Song, L., Applied Biochemistry and Biotechnology 128:149-158 (2006).

### Nerolidol

Nerolidol, whose structure is 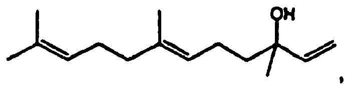 is also known as peruviol which is found in various biological sources including essential oils from neroli, ginger, jasmine, lavender, tea tree, and lemon grass. Biochemically, nerolidol is made from FPP by a hydroxylase such as nerolidol synthase. A non-limiting example of a suitable nucleotide sequence that encodes such an enzyme includes AF529266 from *Zea mays* (maize; gene tps1).

The farnesol and nerolidol disclosed herein may be converted into α-farnesene, β-farnesene or a combination thereof by dehydration with a dehydrating agent or an acid catalyst. Any dehydrating agent or an acid catalyst that can convert an alcohol into an alkene can be used herein. Some non-limiting examples of suitable dehydrating agents or acid catalysts include phosphoryl chloride, anhydrous zinc chloride, phosphoric acid and sulfuric acid.

### General Procedures of Making Polyfarnesenes

The polymerization of a farnesene or the copolymerization of a farnesene with a vinyl comonomer can be performed over a wide temperature range. In certain embodiments, the polymerization temperature is from -30°C to 280°C, from 30°C to 180 °C, or from 60 °C to 100 °C. The partial pressures of the vinyl comonomers can range from 15 psig (0.1 MPa) to 50,000 psig (245 MPa), from 15 psig (0.1 MPa) to 25,000 psig (172.5 MPa), from 15 psig (0.1 MPa) to 10,000 psig (69 MPa), from 15 psig (0.1 MPa) to 5,000 psig (34.5 MPa) or from 15 psig (0.1 MPa) to 1,000 psig (6.9 MPa).

The concentration of the catalyst used for making the polyfarnesenes disclosed herein depends on many factors. In some embodiment, the concentration ranges from 0.01 micromoles per liter to 100 micromoles per liter. The polymerization time depends on the type of process, the catalyst concentration, and other factors. Generally, the polymerization time is within several minutes to several hours.

A non-limiting example of solution polymerization procedure for farnesene homopolymer is outlined below. A farnesene such as β-farnesene can be added to a solvent such as cyclohexane to form a solution in a reactor which may be optionally under a nitrogen or argon atmosphere. The solution can be dried over a drying agent such as molecular sieves. A catalyst such as organolithium reagent can be added into the reactor, and then the reactor is heated to an elevated temperature until all or a substantial portion of farnesene is consumed. The farnesene homopolymer can then be precipitated from the reaction mixture and dried in a vacuum oven.

A non-limiting example of solution polymerization procedure for farnesene interpolymer is outlined below. A farnesene such as β-farnesene can be added to a solvent such as cyclohexane to form a farnesene solution in a reactor optionally under a nitrogen or argon atmosphere. The farnesene solution can be dried over a drying agent such as molecular sieves. In a second reactor optionally under nitrogen or argon atmosphere, a solution of styrene in cyclohexane with 10% is similarly prepared and dried over a drying agent such as molecular sieves. The styrene is polymerized by a catalyst such as organolithium reagent at an elevated temperature until all or a substantial portion of styrene is consumed. Then, the farnesene solution is transferred to the second reactor. The reaction is allowed to react until all or a substantial portion of farnesene is consumed. Then a dichlorosilane coupling agent (*e.g.*, dichlorodimethylsilane in 1,2-dichloroethane) is then added into the second reactor to form a farnesene interpolymer.

### Polyfarnesene Compositions

The polyfarnesene disclosed herein can be used to prepare useful compositions or polyfarnesene compositions such as adhesives compositions. The polyfarnesene compositions comprise the polyfarnesene and an tackifier. In some embodiments, the polyfarnesene compositions comprise an optional second polymer: . In certain embodiments, the polyfarnesene compositions do not comprise a second polymer.

The second polymer can be a vinyl polymer or copolymer, a non-vinyl polymer or copolymer, or a combination thereof. Some non-limiting examples of vinyl polymers and copolymers are disclosed in Malcolm P. Stevens, "Polymer Chemistry, an Introduction," Third Edition, Oxford University Press, pp. 17-21 and 167-279 (1999). Some non-limiting examples of suitable second polymer include a polyolefin, polyurethane, polyester, polyamide, styrenic polymer, phenolic resin, polyacrylate, polymethacrylate or a combination thereof.

In certain embodiments, the ratio of the farnesene interpolymer to the second polymer is from 1:99 to 99:1, from 1 :50 to 50:1, from 1:25 to 25:1 or from 1:10 to 10:1.

In some embodiments, the second polymer is a polyolefin (*e.g*., polyethylene, polypropylene, an ethylene/α-olefin interpolymer, a copolymer of ethylene and propylene, and a copolymer of ethylene and vinyl acetate (EVA)), polyurethane, polyester, polyamide, styrenic polymer (*e.g.*, polystyrene, poly(acrylonitrile-butadiene-styrene), poly(styrene-butadiene-styrene) and the like), phenolic resin, polyacrylate, polymethacrylate or a combination thereof. In some embodiments, the second polymer is polyethylene, polypropylene, polystyrene, a copolymer of ethylene and vinyl acetate, poly(acrylonitrile-butadiene-styrene), poly(styrene-butadiene-styrene) or a combination thereof. The second polymer may be blended with the farnesene interpolymer before it is added to the polyfarnesene composition. In some embodiments, the second polymer is added directly to the polyfarnesene composition without pre-blending with the farnesene interpolymer.

The weight ratio of the polyfarnesene to the second polymer, such as EVA, in the polyfarnesene composition can be between 1:99 and 99:1, between 1:50 and 50:1, between 1: 25 and 25:1, between 1:10 and 10:1, between 1:9 and 9:1, between 1:8 and 8:1, between 1:7 and 7:1, between 1:6 and 6:1, between 1:5 and 5:1, between 1:4 and 4:1, between 1:3 and 3:1, between 1:2 and 2:1, between 3:7 and 7:3 or between 2:3 and 3:2.

In some embodiments, the second polymer is a polyolefin. Any polyolefin that is partially or totally compatible with the farnesene interpolymer may be used. Non-limiting examples of suitable polyolefins include polyethylenes; polypropylenes; polybutylenes (*e.g*., polybutene-1); polypentene-1; polyhexene-1; polyoctene-1; polydecene-1; poly-3-methylbutene-1; poly-4-methylpentene-1; polyisoprene; polybutadiene; poly-1,5-hexadiene; interpolymers derived from olefins; interpolymers derived from olefins and other polymers such as polyvinyl chloride, polystyrene, polyurethane, and the like; and mixtures thereof. In some embodiments, the polyolefin is a homopolymer such as polyethylene, polypropylene, polybutylene, polypentene-1, poly-3-methylbutene-1, poly-4-methylpentene-1, polyisoprene, polybutadiene, poly-1,5-hexadiene, polyhexene-1, polyoctene-1 and polydecene-1.

Some non-limiting examples of suitable polyethylenes include ultra low density polyethylene (ULDPE), linear low density polyethylene (LLDPE), low density polyethylene (LDPE), medium density polyethylene (MDPE), high density polyethylene (HDPE), high molecular weight high density polyethylene (HMW-HDPE), ultra high molecular weight polyethylene (UHMW-PE) and combinations thereof. Some non-limiting examples of polypropylenes-include low density polypropylene (LDPP), high density polypropylene (HDPP), high-melt strength polypropylene (HMS-PP) and combination thereof. In some embodiments, the second polymer is or comprises high-melt-strength polypropylene (HMS-PP), low density polyethylene (LDPE) or a combination thereof.

Any material that can be added to an elastomer to produce an adhesive can be used herein as a tackifier. Some non-limiting examples of tackifiers include a natural and modified resin; a glycerol or pentaerythritol ester of natural or modified rosin; a copolymer or terpolymer of natured terpene; a polyterpene resin or a hydrogenated polyterpene resin; a phenolic modified terpene resin or a hydrogenated derivative thereof; an aliphatic or cycloaliphatic hydrocarbon resin or a hydrogenated derivative thereof; an aromatic hydrocarbon resin or a hydrogenated derivative thereof; an aromatic modified aliphatic or cycloaliphatic hydrocarbon resin or a hydrogenated derivative thereof; or a combination thereof. In certain embodiments, the tackifier has a ring and ball (R&B) softening point equal to or greater than 60°C, 70°C, 75°C, 80°C, 85°C, 90°C or 100°C, as measured in accordance with ASTM 28-67, which is incorporated herein by reference. In certain embodiments, the tackifier has a R&B softening point equal to or greater than 80°C, as measured in accordance with ASTM 28-67.

In certain embodiments, the amount of tackifier in the compositions disclosed herein is from 0.1 wt.% to 70 wt.%, from 0.1 wt.% to 60 wt.%, from 1 wt.% to 50 wt.%, or from 0.1wt.% to 40 wt.% or from 0.1 wt.% to 30 wt% or from 0.1 wt.% to 20 wt.%, or from 0.1 wt% to 10 wt%, based on the total weight of the composition. In other embodiments, the amount of tackifier in the compositions disclosed herein is from 1 wt.% to 70 wt%, from 5 wt.% to 70 wt.%, from 10 wt.% 70 wt.%, from 15 wt.% to 70 wt.%, from 20 wt.% to 70 wt.%, or from 25 wt.% to 70 wt%, based on the total weight of the composition.

Optionally, the compositions disclosed herein comprise at least another additive for the purposes of improving and/or controlling the processibility, appearance, physical, chemical, and/or mechanical properties of the polyfarnesene compositions. In some embodiments, the compositions do not comprise an additive. Any plastics additive known to a person of ordinary skill in the art may be used in the compositions disclosed herein. Non-limiting examples of suitable additives include plasticizers, oils, waxes, antioxidants, UV stabilizers, colorants or pigments, fillers, flow aids, coupling agents, crosslinking agents, surfactants, solvents, and combinations thereof. In certain embodiments, the additive is plasticizer, such as a mineral oil, liquid polybutene or a combination thereof.

The total amount of the additives can range from about greater than 0 to 80%, from 0.001 % to 70%, from 0.01 % to 60%, from 0.1 % to 50%, from 1 % to 40%, or from 10 % to 50% of the total weight of the polyfarnesene composition. The amount of each of the additives can range from about greater than 0 to 25%, from 0.001 % to 20%, from 0.01 % to 15%, from 0.1 % to 10%, from 0.1 % to 5%, or from 0.1 % to 2.5% of the total weight of the polyfarnesene composition. Some polymer additives have been described in Zweifel Hans et al., "Plastics Additives Handbook" Hanser Gardner Publications, Cincinnati, Ohio, 5th edition (2001),

Optionally, the compositions disclosed herein can comprise a wax, such as a petroleum wax, a low molecular weight polyethylene or polypropylene, a synthetic wax, a polyolefin wax, a beeswax, a vegetable wax, a soy wax, a palm wax, a candle wax for an ethylene/α-olefin interpolymer having a melting point of greater than 25 °C. In certain embodiments, the wax is a low molecular weight polyethylene or polypropylene having a number average molecular weight of 400 to 6,000 g/mole. The wax can be present from 10% to 50% or 20% to 40% by weight of the total composition.

Optionally, the compositions disclosed herein can comprise a plasticizer. In general, a plasticizer is a chemical that can increase the flexibility and lower the glass transition temperature of polymers. Any plasticizer known to a person of ordinary skill in the art may be added to the polyfarnesene compositions disclosed herein. Non-limiting examples of plasticizers include mineral oils, abietates, adipates, alkyl sulfonates, azelates, benzoates, chlorinated paraffins, citrates, epoxides, glycol ethers and their esters, glutarates, hydrocarbon oils, isobutyrates, oleates, pentaerythritol derivatives, phosphates, phthalates, esters, polybutenes, ricinoleates, sebacates, sulfonamides, tri- and pyromellitates, biphenyl derivatives, stearates, difuran diesters, fluorine-containing plasticizers, hydroxybenzoic acid esters, isocyanate adducts, multi-ring aromatic compounds, natural product derivatives, nitriles, siloxane based plasticizer, tar based products, thioesters and combinations thereof. Where used, the amount of the plasticizer in the polyfarnesene composition can be from greater than 0 to 15 wt.%, from 0.5 wt.% to 10 wt.%, or from 1 wt% to 5 wt.% of the total weight of the polyfarnesene composition. Some plasticizers have been described in George Wypych, "Handbook of Plasticizers," ChemTec Publishing, Toronto-Scarborough, Ontario (2004).

In some embodiments, the compositions disclosed herein optionally comprise an antioxidant that can prevent the oxidation of polymer components and organic additives in the polyfarnesene compositions. Any antioxidant known to a person of ordinary skill in the art may be added to the polyfarnesene compositions disclosed herein. Non-limiting examples of suitable antioxidants include aromatic or hindered amines such as alkyl diphenylamines, phenyl-α-naphthylamine, alky or aralkyl substituted phenyl-α-naphthylamine, alkylated p-phenylene diamines, tetramethyl-diaminodiphenylamine and the like; phenols such as 2,6-di-t-butyl-4-methylphenol; 1,3,5-trimethyl-2,4,6-tris(3',5'-di-t-butyl-4'-hydroxybenzyl)benzene; tetrakis[(methylene(3,5-di-t-butyl-4-hydroxyhydrocinnamate)]methane (*e.g*., IRGANOX^{™} 1010, from Ciba Geigy, New York); acryloyl modified phenols; octadecyl-3,5-di-t-butyl-4-hydroxycinnamate (*e.g*., IRGANOX^{™} 1076, commercially available from Ciba Geigy); phosphites and phosphonites; hydroxylamines; benzofuranone derivatives; and combinations thereof. Where used, the amount of the antioxidant in the polyfarnesene composition can be from about greater than 0 to 5 wt.%, from 0.0001 to 2.5 wt.%, from 0.001 wt.% to 1 wt.%, or from 0.001 wt.% to 0.5 wt.% of the total weight of the polyfarnesene composition. Some antioxidants have been described in Zweifel Hans et al., "Plastics Additives Handbook," Hanser Gardner Publications, Cincinnati, Ohio, 5th edition, Chapter 1, pages 1-140 (2001).

In other embodiments, the compositions disclosed herein optionally comprise an UV stabilizer that may prevent or reduce the degradation of the polyfarnesene compositions by UV radiations. Any UV stabilizer known to a person of ordinary skill in the art may be added to the polyfarnesene compositions disclosed herein. Non-limiting examples of suitable UV stabilizers include benzophenones, benzotriazoles, aryl esters, oxanilides, acrylic esters, formamidines, carbon black, hindered amines, nickel quenchers, hindered amines, phenolic antioxidants, metallic salts, zinc compounds and combinations thereof. Where used, the amount of the UV stabilizer in the polyfarnesene composition can be from about greater than 0 to 5 wt.%, from 0.01 wt.% to 3 wt.%, from 0.1 wt% to 2 wt.%, or from 0.1 wt.% to 1 wt.% of the total weight of the polyfarnesene composition. Some UV stabilizers have been described in Zweifel Hans et al., "Plastics Additives Handbook," Hanser Gardner Publications, Cincinnati, Ohio, 5th edition, Chapter 2, pages 141-426 (2001)_{.}

In further embodiments, the compositions disclosed herein optionally comprise a colorant or pigment that can change the look of the polyfarnesene compositions to human eyes. Any colorant or pigment known to a person of ordinary skill in the art may be added to the polyfarnesene compositions disclosed herein. Non-limiting examples of suitable colorants or pigments include inorganic pigments such as metal oxides such as iron oxide, zinc oxide, and titanium dioxide, mixed metal oxides, carbon black, organic pigments such as anthraquinonesTanthantnrohes, azo and monoazo compounds, arylamides, benzimidazolones, BONA lakes, diketopyrrolo-pyrroles, dioxazines, disazo compounds, diarylide compounds, flavanthrones, indanthrones, isoindolinones, isoindolines, metal complexes, monoazo salts, naphthols, b-naphthols, naphthol AS, naphthol lakes, perylenes, perinones, phthalocyanines, pyranthrones, quinacridones, and quinophthalones, and combinations thereof. Where used, the amount of the colorant or pigment in the polyfarnesene composition can be from about greater than 0 to 10 wt.%, from 0.1 wt.% to 5 wt.%, or from 0.25 wt.% to 2 wt.% of the total weight of the polyfarnesene composition. Some colorants have been described in Zweifel Hans et al., "Plastics Additives Handbook," Hanser Gardner Publications, Cincinnati, Ohio, 5th edition, Chapter 15, pages 813-882 (2001) .

Optionally, the compositions disclosed herein can comprise a filler which can be used to adjust, *inter alia,* volume, weight, costs, and/or technical performance. Any filler known to a person of ordinary skill in the art may be added to the polyfarnesene compositions disclosed herein. Non-limiting examples of suitable fillers include talc, calcium carbonate, chalk, calcium sulfate, clay, kaolin, silica, glass, fumed silica, mica, wollastonite, feldspar, aluminum silicate, calcium silicate, alumina, hydrated alumina such as alumina trihydrate, glass microsphere, ceramic microsphere, thermoplastic microsphere, barite, wood flour, glass fibers, carbon fibers, marble dust, cement dust, magnesium oxide, magnesium hydroxide, antimony oxide, zinc oxide, barium sulfate, titanium dioxide, titanates and combinations thereof. In some embodiments, the filler is barium sulfate, talc, calcium carbonate, silica, glass, glass fiber, alumina, titanium dioxide, or a mixture thereof. In other embodiments, the filler is talc, calcium carbonate, barium sulfate, glass fiber or a mixture thereof. Where used, the amount of the filler in the polyfarnesene composition can be from about greater than 0 to 80 wt.%, from _{0.1} wt.% to 60 wt.%, from 0.5 wt.% to 40 wt.%, from 1 wt.% to 30 wt.%, or from 10 wt.% to 40 wt.% of the total weight of the polyfarnesene composition. Some fillers have been disclosed in U.S. Patent No. 6,103,803 and Zweifel Hans et al., "Plastics Additives Handbook," Hanser Gardner Publications, Cincinnati, Ohio, 5th edition, Chapter 17, pages 901-948 (2001) .

Optionally, the polyfarnesene compositions disclosed herein may be crosslinked, partially or completely. When crosslinking is desired, the polyfarnesene compositions disclosed herein comprise a cross-linking agent that can be used to effect the cross-linking of the polyfarnesene compositions, thereby increasing their modulus and stiffness, among other things. An advantage of a polyfarnesene composition is that crosslinking can occur in its side chains instead of the polymer backbone like other polymers such as polyisoprene and polybutadiene. Any cross-linking agent known to a person of ordinary skill in the art may be added to the polyfarnesene compositions disclosed herein. Non-limiting examples of suitable cross-linking agents include organic peroxides *(e.g*., alkyl peroxides, aryl peroxides, peroxyesters, peroxycarbonates, diacylperoxides, peroxyketals, and cyclic peroxides) and silanes (*e.g*., vinyltrimethoxysilane, vinyltriethoxysilane, vinyltris(2-methoxyethoxy)silane, vinyltriacetoxysilane, vinylmethyldimethoxysilane, and 3-methacryloyloxypropyltrimethoxysilane). Where used, the amount of the cross-linking agent in the polymer composition can be from about greater than 0 to 20 wt.%, from 0.1 wt.% to 15 wt.%, or from 1 wt.% to 10 wt.% of the total weight of the polymer composition. Some suitable cross-linking agents have been disclosed in Zweifel Hans et al., "Plastics Additives Handbook," Hanser Gardner Publications, Cincinnati, Ohio, 5th edition, Chapter 14, pages 725-812 (2001).

In some embodiments, the farnesene interpolymers disclosed herein includes farnesene-modified polymers prepared by copolymerizing one or more farnesene with one or more vinyl monomers. In certain embodiments, the unmodified polymer derived from the one or more vinyl monomers can be any known olefin homopolymer or interpolymer. In further embodiments, none of the one or more other vinyl monomers has an unsaturated side chain capable of reacting with a cross-linking agent. Because of the unsaturated side chains derived from the farnesene, the farnesene-modified polymer disclosed herein can be crosslinked by a cross-linking agent disclosed herein.

In certain embodiments, the amount of the farnesene in the farnesene-modified polymer disclosed herein is from 1 wt% to 20 wt.%, from 1 wt.% to 10 wt.%, from 1 wt.% to 7.5 wt.%, from 1 wt.% to 5 wt.%, from 1 wt% to 4 wt.%, from 1 wt.% to 3 wt.%, or from 1 wt.% to 2 wt%, based on the total weight of the farnesene-modified polymer. In other embodiments, the amount of the one or more other vinyl monomers in the farnesene-modified polymer disclosed herein is from 80 wt.% to 99 wt.%, from 90 wt.% to 99 wt.%, from 92.5 wt.% to 99 wt.%, from 95 wt.% to 99 wt.%, from 96 wt.% to 99 wt%, from 97 wt.% to 99 wt.%, or from 98 wt.% to 99 wt.%, based on the total weight of the farnesene-modified polymer.

The cross-linking of the polyfarnesene compositions can also be initiated by any radiation means known in the art, including, but not limited to, electron-beam irradiation, beta irradiation, gamma irradiation, corona irradiation, and UV radiation with or without cross-linking catalyst. U.S. Patent Application No. 10/086,057 (published as US2002/0132923 A1) and U.S. Patent No. 6,803,014 disclose electron-beam irradiation methods that can be used in embodiments of the invention.

Irradiation may be accomplished by the use of high energy, ionizing electrons, ultra violet rays, X-rays, gamma rays, beta particles and the like and combination thereof. Preferably, electrons are employed up to 70 megarads dosages. The irradiation source can be any electron beam generator operating in a range of 150 kilovolts to 6 megavolts with a power output capable of supplying the desired dosage. The voltage can be adjusted to appropriate levels which may be, for example, 100,000, 300,000, 1,000,000 or 2,000,000 or 3,000,000 or 6,000,000 or higher or lower. Many other apparati for irradiating polymeric materials are known in the art. The irradiation is usually carried out at a dosage between 3 megarads to 35 megarads, preferably between 8 to 20 megarads. Further, the irradiation can be carried out conveniently at room temperature, although higher and lower temperatures, for example, 0° C to 60° C, may also be employed. Preferably, the irradiation is carried out after shaping or fabrication of the article. Also, in a preferred embodiment, the farnesene interpolymer which has been incorporated with a pro-rad additive is irradiated with electron beam radiation at 8 to 20 megarads.

Crosslinking can be promoted with a crosslinking catalyst, and any catalyst that will provide this function can be used. Suitable catalysts generally include organic bases; carboxylic acids; organometallic compounds including organic titanates and complexes or carboxylates of lead, cobalt, iron, nickel, zinc and tin; dibutyltindilautate, dioctyltinmaleate, dibutyltindiacetate, dibutyltindioctoate, stannous acetate, stannous octoate, lead naphthenate, zinc caprylate, cobalt naphthenate; and the like. The catalyst (or mixture of catalysts) is present in a catalytic amount, typically between 0.015 and 0.035 phr.

### Blending of the Ingredients Of the Polyfarnesen Compositions

The ingredients (*i.e.*, the polyfamesene, the optional second polymer and additives) of the polyfarnesene compositions disclosed herein, can be mixed or blended using methods known to a person of ordinary skill in the art. Non-limiting examples of suitable blending methods include melt blending, solvent blending, extruding, and the like.

In some embodiments, the ingredients of the compositions disclosed herein are melt blended by a method as described by Guerin et al. in U.S. Patent No. 4,152,189. First, all solvents, if there are any, are removed from the ingredients by heating to an appropriate elevated temperature of 100°C to 200 °C or 150°C to 175 °C at a pressure of 5 torr (667 Pa) to 10 torr (1333 Pa). Next, the ingredients are weighed into a vessel in the desired proportions and the foam is formed by heating the contents of the vessel to a molten state while stirring.

In other embodiments, the ingredients of the compositions disclosed herein are processed using solvent blending. First, the ingredients are dissolved in a suitable solvent and the mixture is then mixed or blended. Next, the solvent is removed to provide the compositions disclosed herein.

In further embodiments, physical blending devices that can provide dispersive mixing, distributive mixing, or a combination of dispersive and distributive mixing can be used in preparing homogenous blends. Both batch and continuous methods of physical blending can be used. Non-limiting examples of batch methods include those methods using BRABENDER^{®} mixing equipments (*e.g.*, BRABENDER PREP CENTER^{®}, available from C. W. Brabender Instruments, Inc., South Hackensack, N.J.) or BANBURY^{®} internal mixing and roll milling (available from Farrel Company, Ansonia, Conn.) equipment. Non-limiting examples of continuous methods include single screw extruding, twin screw extruding, disk extruding, reciprocating single screw extruding, and pin barrel single single screw extruding. In some embodiments, the additives can be added into an extruder through a feed hopper or feed throat during the extrusion of the farnesene interpolymer, the optional second polymer or the foam. The mixing or blending of polymers by extrusion has been described in C. Rauwendaal, "Polymer Extrusion", Hanser Publishers, New York, NY, pages 322-334 (1986).

When one or more additives are required in the compositions disclosed herein, the desired amounts of the additives can be added in one charge or multiple charges to the polyfarnesene, the second polymer or the composition. Furthermore, the addition can take place in any order. In some embodiments, the additives are first added and mixed or blended with the polyfarnesene and then the additive-containing polyfarnesene is blended with the second polymer. In other embodiments, the additives are first added and mixed or blended with the second polymer and then the additive-containing second polymer is blended with the polyfamesene. In further embodiments, the polyfarnesene is blended with the second polymer first and then the additives are blended with the composition.

The ingredients of the composition disclosed herein can be mixed or blended in any suitable mixing or blending devices known to skilled artisans. The ingredients in the composition disclosed herein can then be mixed at a temperature below the decomposition temperature of the ingredients to ensure that all ingredients are homogeneously mixed and remain intact.

### Applications of the Composition Comprising the Polyfarnesenes

The polyfarnesene compositions disclosed herein can be used as hot melt adhesives or pressure sensitive adhesives. It can be applied to manufacture any article that requires or comprises a hot melt adhesive or a pressure sensitive adhesive. Non-limiting examples of suitable articles include paper products, packaging materials, laminated wood panels, kitchen countertops, vehicles, labels, disposable diapers, hospital pads, feminine sanitary napkins, surgical drapes, tapes, cases, cartons, trays, medical devices, and bandages. In a further embodiment, the adhesive composition can be used in tapes, cases, cartons, trays, medical devices, and bandages.

In some embodiments, the compositions disclosed herein are used as hot melt adhesives. Such hot melt adhesive compositions can be used in industrial applications including packaging, particularly for low temperature use such as for dairy products or for freezer packaging of food products, and in sanitary disposable consumer articles, for example, diapers, feminine care pads, napkins, and the like. Some other suitable applications include book-binding, wood working and labeling. Some hot melt adhesives are described in A. V. Pocius, "Adhesion and Adhesives Technology," Hanser Gardner Publications; 2nd edition, Chapter 10, pp. 270-280 (2002), and M. J Satriana, "Hot melt adhesives: Manufacture and applications," Noyes Data Corp (1974).

In other embodiments, the compositions disclosed herein may be used as PSAs. Such PSA adhesive compositions can be applied to sheeting products (*e.g.*, decorative, reflective, and graphical), labelstock, and tape backings. The substrate can be any suitable type of material depending on the desired application. In certain embodiments, the substrate comprises a nonwoven, paper, polymeric film (*e.g.*, polypropylene (e.g., biaxially oriented polypropylene (BOPP)), polyethylene, polyurea, or polyester (*e.g.*, polyethylene terephthalate (PET)), or release liner (*e.g.*, siliconized liner). Some PSAs are described in A. V. Pocius, "Adhesion and Adhesives Technology," Hanser Gardner Publications; 2nd edition, Chapter 9, pp. 238-259 (2002); and Istvan Benedek, "Technology of Pressure-Sensitive Adhesives and Products," CRC; (2008).

In still other embodiments, the compositions can be utilized to form tape. For example, the PSA or hot melt adhesive composition is applied to at least one side of the backing of the tape. The adhesive composition may then be crosslinked to further improve its shear strength. Any suitable crosslinking method (*e.g.*, exposure to radiation, such as ultraviolet or electron beam) or crosslinker additive (*e.g.*, phenolic and silane curatives) may be utilized.

The adhesive compositions disclosed herein may be applied to the desired substrate or adhered in any manner known in the art, particularly those methods used traditionally for making tapes, cases, cartons, trays, medical devices, and bandages. In other embodiments, the adhesive compositions can be applied by a coating head or nozzle, with associated equipment. The adhesive compositions can be applied as fine lines, dots or spray coatings, in addition to other traditional forms as desired.

In some embodiments, the adhesive compositions can be applied using melt extrusion techniques. The adhesive composition can be applied by either continuous or batch processes. An example of a batch process is the placement of a portion of the adhesive composition between a substrate to which the adhesive composition is to be adhered and a surface capable of releasing the adhesive to form a composite structure. An example of a continuous forming method includes drawing the adhesive composition out of a heated film die and subsequently contacting the drawn composition to a moving plastic web or other suitable substrate.

In other embodiments, the adhesive compositions can be coated using a solvent-based method. For example, the solvent-based adhesive composition can be coated by such methods as knife coating, roll coating, gravure coating, rod coating, curtain coating, and air knife coating. The coated solvent-based adhesive composition is then dried to remove the solvent. Preferably, the applied solvent-based adhesive composition is subjected to elevated temperatures, such as those supplied by an oven, to expedite drying.

In certain embodiments, the compositions are used as hot-melt pressure-sensitive adhesives. Some hot-melt pressure-sensitive adhesives are described in Istvan Benedek, "Technology of Pressure-Sensitive Adhesives and Products," CRC; Chapter 3, (2008).

In some embodiments, the compositions are used as rubber-based adhesives or contact bond adhesives. Some rubber-based adhesives or contact bond adhesives are described in A. V. Pocius, "Adhesion and Adhesives Technology," Hanser Gardner Publications; 2nd edition, Chapter 9, pp. 259-265 (2002).

The following examples are presented to exemplify embodiments of the invention but are not intended to limit the invention to the specific embodiments set forth. Unless indicated to the contrary, all parts and percentages are by weight. All numerical values are approximate. When numerical ranges are given, it should be understood that embodiments outside the stated ranges may still fall within the scope of the invention. Specific details described in each example should not be construed as necessary features of the invention.

### EXAMPLES

### Purification of Starting Materials

β-farnesene having 97.6% purity by weight was obtained from Amyris Biotechnologies Inc., Emeryville, CA. β-Farnesene included hydrocarbon-based impurities such as zingiberene, bisabolene, farnesene epoxide, farnesol isomer, E,E-farnesol, squalene, ergosterol, and some dimers of famesene. β-farnesene was purified with a 3Å molecular sieve to remove the impurities and were then redistilled under nitrogen atmosphere to improve purity. Cyclohexane was distilled under nitrogen atmosphere to eliminate moisture and stored with a drying agent.

### Differential Scanning Calorimetry

A TA Q200 differential scanning calorimeter was utilized to determine glass transition temperatures (T_{g}) of polymer samples disclosed herein. A 5 mg sample was placed in an aluminum pan. An empty reference pan and the sample pan were maintained within ± 0.01 mg. Samples were scanned from about -175 °C to about 75 °C at a rate of 10 °C/min. T_{g} was identified as a step change transition in the heat flow. The mid-point of the transition was reported as the T_{g} of the sample.

### Gel Permeation Chromatography

GPC was utilized to determine the molecular weights and polydispersities of polymer samples. A Waters 2414 refractive index detector was used with a Waters 1515 isocratic HPLC pump. HPLC grade tetrahydrofuran was used as solvent. Polydispersed fractions were collected from GPC. The molecular weight of a sample was generally recorded as the number averaged molecular weight (Mₙ) or the weight average (M_{w}). When there were overlapping peaks which prohibited the determination of a unique polydispersity of each peak, a peak molecular weight (Mₚ) was incorporated herein.

### Thermal Gravimetric Analysis

The degradation temperatures of samples were determined by thermal gravimetric analysis (TGA). About 20 mg of a sample was placed in a tared pan. The pan was then loaded into a furnace. Air flow was allowed to equilibrate. The sample was then heated from room temperature to 580 °C at 10 °C/min. Temperatures for 1% and 5% weight loss of samples were reported respectively.

### Ultraviolet-Visible Spectroscopy

Ultraviolet-visible (UV-Vis) spectroscopy was utilized to monitor monomer consumption during the reaction. The reaction was allowed to continue until all monomers had been consumed. A Shimadzu UV-2450 UV-Vis spectrophotometer was utilized. Background measurement was averaged from five measurements with an empty quartz cuvette. Aliquots were periodically taken from the reaction vessel, which was then placed in a square quartz cuvette with having 1 cm beam distance. The absorbance of the sample is directly proportional to the concentration of the monomer in the aliquot. The progress of the reaction was monitored by UV-Vis spectroscopy with the characteristic absorption peak of β-farnesene at 230 nm.

### Tensile strength

Tensile strength of samples were determined using an INSTRON^{™} tensile tester. A sample was cast into films and cut to the appropriate dimensions. The thickness and width of the sample after processing were measured. A gauge length of 2.54 cm was used with a crosshead speed 25 mm/min.

### Lap Test

Lap test was used to characterize adhesive properties of samples. Two substrates were held together by an adhesive. Substrates were then pulled apart, shearing the adhesive. The construct fails in one of three ways. When the substrate failed, it was called a substrate failure. When the adhesive was tom apart, it was called a cohesive failure. When the interface between the substrate and adhesive failed, it was called an adhesive failure. An INSTRON^{™} tensile tester was used to characterize the forces involved in the failure. The adhesive was applied to a 2 cm² section of the substrate with a crosshead speed of 25 mm/min. Aluminum was used as the substrate. Aluminum was cleaned with acetone before bonding.

### ¹H and ¹³C Nuclear Magnetic Resonance

¹H and ¹³C Nuclear Magnetic Resonance was utilized to characterize chemical microstructures of the samples. A Varian Mercury 300 MHz NMR was utilized for these measurements. Deuterated chloroform was used as the solvent. Several measurements were repeated for collecting spectra.

### Example 1 - 1,4-polyfarnesene Having a Mₙ of 105,000

To a dried three-neck reactor under argon atmosphere, a pre-dried solution comprising 92.29 g of β-farnesene in 13.7% in cyclohexane was added. *n*-Butyl lithium (1.85x 10⁻³ mol, obtained from Acros, Morris Plains, NJ) was added into the reactor as an initiator, and the reactor was heated at about 50 °C for about 19 hours, until all β-farnesene was consumed, monitored by UV-Vis spectroscopy. Example 1 was precipitated from the reaction mixture with a 1% solution of ethanol and t-butyl catachol (obtained from Sigma-Aldrich, St. Louis, MO). After drying in a vacuum oven at about 60 °C for about 2 hours, Example 1 was kept under vacuum for about 16 hours. Afterwards, Example 1, collected at 89.83 g (yield 97%), was stored in a refrigerator to prevent any crosslinking before characterization.

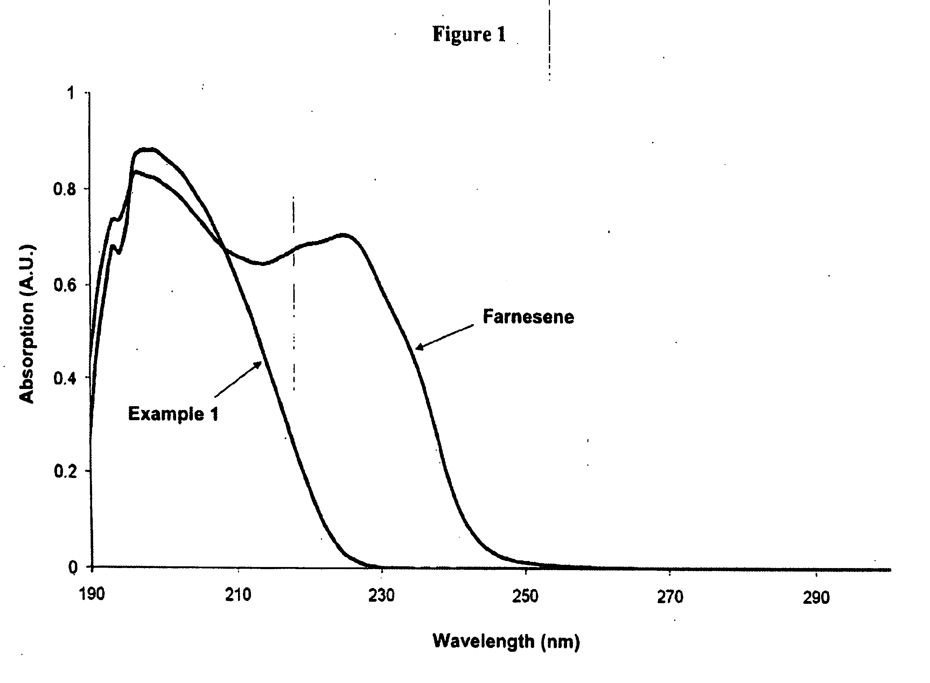

The progress of synthesizing Example 1 was monitored by the disappearance of β-farnesene, as measured by UV-Vis in the reaction mixture. Figure 1 shows the Ultraviolet-Visible (UV-Vis) spectra of Example 1 and β-farnesene. The characteristic absorption peak of β-farbesene at 230 nm is present in the UV-Vis spectrum for β-farnesene in Figure 1, but absent in the UV-Vis spectrum for Example 1 in Figure 1.

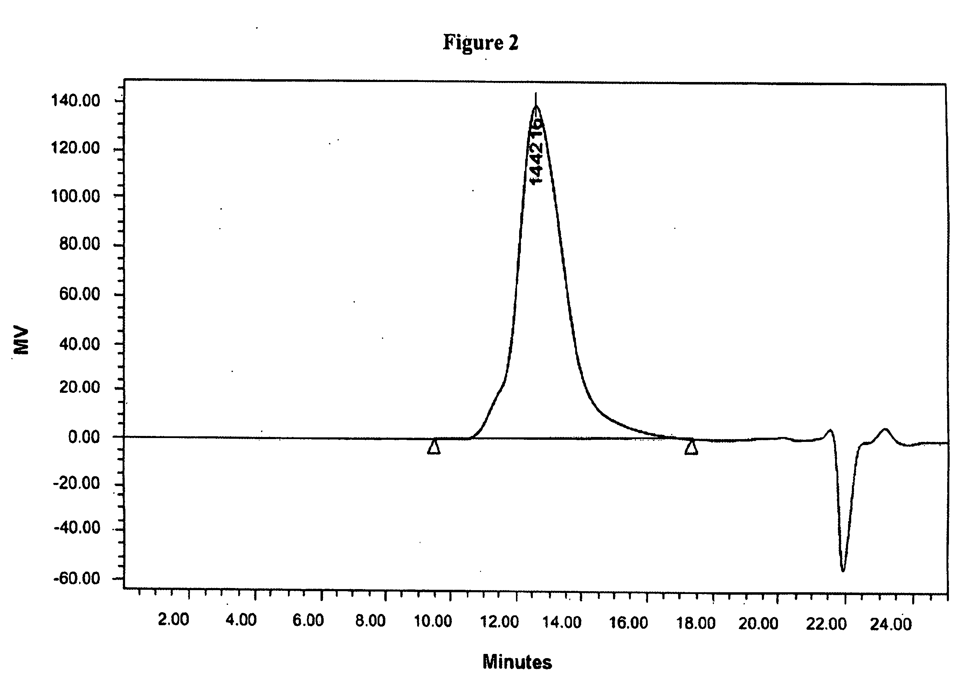

The molecular weight and polydispersity of Example 1 were determined by GPC. Figure 2 shows the GPC curve of Example 1. The number average molecular weight (Mₙ), weight average molecular weight (M_{w}), peak molecular weight (Mₚ), *z* average molecular weight (M_{z}), *z*+*1* average molecular weight (M_{z+1}), M_{w}/Mₙ (*i.e.*, polydispersity), M_{z}/M_{w}, and M_{z+1}/M_{w} of Example 1 are shown in Table 1. The definitions of Mₙ, M_{w}, M_{z}, M_{z+1}, Mₚ, and polydispersity can be found in Technical Bulletin TB021, "Molecular Weight Distribution and Definitions of MW Averages," published by Polymer Laboratories, which is incorporated herein by reference. Some methods of measuring the molecular weights of polymers can be found in the book by Malcolm P. Stevens, "Polymer Chemistry: An Introduction," Oxford University Press, Chapter 2 (1999), pp. 35-58, which is incorporated herein by reference. The number of farnesene units in Example 1 was calculated to be about 490.

**Table 1**

| **Properties** | **Example 1** |
|---|---|
| Mₙ | 104,838 g/mol |
| M_{w} | 147,463 g/mol |
| Mₚ | 144,216 g/mol |
| M_{z} | 207,084 g/mol |
| M_{z+1} | 314,887 g/mol |
| Polydispersity | 1.406588 |
| M_{z}/M_{W} | 1.404311 |
| M_{z+1}/M_{w} | 2.135360 |

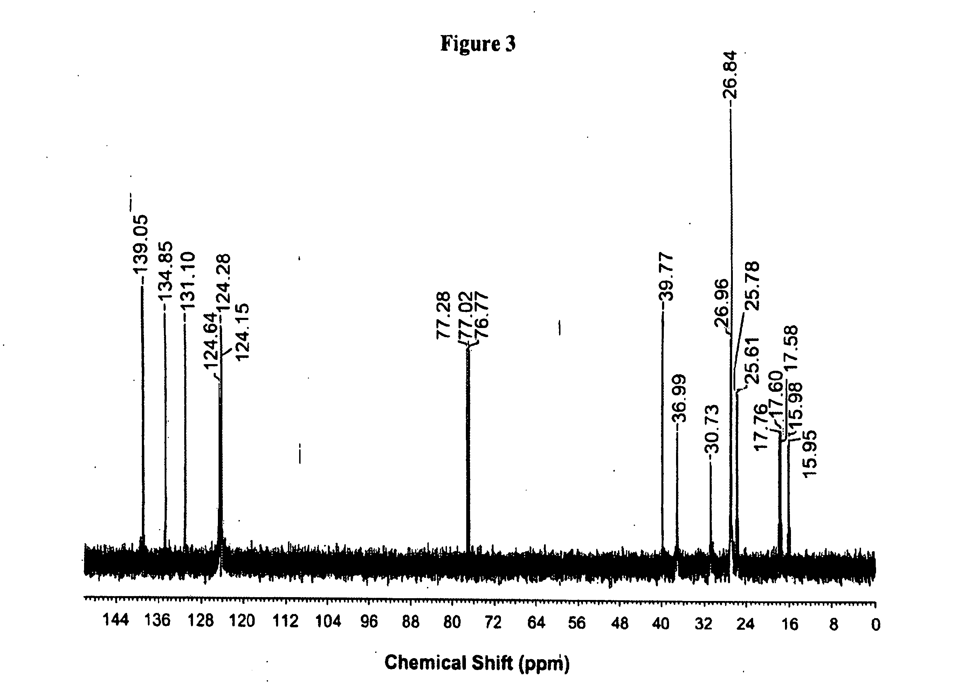

Figure 3 shows the ¹³C NMR spectrum of Example 1. Peaks at 77.28 ppm, 77.02 ppm, and 76.77 ppm were peaks associated with the deuterated chloroform used for collecting the ¹³C NMR spectrum. The characteristic peak identifying Example 1 was at 139.05 ppm.

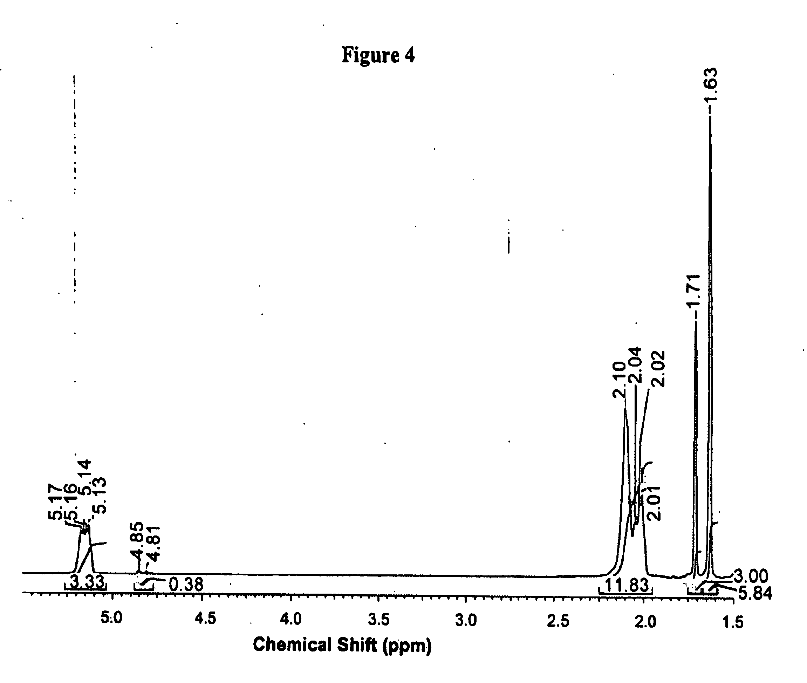

Figure 4 shows the ¹H spectrum of Example 1. Peaks at 4.85 ppm and 4.81 ppm were peaks associated with 3,4-microstructure. Peaks at 5.17 ppm, 5.15 ppm, 5.14 ppm, and 5.13 ppm were peaks associated with 1,4- and 3,4-microstructures. Based on the areas under the peaks of Figure 4, about 12% of farnesene units in Example 1 was found to have 3,4-microstructure.

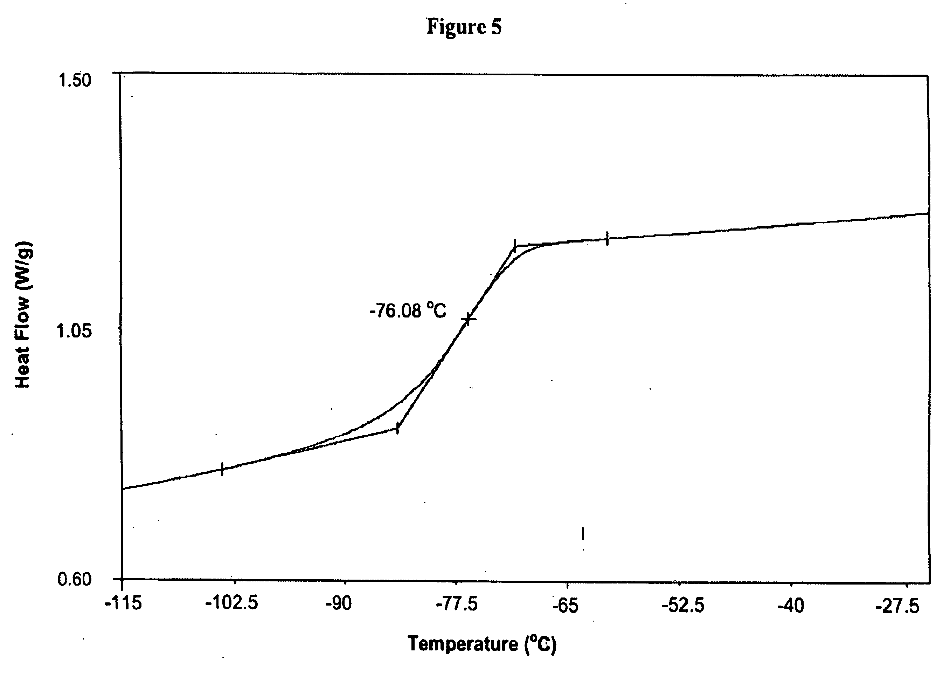

The DSC curve of Example 1 is shown in Figure 5. The thermal characteristics of Example 1 were measured by DSC. The T_{g} of Example 1 was found to be about -76 °C. No other thermal event was detected between -175 °C and 75 °C.

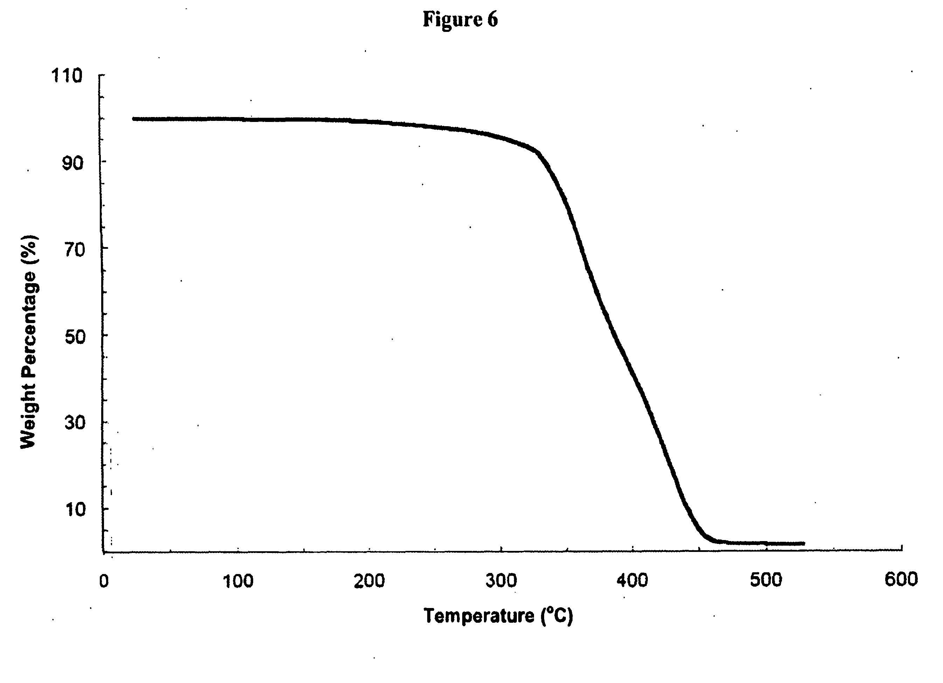

The TGA curve of Example 1 measured in air is shown in Figure 6. The decomposition temperature of Example 1 in air was determined by TGA. The 1% weight loss of Example 1 in air was recorded at 210 °C and the 5% weight loss of Example 1 in air was recorded at 307 °C.

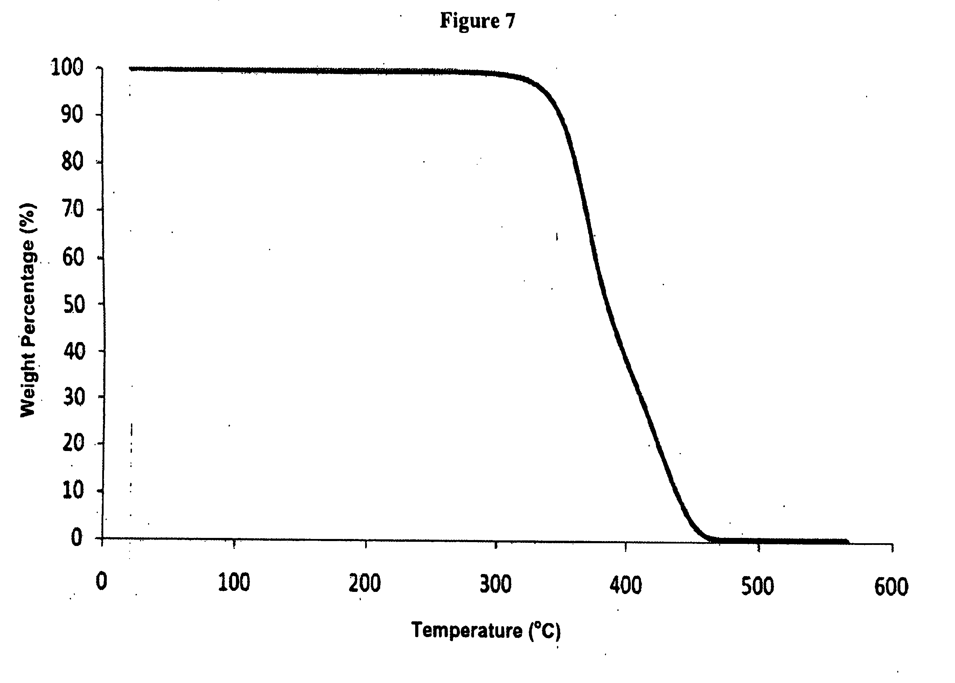

The TGA curve of Example 1 measured under nitrogen atmosphere is shown in Figure 7. The 1% weight loss of Example 1 under nitrogen atmosphere was recorded at 307 °C and the 5% weight loss of Example 1 under nitrogen atmosphere was recorded at 339 °C.

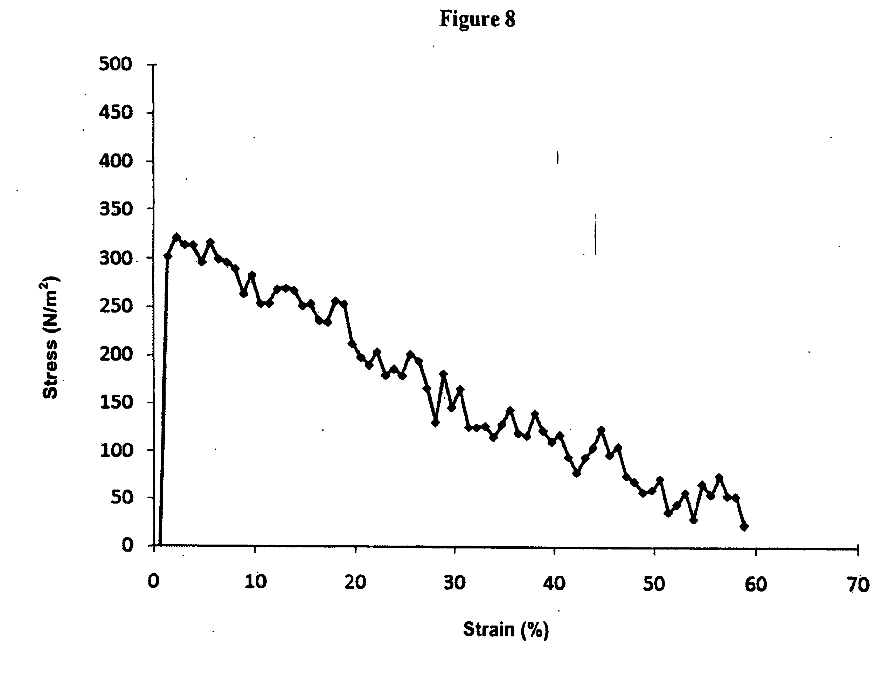

Example 1 was observed to be tacky. The lap test results of Example 1 are shown in Figure 8. The adhesive capability of Example 1 was measured by the lap test. The adhesive energy of Example 1 was found to be about 11,400 J/m² with a peak stress of about 314 N/m².

### Example 2 - 1,4-polyfarnesene having a Mₙ of 245,000

Example 2 is a 1,4-polyfamesene having a Mₙ of about 245,000 g/mol. Example 2 was synthesized similarly according to the procedure for Example 1, except *sec-*butyl lithium was used as the initiator. The net weight of Example 2 was found to be 83.59 g (yield 71.4%). The yield is lower because aliquots were removed to monitor the progression of the reaction.

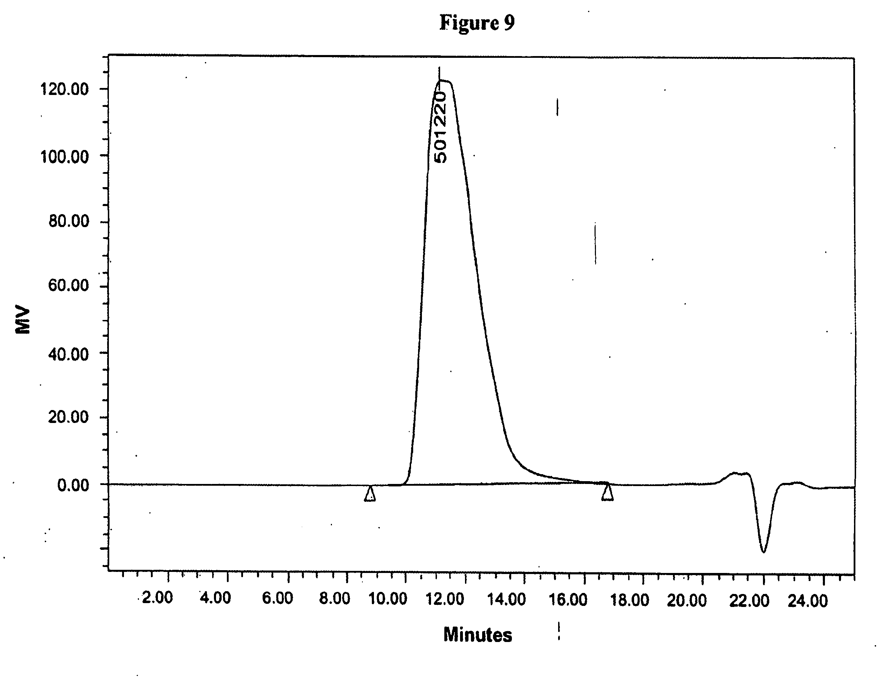

The molecular weight and polydispersity of Example 2 were determined by GPC. Figure 9 shows the GPC curve of Example 2. The Mₙ, M_{w}, Mₚ, M_{z}, M_{z+1}, polydispersity, M_{z}/M_{w}, and M_{z+1}/M_{w} of Example 2 are shown in Table 2. The number of farnesene units in Example 2 was calculated to be about 2000. Because of the increased molecular weight of Example 2, it had a higher level of entanglement and longer relaxation time than Example 1.

**Table 2**

| **Properties** | **Example 2** |
|---|---|
| Mₙ | 244,747 g/mol |
| M_{w} | 457,340 g/mol |
| Mₚ | 501,220 g/mol |
| M_{z} | 768,187 g/mol |
| M_{z+1} | 1,132,362 g/mol |
| Polydispersity | 1.868622 |
| M_{z}/M_{w} | 1.679684 |
| M_{z+1}/M_{w} | 2.475971 |

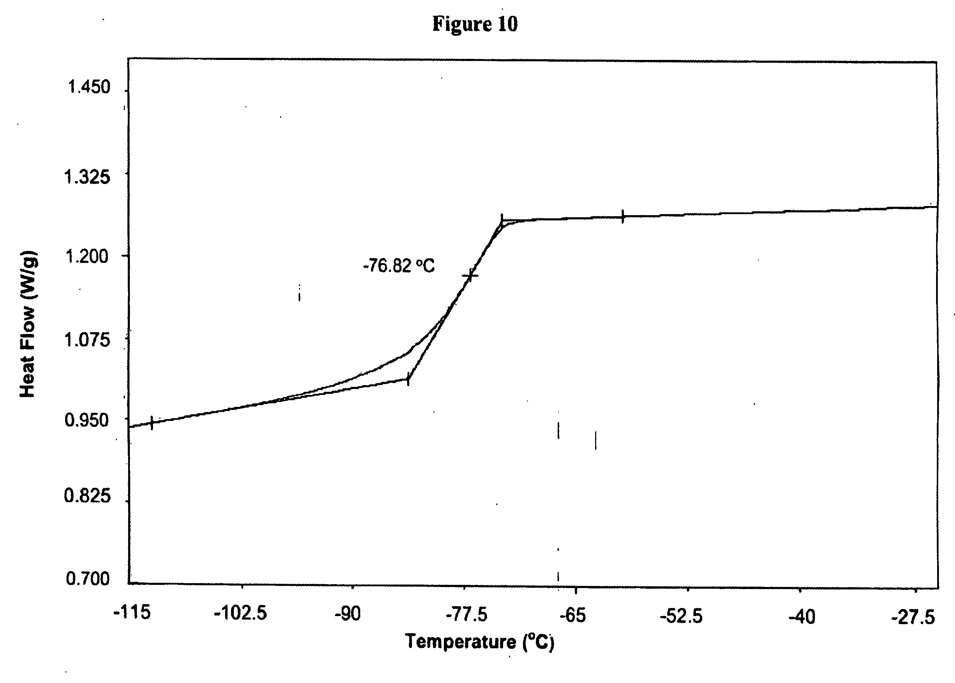

The DSC curve of Example 2 is shown in Figure 10. The thermal characteristics of Example 2 were measured by DSC. The T_{g} of Example 2 was found to be about -76 °C.

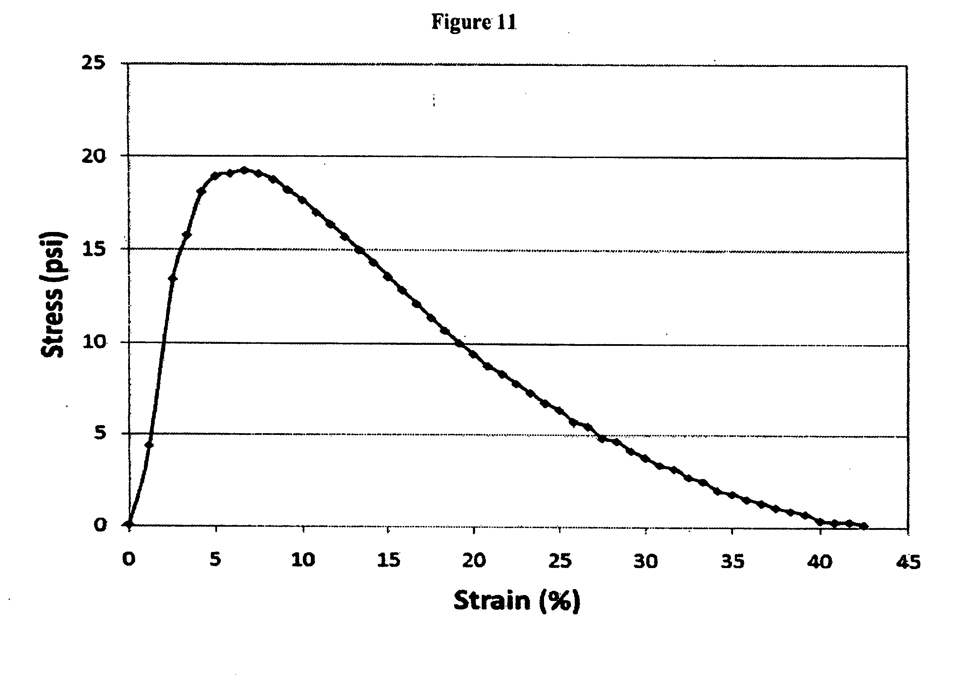

The tensile test results of Example 2 are shown in Figure 11. The tensile strength of Example 2 was measured by a tensile test. Example 2 was observed to be soft, tacky and yielded quickly. As shown in Figure 11, the peak elongation of Example 2 was found to be about 6% with a maximum tensile strength of about 19 psi. The modulus of Example 2 was calculated to be about 4.6 kpsi. Example 2 continued to yield to about 40% elongation.

### Example 3 - 3,4-polyfarnesene

Example 3 was synthesized similarly according to the procedure for Example 1 except that n-butyl lithium (1.71x10⁻³ mol) was added in the presence of N,N,N',N'-tetramethylethylenediamine (1.71x10⁻³ mol, TMEDA, obtained from Sigma-Aldrich, St. Louis, MO). The net weight of Example 3 was found to be 82.72 g (yield 97%).

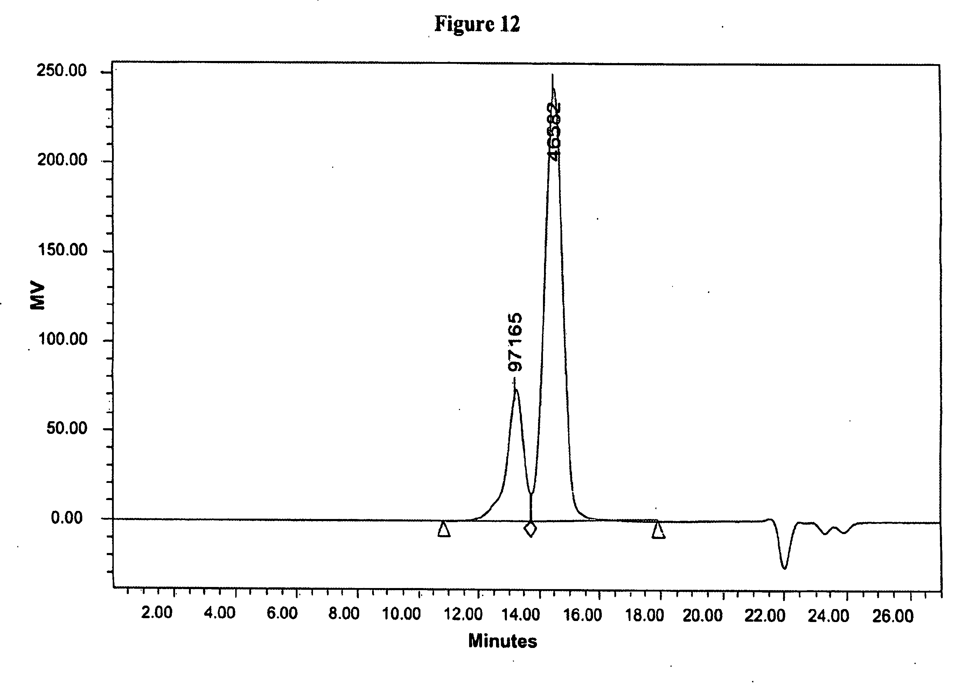

The molecular weight and polydispersity of Example 3 were determined by GPC. Figure 12 shows the GPC curve of Example 3. The two peaks in Figure 12 indicated that two distinct weight fractions formed in Example 3. The Mₙ, M_{w}, M_{z}, M_{z+1}, polydispersity, M_{z}/M_{w}, and M_{z+1}/M_{w} of Example 3 are shown in Table 3. The Mₚ of the first peak in Figure 12 was about 97,165 g/mol. The Mₚ of the second peak in Figure 12 was about 46,582 g/mol. The number of farnesene units in Example 3 was calculated to be about 240.

**Table 3**

| **Properties** | **Example 3** |
|---|---|
| Mₙ | 45,818 g/mol |
| M_{w} | 47,644 g/mol |
| M_{z} | 49,134 g/mol |
| M_{z+1} | 50,527 g/mol |
| Polydispersity | 1.039844 |
| M_{z}/M_{w} | 1.031269 |
| M_{z+1}/M_{w} | 1.060509 |

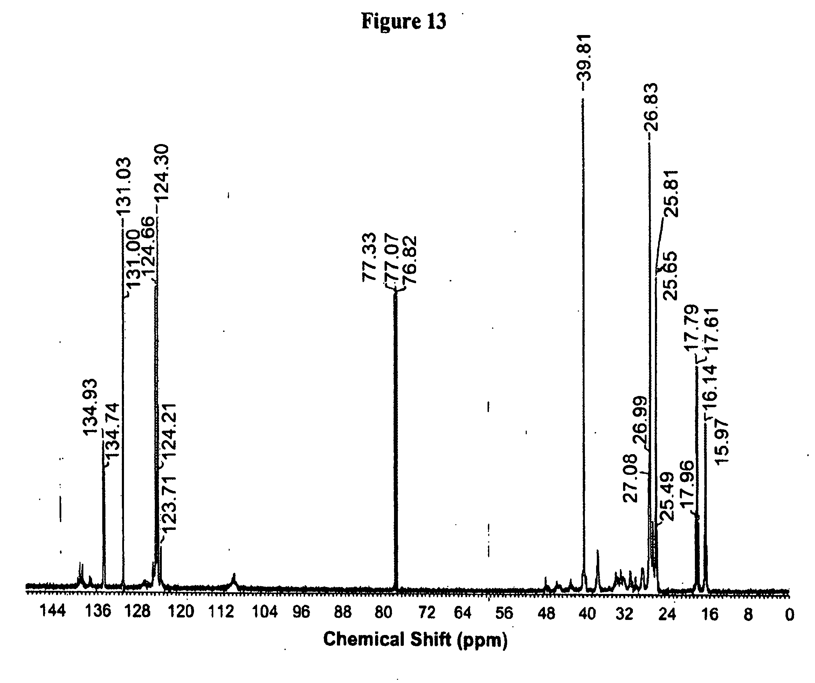

Figure 13 shows the ¹³C NMR spectrum of Example 3. Peaks at 77.28 ppm, 77.02 ppm, and 76.77 ppm were peaks of deuterated chloroform used for collecting the ¹³C NMR spectrum. The characteristic peak identifying Example 1 at 139.05 ppm was absent in Figure 13, indicating a regular microstructure of Example 3.

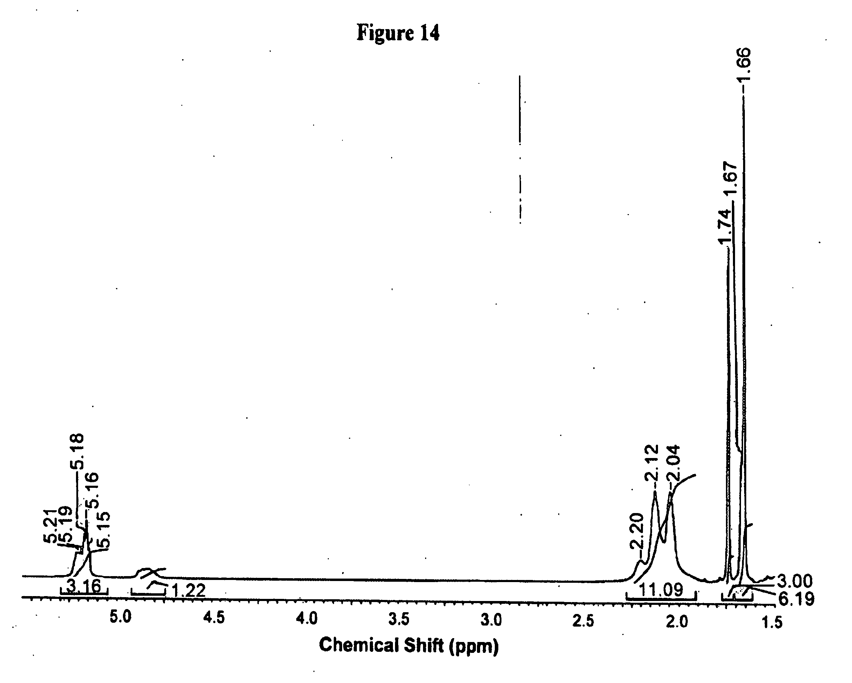

Figure 14 shows the ¹H MR spectrum of Example 3. Peaks at 4.85 ppm and 4.81 ppm were peaks showing microstructure of Example 3. Peaks at 5.17 ppm, 5.16 ppm, 5.14 ppm, and 5.13 ppm were peaks associated with 1,4- and 3,4-microstructures. Based on the areas under the peaks of Figure 14, about 10% of farnesene units in Example 3 was found to have 1,4-microstructure.

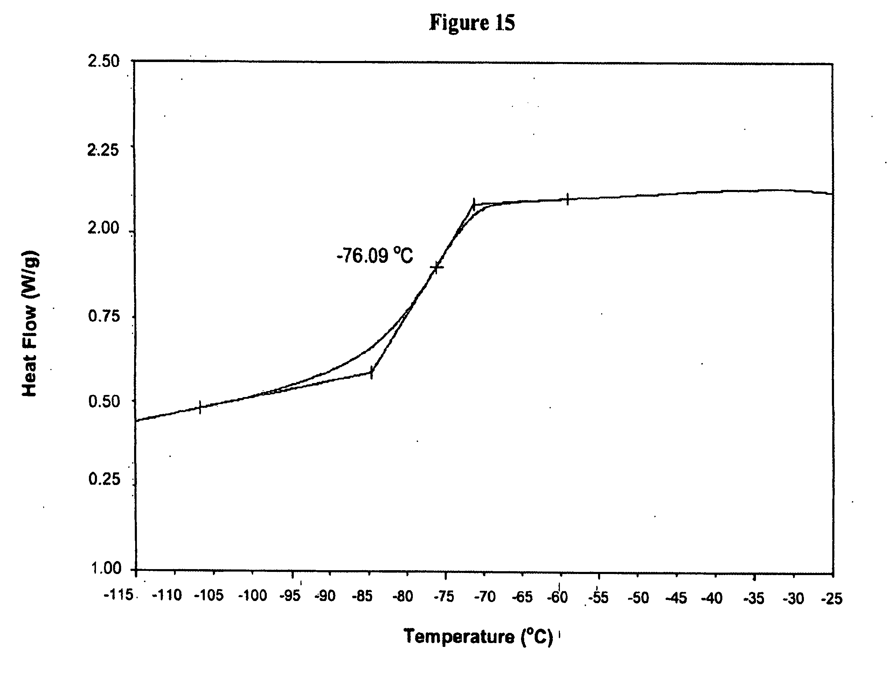

The DSC curve of Example 3 is shown in Figure 15. The thermal characteristics of Example 3 were measured by DSC. The T_{g} of Example 3 was found to be about -76 °C. No other thermal event was detected between -175 °C and 75 °C.

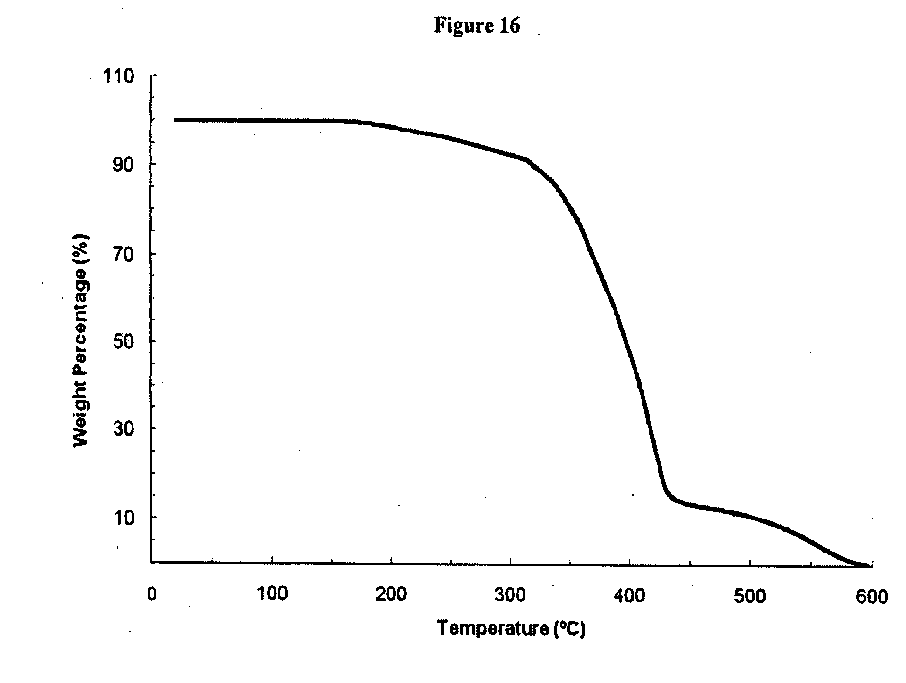

The TGA curve of Example 1 measured in air is shown in Figure 16. The decomposition temperature of Example 3 in air was determined by TGA. The 1% weight loss of Example 1 in air was recorded at 191 °C and the 5% weight loss of Example 1 in air was recorded at 265 °C.

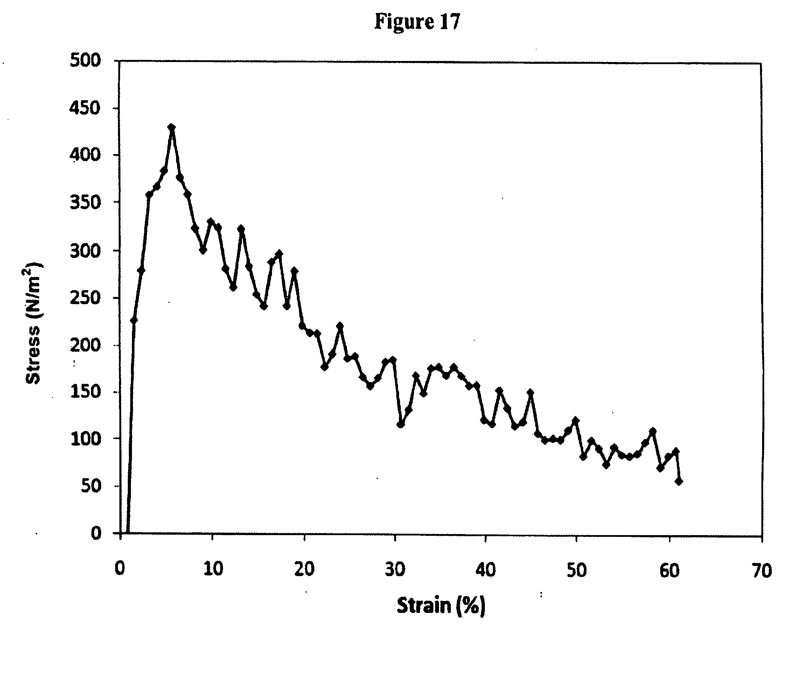

Example 3 was observed to be a highly tacky viscous fluid. The lap test results of Example 3 are shown in Figure 17. The adhesive capability of Example 3 was measured by the lap test. The adhesive energy of Example 3 was found to be about 12,900 J/m² with a peak stress of about 430 N/m².

### Example 4 - polystyrene-1,4-polyfarnesene-polystyrene

To a first dried three neck reactor under argon atmosphere, a pre-dried solution of 12% β-farnesene in cyclohexane was added. To a second dried three neck reactor under argon atmosphere, a 20.65 g solution of 10% styrene in cyclohexane was added. Afterwards, to the styrene solution, n-butyl lithium (6.88x10⁻⁴ mol) was added into the reactor as an initiator, and the reactor was heated at about 50°C for about 16 hours, until all styrene was consumed, as monitored by GPC. Then, 161.8 β-farnesene solution (*i.e.*, 19.61 g of β-farnesene) was transferred to the reactor under argon atmosphere. The reaction was allowed to react until completion for about 7 hours, monitored by GPC. Three equal aliquots of dichlorosilane coupling agent (3.44x 10⁻⁴ mol, obtained from Acros, Morris Plains, NJ) were then added into the reactor such that the mole ratio of Li to Cl of the reaction mixture was 1:2. The reaction mixture was allowed to react until completion as indicated by a color change from yellow to clear in the reactor. Example 4 was precipitated from the reaction mixture with a 1% solution of *t*-butyl catachol in ethanol. After drying in a vacuum oven at about 60 °C for about 2 hours, Example 4 was kept under vacuum for about 16 hours. Afterwards, Example 4, collected at 39.15 g (yield 97%), was stored in a refrigerator to prevent any crosslinking before characterization.

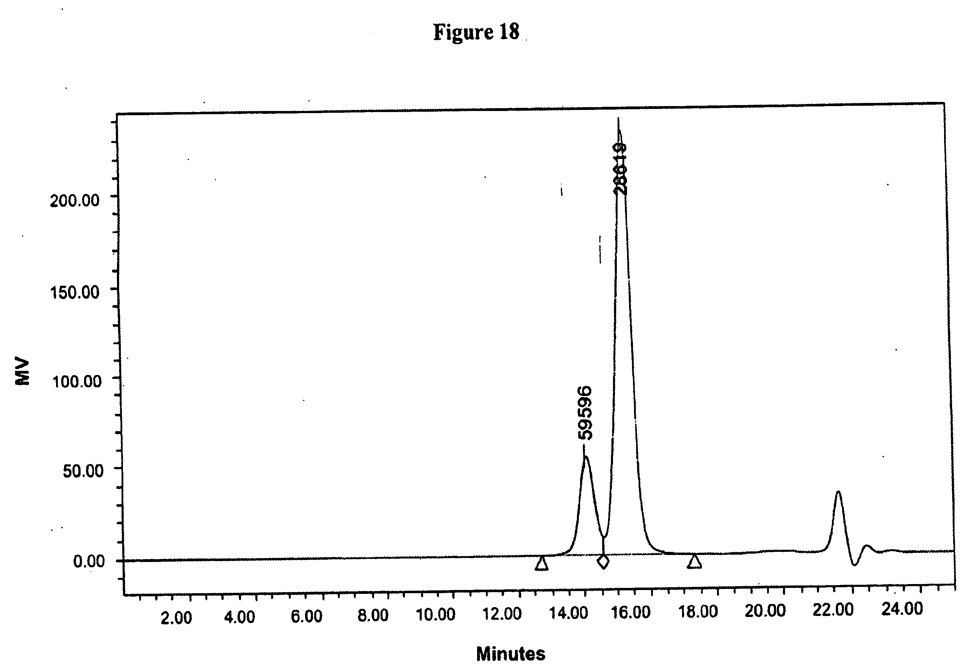

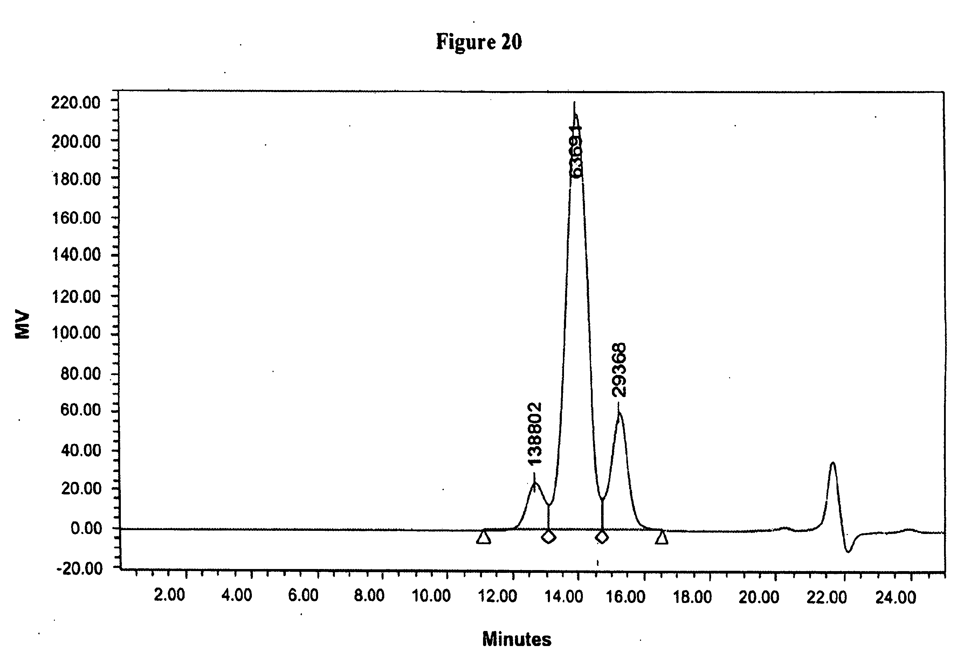

The GPC curve of polystyrene is shown in Figure 18. The progress of polystyrene synthesis reaction was monitored by GPC. The two peaks in Figure 18 indicated that there were two distinct weight fractions of polystyrene formed. The Mₙ, M_{w}, M_{z}, M_{z+1}, polydispersity, M_{z}/M_{w}, and M_{z+1}/M_{w} of the polystyrene are shown in Table 4. The Mₚ of the first peak in Figure 18 was found to be about 59,596 g/mol. The Mₚ of the second peak in Figure 20 was found to be about 28,619 g/mol.

**Table 4**

| **Properties** | **Polystyrene** |
|---|---|
| Mₙ | 28,396 g/mol |
| M_{w} | 29,174 g/mol |
| M_{g} | 29,895 g/mol |
| M_{z+1} | 30,598 g/mol |
| Polydispersity | 1.027385 |
| M_{z}/M_{w} | 1.024739 |
| M_{z+1}/M_{w} | 1.048810 |

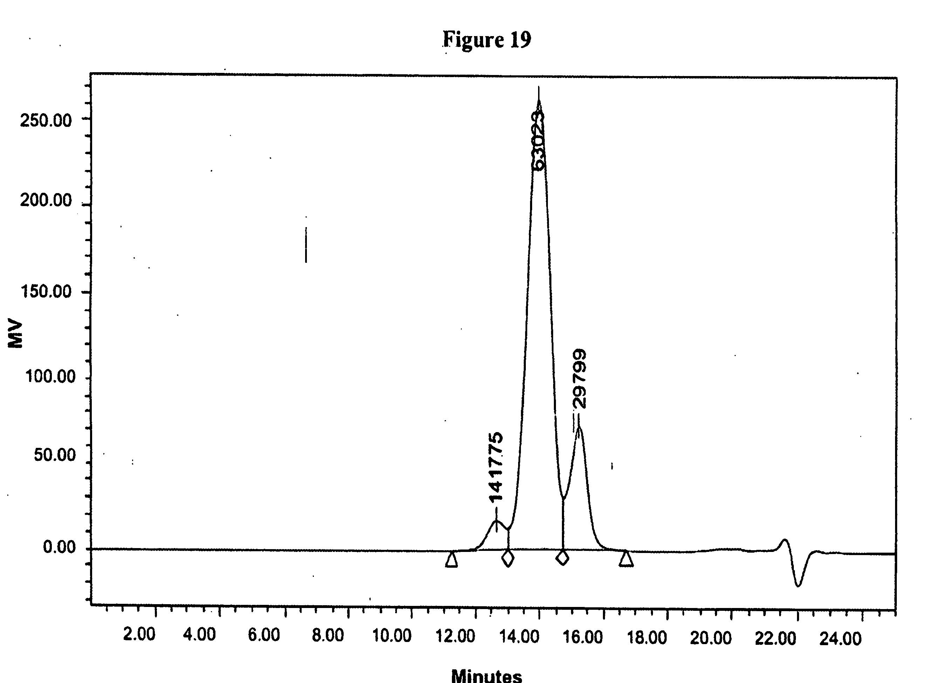

The polystyrene formed then acted as an initiator to initiate the polymerization with β-farnesene to form a polystyrene-1,4-polyfarnesene di-block copolymer. The GPC curve of the di-block copolymer is shown in Figure 19. The progress of the di-block copolymer synthetic reaction was monitored by GPC. The three peaks in Figure 19 indicated that there were three distinct weight fractions in the di-block copolymer reaction solution. The Mₙ, M_{w}, Mₚ, M_{z}, M_{z+1}, polydispersity, M_{z}/M_{w}, and M_{z+1}/M_{w} of the di-block copolymer are shown in Table 5. The Mₚ of the first peak in Figure 19, corresponding to polystyrene-1,4-polyfarnesene-polystyrene, was found to be about 141,775 g/mol. The Mₚ of the second peak in Figure 19, corresponding to the di-block copolymer, was found to be about 63,023 g/mol. The molecular weight of 1,4-polyfarnesene in the di-block copolymer was calculated to be about 35,000 g/mol. The Mₚ of the third peak in Figure 19, corresponding to polystyrene, was found to be about 29,799 g/mol.

**Table 5**

| **Properties** | **Polystyrene-1,4-polyfarnesene Di-block Copolymer** |
|---|---|
| Mₙ | 29,434 g/mol |
| M_{w} | 30,345 g/mol |
| Mₚ | 29,799 g/mol |
| M_{z} | 31,172 g/mol |
| M_{z+1} | 31,936 g/mol |
| Polydispersity | 1.030949 |
| M_{z}/M_{w} | 1.027264 |
| M_{z+1}/M_{w} | 1.052449 |

The polystyrene-1,4-polyfarnesene di-block copolymer was further coupled to form Example 4. Figure 20 shows the GPC curve of Example 4. The molecular weight and polydispersity of Example 4 were determined by GPC. The three peaks in Figure 20 indicated that there were three distinct weight fractions for the coupling product formed. The Mₙ, M_{w}, M_{z}, M_{z+1}, polydispersity, M_{z}/M_{w}, and M_{z+1}/M_{w} of the coupling product are shown in Table 6. The Mₚ of the first peak in Figure 20, corresponding to Example 4, was found to be about 138,802 g/mol. Example 4 was obtained in about 10% of the coupling product. The number of farnesene monomer units in Example 4 was calculated to be about 300. The Mₚ of the second peak in Figure 20, which corresponds to polystyrene-1,4-polyfarnesene di-block copolymers, was found to be about 63,691 g/mol. The Mₚ of the third peak in Figure 20, corresponding to polystyrene, was found to be about 29,368 g/mol.

**Table 6**

| **Properties** | **Example 4** |
|---|---|
| Mₙ | 138,240 g/mol |
| M_{w} | 142,147 g/mol |
| M_{g} | 146,636 g/mol |
| M_{z+1} | 151,848 g/mol |
| Polydispersity | 1.028264 |
| M_{z}/M_{w} | 1.031576 |
| M_{z+1}/M_{w} | 1.068242 |

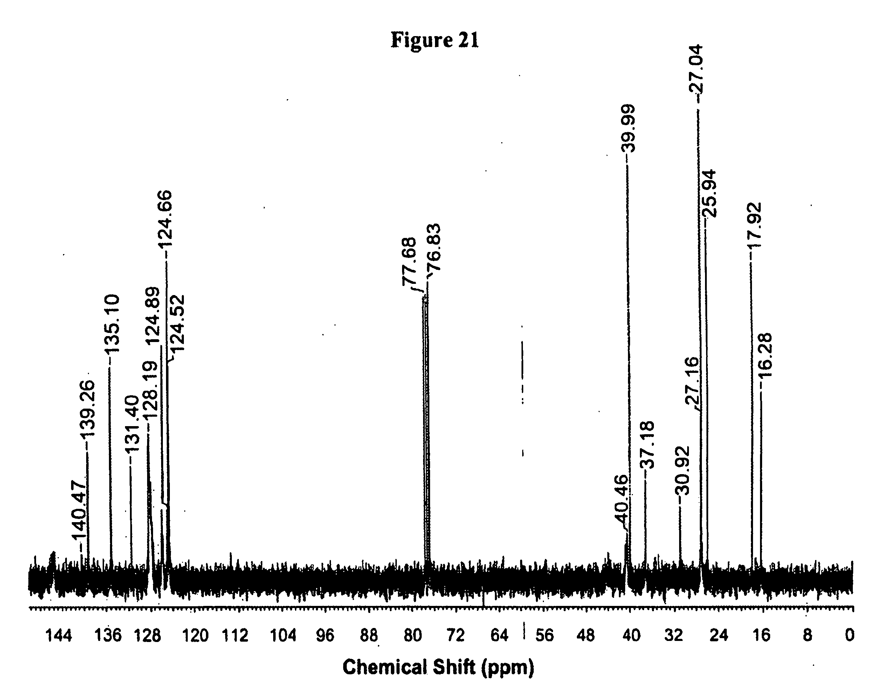

Figure 21 shows the ¹³C NMR spectrum of Example 4. Peaks at 77.69 ppm and 76.80 ppm were peaks of associated with the deuterated chloroform used for collecting the ¹³C NMR spectrum. Other peaks in Figure 21 were peaks associated with 1,4-polyfarnesene and polystyrene. The characteristic peak identifying 1,4-polyfarnesene at 139.25 ppm was present in Figure 21, indicating the present of 1,4 polyfarnesene in Example 4.

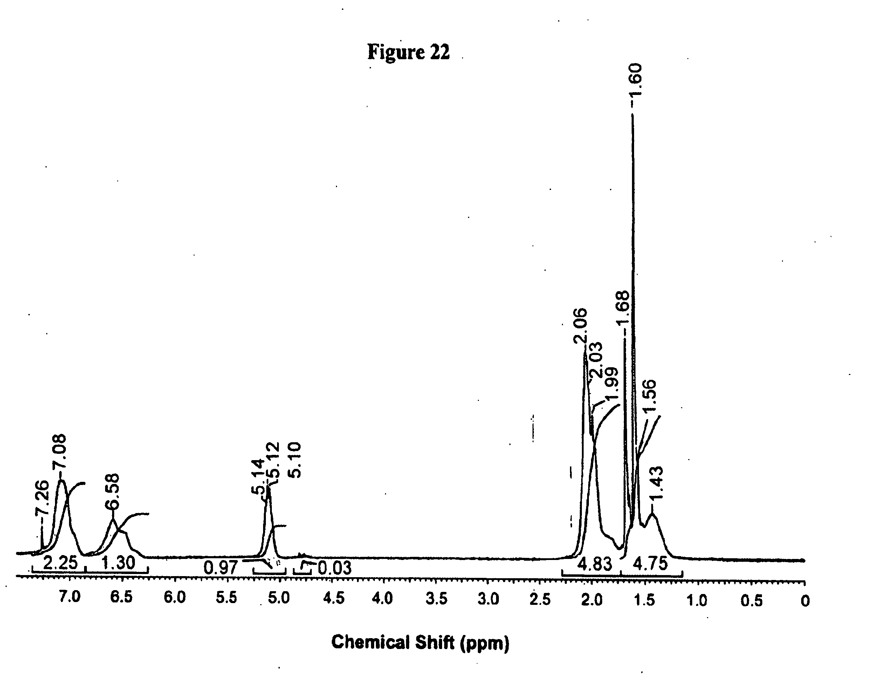

Figure 22 shows the ¹H NMR spectrum of Example 4. Peaks at 4.85 ppm and 4.81 ppm were peaks associated with 3,4-microstructure. Peaks at 5.10 ppm, 5.12 ppm, and 5.14 ppm were peaks associated with 1,4- and 3,4-microstructures. Based on the areas under the peaks of Figure 22, about 3% of farnesene units in Example 4 was found to have 3,4-microstructure.

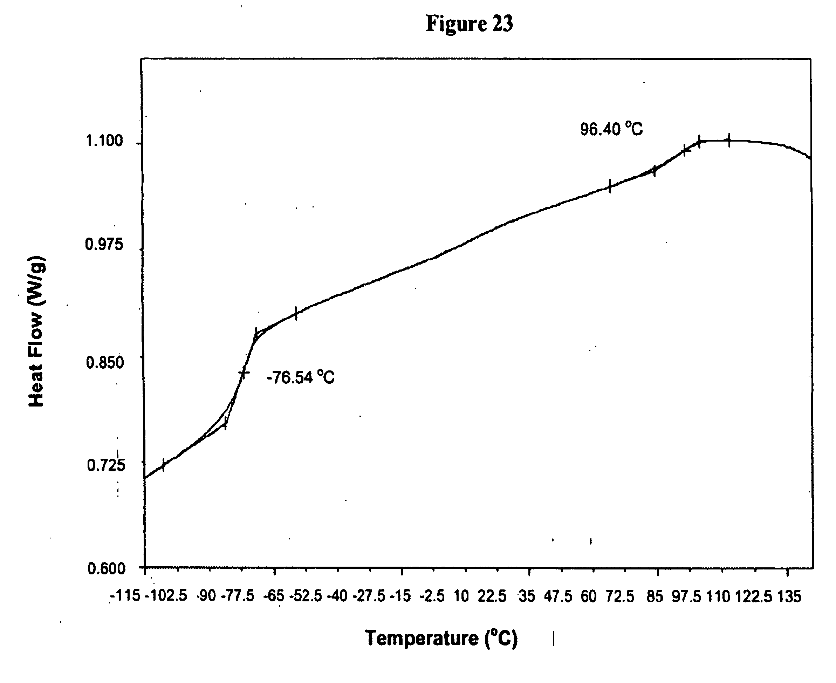

The DSC curve of Example 4 is shown in Figure 23. The thermal characteristics of Example 4 were measured by DSC. The T_{g} of 1,4-polyfarnesene in Example 4 was found to be about -76 °C. The T_{g} of polystyrene in Example 4 was found to be about 96 °C. No other thermal event was detected between -175 °C and 75 °C.

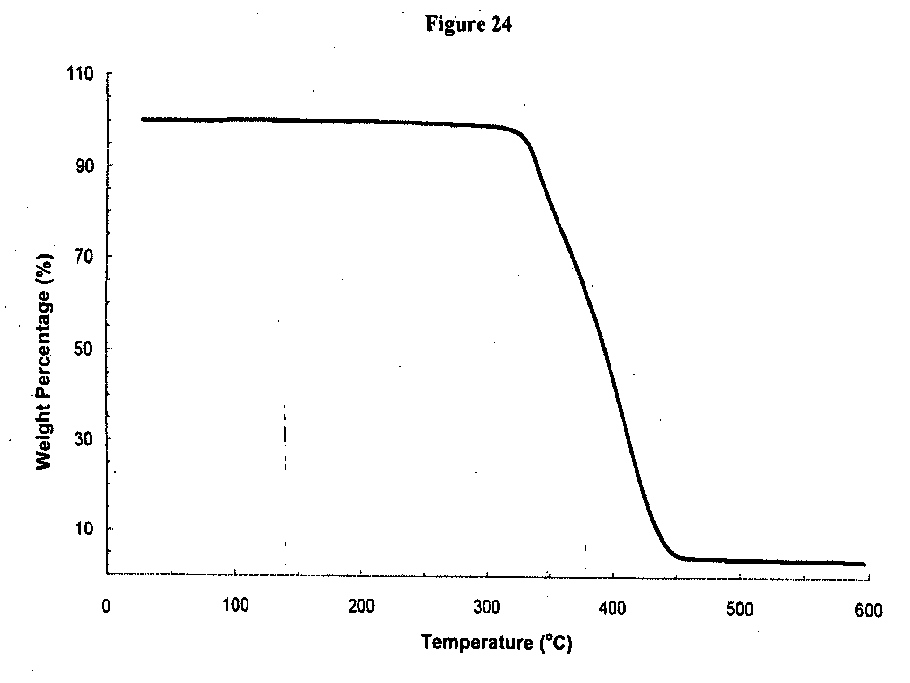

The TGA curve of Example 4 measured in air is shown in Figure 24. The decomposition temperature of Example 4 in air was determined by TGA. The 1% weight loss of Example 4 in air was recorded at 307 °C and the 5% weight loss of Example 4 in air was recorded at 333 °C.

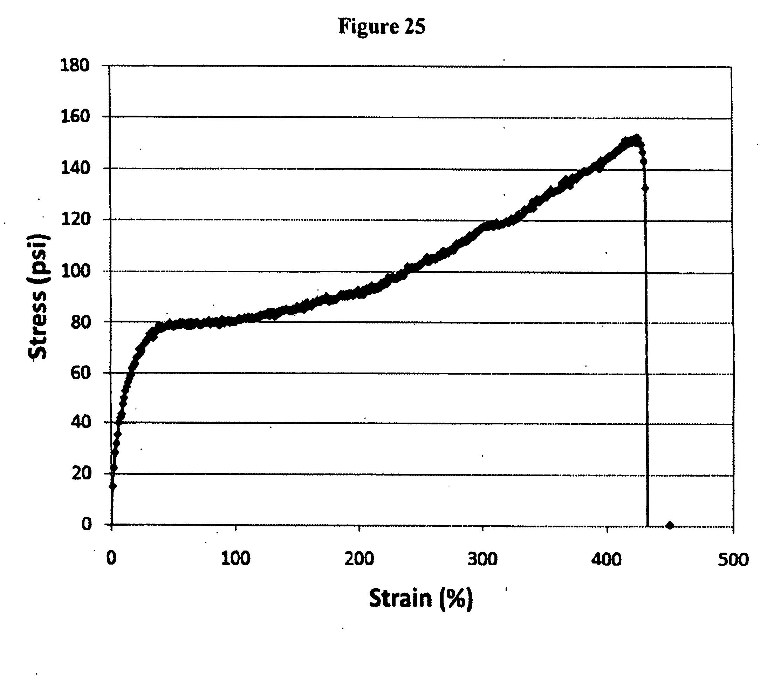

The tensile test results of Example 4 are shown in Figure 25. The tensile strength of Example 4 was measured by a tensile test. Example 4 was stiff but yielded. As shown in Figure 25, the elongation at break of Example 4 was found to be about 425% with a maximum tensile strength of about 152 psi. The modulus of Example 4 was calculated to be about 31.9 kpsi. Stress at 330% elongation of Example 4 was about 33.4 psi.

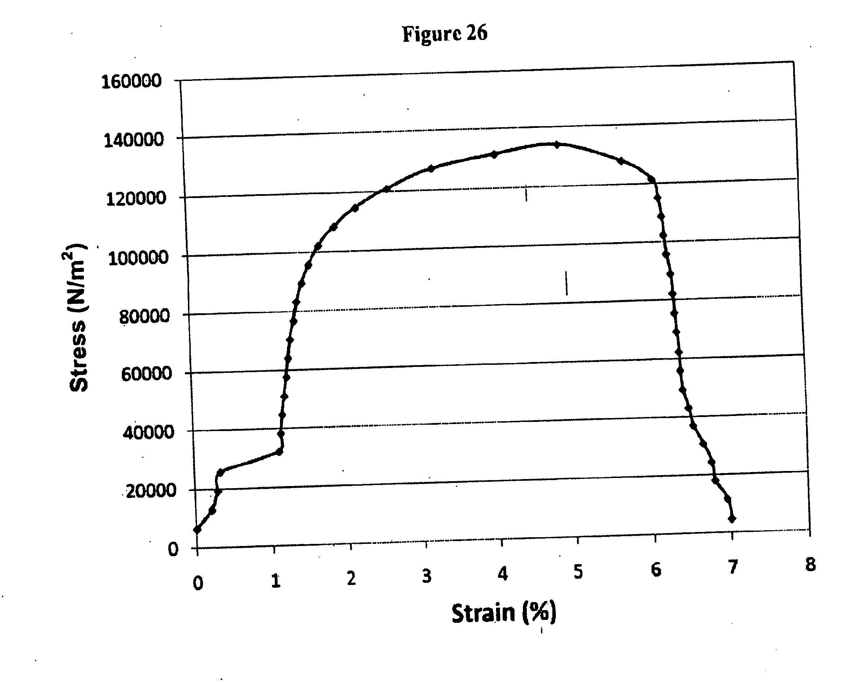

Example 4 was observed to be tacky. The lap test results of Example 4, due to an adhesive failure, are shown in Figure 26. The adhesive energy of Example 4 was found to be about 2,928,000 J/m² with a peak stress of about 134,000 N/m².

### Example 5 - polystyrene-3,4-polyfarnesene-polystyrene

To a first dried three neck reactor under argon atmosphere, a pre-dried 12% solution of β-farnesene in cyclohexane was added. To a second dried three neck reactor under argon atmosphere, a pre-dried solution of 10% styrene in cyclohexane was added. Afterwards, 141.1 g of the styrene solution (i.e., 14.82 g of styrene) was transferred to a dried reactor under argon atmosphere. A mixture of n-butyl lithium (5.84x10⁻⁴ mol) and TMEDA (5.02x 10⁻⁴ mol) was added into the reactor as an initiator, and the reactor was heated at about 50 °C for about 16 hours, until all styrene was consumed, as monitored by GPC. Then, 143.07g of β-farnesene solution (*i.e.*, 15.74 g of β-farnesene) was transferred to the reactor under argon atmosphere. The reaction was allowed to react until completion for about 16 hours, as monitored by GPC. Dichlorosilane coupling agent was then added into the reactor in three equal aliquots, such that the mole ratio of Li to Cl was 1:2. The reaction mixture was allowed react until completion as indicated by a color change from yellow to clear in the reactor. Example 5 was precipitated from the reaction mixture by a 1% solution of *t*-butyl catachol in ethanol. After drying in a vacuum oven at about 60 °C for about 2 hours, Example 5 was kept under vacuum for about 16 hours. Afterwards, Example 5, collected at 28.75 g (yield 96%), was stored in a refrigerator to prevent any crosslinking before characterization.

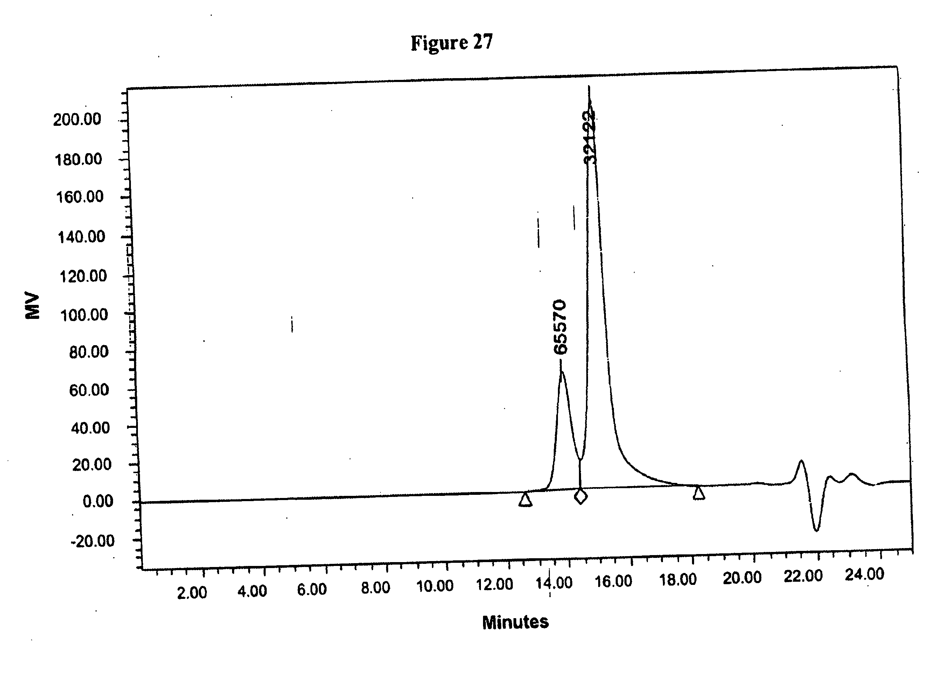

The GPC curve of polystyrene is shown in Figure 27. The progress of synthesizing polystyrene was monitored by GPC. The two peaks in Figure 27 indicated that there were two distinct weight fractions of polystyrene. The Mₙ, M_{w}, M_{z}, M_{z+1}, polydispersity, M_{z}/M_{w}, and M_{z+1}/M_{w} of polystyrene are shown in Table 7. The Mₚ of the first peak in Figure 27 was found to be about 65,570 g/mol. The Mₚ of the second peak in Figure 27 was found, to be about 32,122 g/mol.

**Table 7**

| **Proerties** | **Polystyrene** |
|---|---|
| Mₙ | 27,915 g/mol |
| M_{w} | 30,898 g/mol |
| M_{g} | 32,608 g/mol |
| M_{z+1} | 33,819 g/mol |
| Polydispersity | 1.106849 |
| M_{z}/M_{w} | 1.055361 |
| M_{z+1}/M_{w} | 1.094557 |

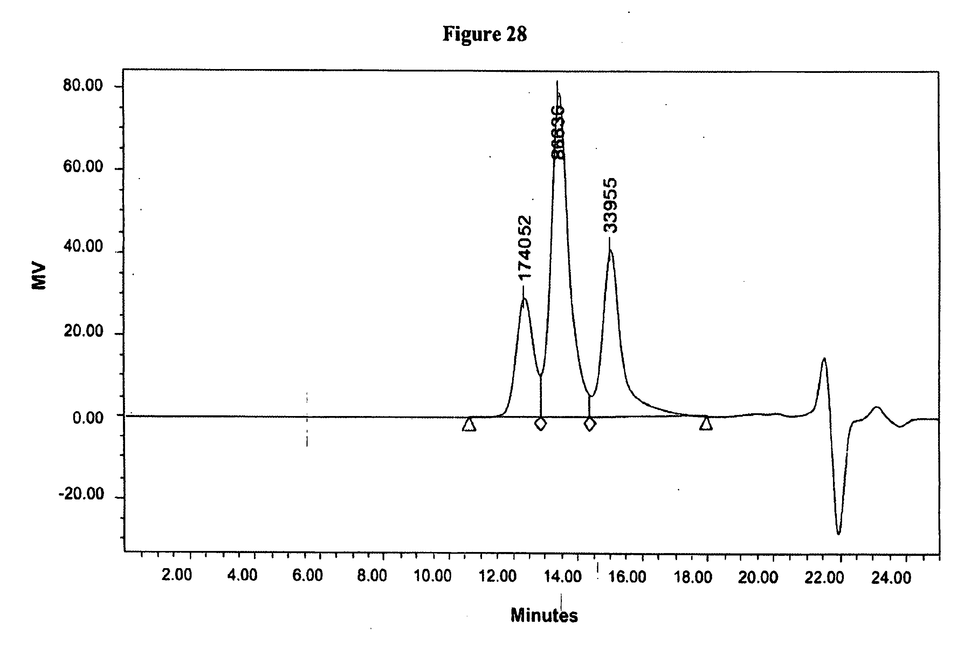

The polystyrene formed then acted as an initiator to initiate the polymerization with β-farnesene to form a polystyrene-3,4-polyfarnesene di-block copolymer. The GPC curve of the di-block copolymer is shown in Figure 28. The progress of the di-block copolymer synthesis was monitored by GPC. The three peaks in Figure 28 indicated that there were three distinct weight fractions in the di-block copolymer reaction solution. The Mₙ, M_{w}, M_{z}, M_{z+1}, polydispersity, M_{z}/M_{w}, and M_{z+1}/M_{w} of the di-block copolymer are shown in Table 8. The Mₚ of the first peak in Figure 28, corresponding to polystyrene-3,4-polyfarnesene-polystyrene, was found to be about 174,052 g/mol. The Mₚ of the second peak in Figure 28, corresponding to the di-block copolymer, was found to be about 86,636 g/mol. The molecular weight of 3,4-polyfarnesene in the di-block copolymer was calculated to be about 54,000 g/mol. The Mₚ of the third peak in Figure 28, corresponding to polystyrene, was found to be about 33,955 g/mol.

**Table 8**

| **Properties** | **Polystyrene-3,4-polyfarnesene Di-block Copolymer** |
|---|---|
| Mₙ | 27,801 g/mol |
| M_{w} | 31,379 g/mol |
| M_{z} | 33,539 g/mol |
| M_{z+1} | 35,033 g/mol |
| Polydispersity | 1.128697 |
| M_{z}/M_{w} | 1.068833 |
| M_{z+1}/M_{w} | 1.116447 |

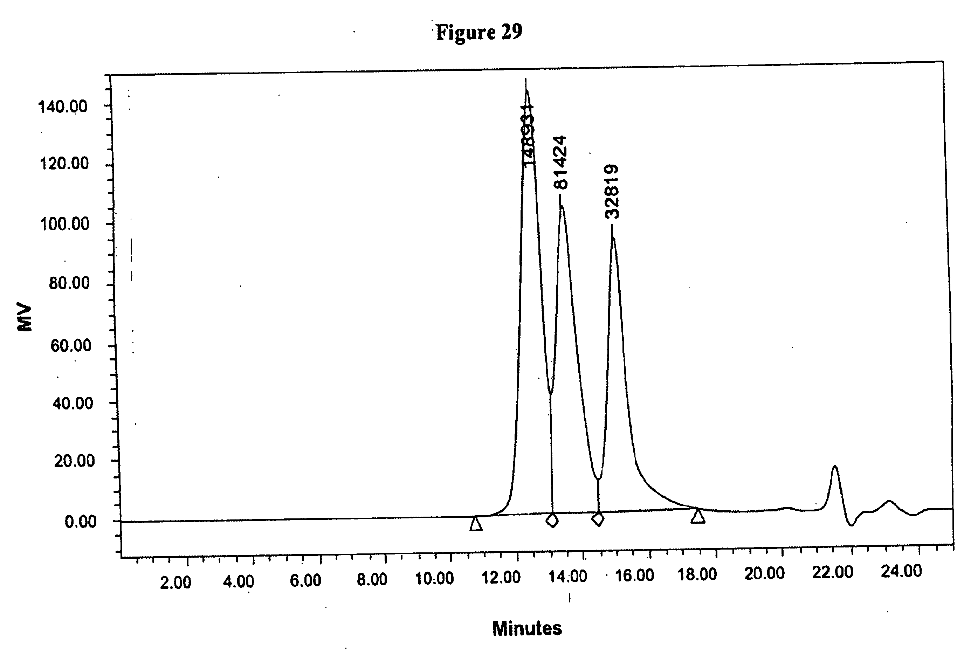

The polystyrene-3,4-polyfarnesene di-block copolymer was further coupled to form Example 5. Figure 29 shows the GPC curve of Example 5. The molecular weight and polydispersity of Example 5 were determined by GPC. The three peaks in Figure 29 indicated that there were three distinct weight fractions for the coupling product formed. The Mₙ, M_{w}, M_{z}, M_{z+1}, polydispersity, M_{z}/M_{w}, and M_{z+1}/M_{w} of Example 5 are shown in Table 9. The Mₚ of the first peak in Figure 29, corresponding to Example 5, was found to be about 148,931 g/mol. Example 5 was obtained at about 33% of the coupling product. The number of farnesene monomer units in Example 5 was calculated to be about 300. The peak molecular weight of the blocks in Example 5 was found to be about 32,000-108,000-32,000 g/mol. The Mₚ of the second peak in Figure 29, corresponding to the polystyrene-3,4-polyfarnesene di-block copolymer, was found to be about 81,424 g/mol. The M_{ρ} of the third peak in Figure 29, corresponding to polystyrene, was found to be about 32,819 g/mol.

**Table 9**

| **Properties** | **Example 4** |
|---|---|
| Mₙ | 28,179 g/mol |
| M_{w} | 30,815 g/mol |
| M_{z} | 32,590 g/mol |
| M_{z+1} | 33,905 g/mol |
| Polydispersity | 1.093554 |
| M_{z}/M_{w} | 1.057606 |
| M_{z+1}/M_{w} | 1.100250 |

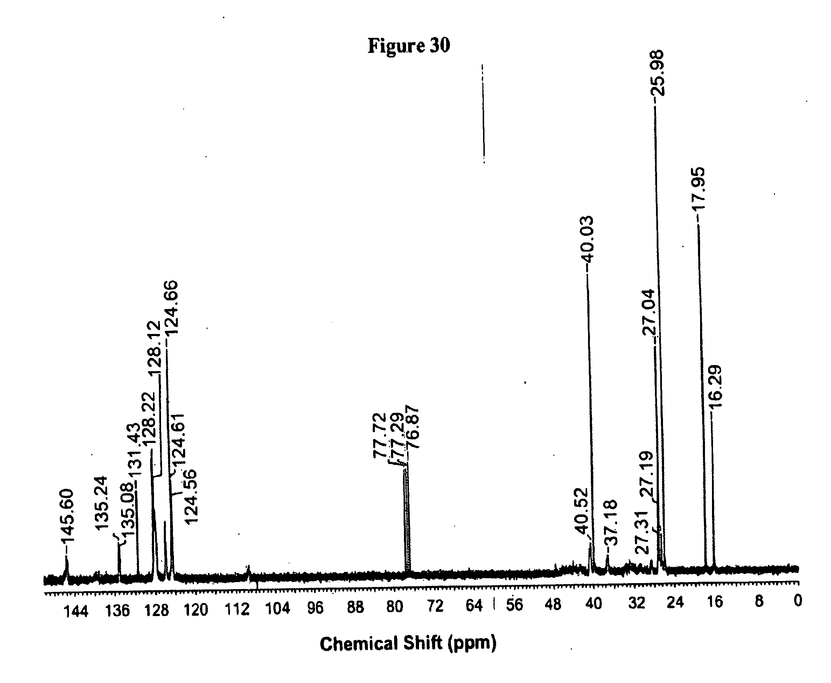

Figure 30 shows the ¹³C NMR spectrum of Example 5. Peaks at 77.72 ppm, 77.29 ppm, and 76.87 ppm were peaks of associated with the deuterated chloroform used for collecting the ¹³C NMR spectrum. Other peaks in Figure 30 were peaks associated with 3,4-polyfarnesene and polystyrene. The characteristic peak identifying 1,4-polyfarnesene at 139.05 ppm was absent in Figure 30, indicating a regular microstructure of Example 5.

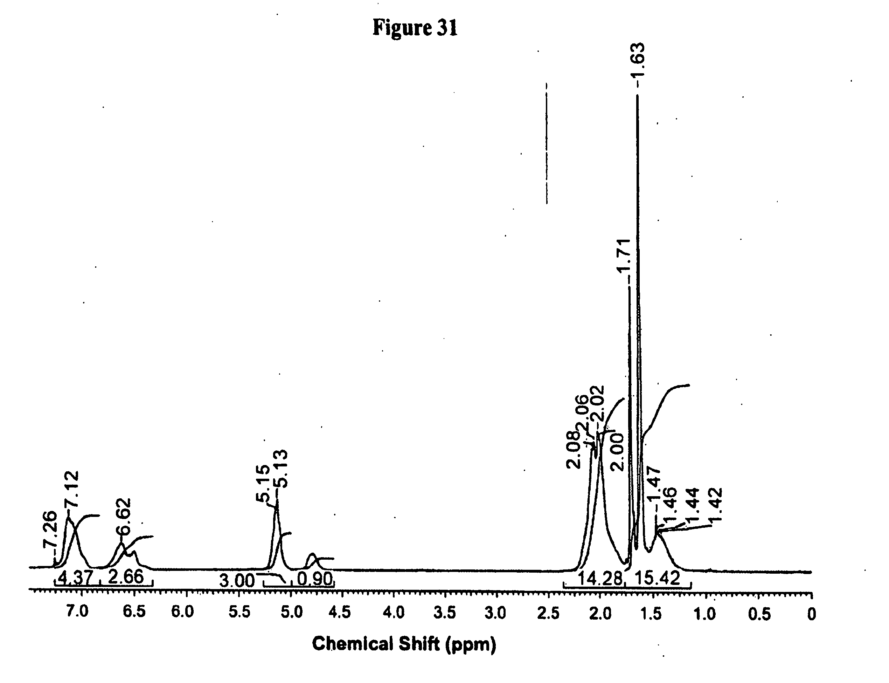

Figure 31 shows the ¹H NMR spectrum of Example 5. Peaks at 4.85 ppm and 4.81 ppm were peaks associated with 3,4-microstructure. Peaks at 5.15 ppm and 5.13 ppm were peaks associated with 1,4- and 3,4-microstructures. Based on the areas under the peaks of Figure 31, about 5% of farnesene units in Example 5 was found to have 1,4-microstructure.

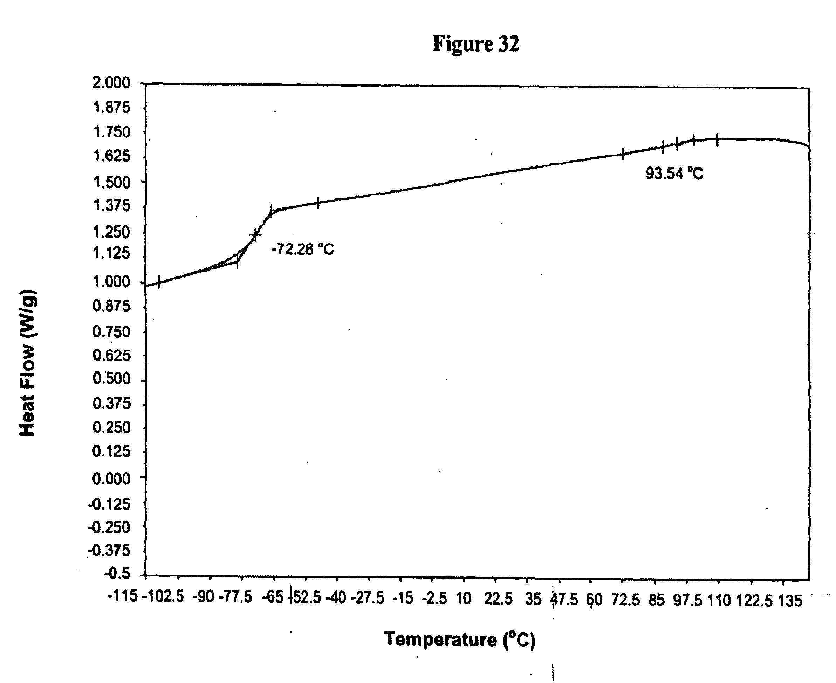

The DSC curve of Example 5 is shown in Figure 32. The thermal characteristics of Example 5 were measured by DSC. The T_{g} of 3,4-polyfarnesene in Example 5 was found to be about -72 °C. The T_{g} of polystyrene in Example 5 was found to be about 94 °C. No other thermal event was detected between -175 °C and 75 °C.

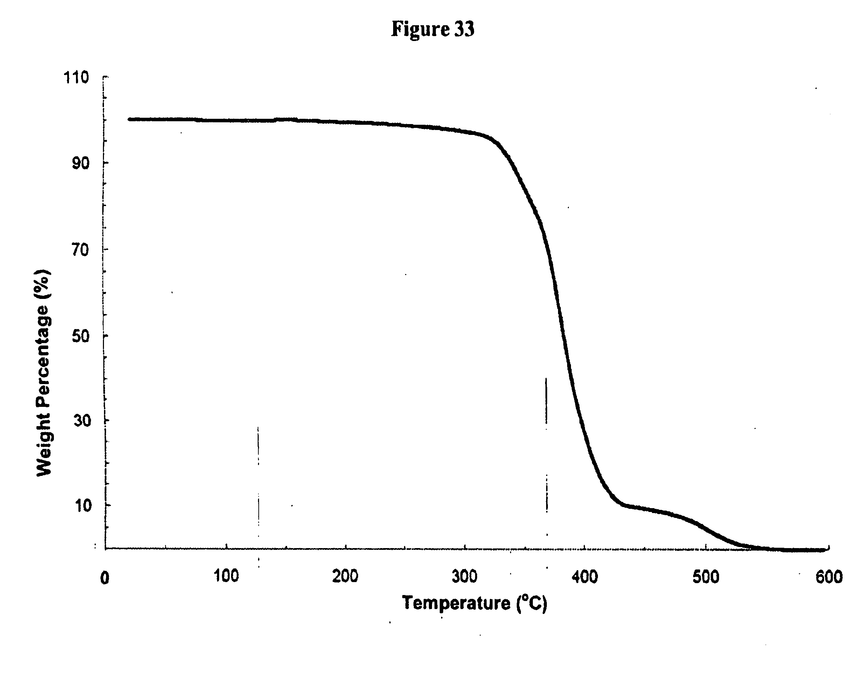

The TGA curve of Example 5 measured in air is shown in Figure 33. The decomposition temperature of Example 5 in air was determined by TGA. The 1% weight loss of Example 5 in air was recorded at 240 °C and the 5% weight loss of Example 5 in air was recorded at 327 °C.

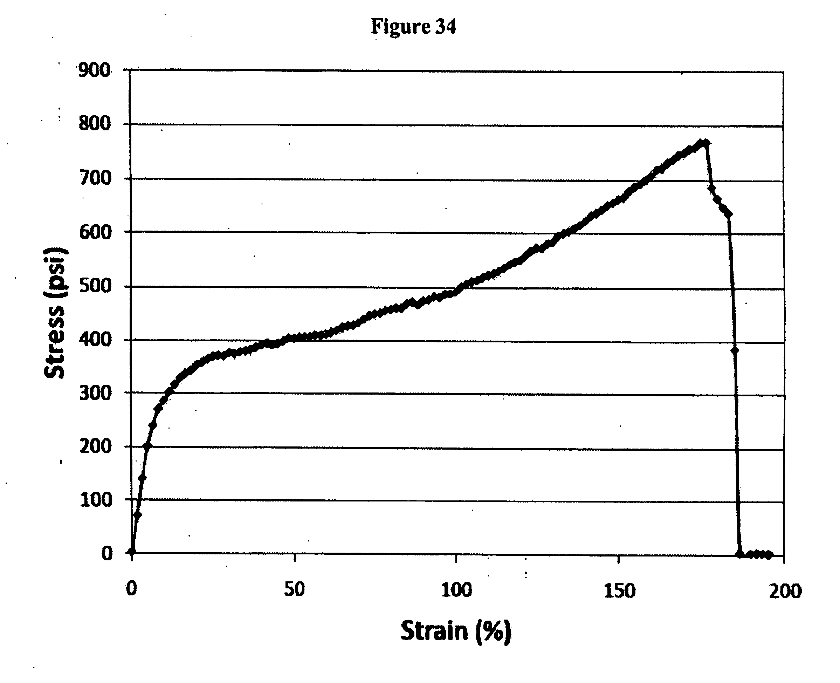

The tensile test results of Example 5 are shown in Figure 34. The tensile strength of Example 5 was measured by a tensile test. Example 5 was stiff but yielded. As shown in Figure 34, the elongation at break of Example 5 was found to be about 175% with a maximum tensile strength of about 768 psi. The modulus of Example 5 was calculated to be about 39.5 kpsi.

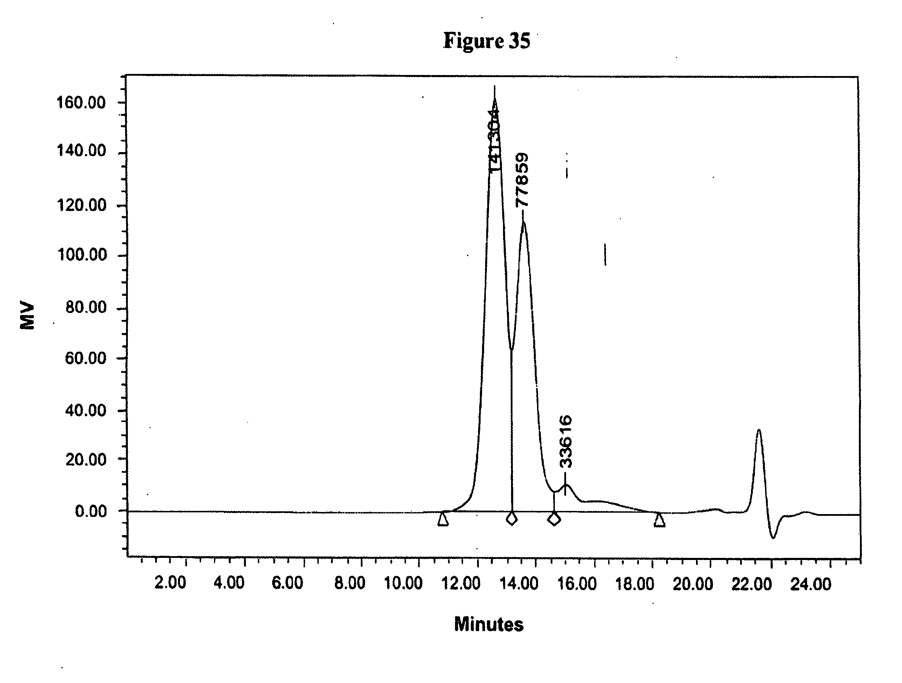

Example 5 was further purified by repeated extraction with solvent hexane 4 times. The GPC curve of the purified Example 5 is shown in Figure 35. The extraction of Example 5 from the coupling product was evaluated by GPC. After the extraction, Example 5, shown as the first peak in Figure 35, was increased to about 60% of the extracted product. The polystyrene-3,4-polyfarnesene di-block copolymer, shown as the second peak in Figure 35, was reduced to about 30% of the extracted product. Polystyrene, shown as the third peak in Figure 35, was reduced to about 10% of the extracted product.

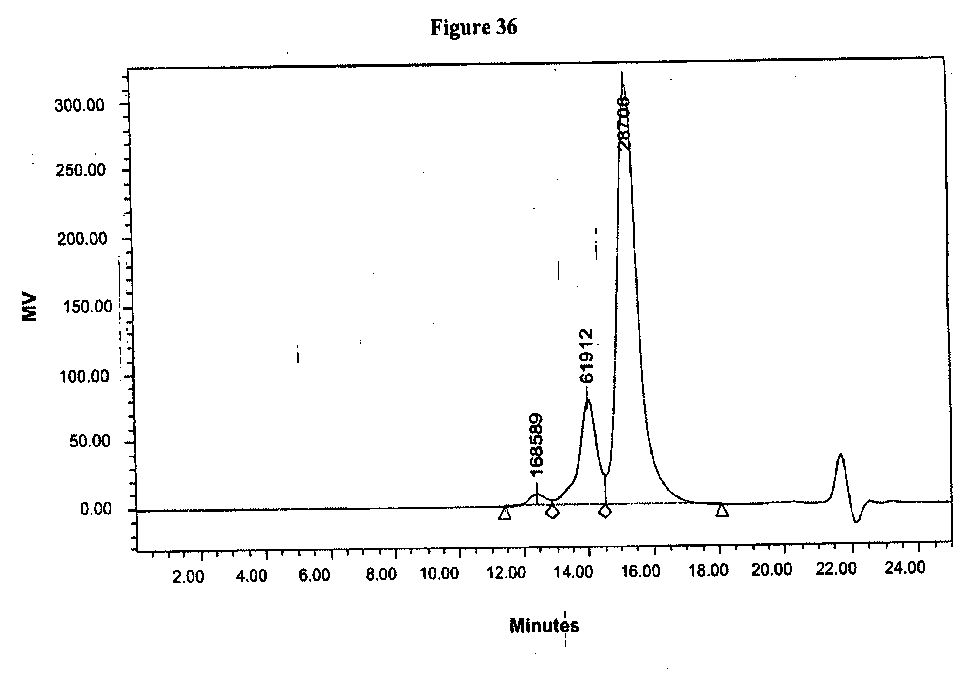

The GPC curve of the extraction solvent hexane is shown in Figure 36. After the extraction, Example 5 existed in very low amount in the extraction solvent, shown as the first peak in Figure 36. A significant amount of the polystyrene-3,4-polyfarnesene di-block copolymer was extracted to the extraction solvent, shown as the second peak in Figure 36. A majority of polystyrene was extracted to the extraction solvent, shown as the third peak in Figure 36.

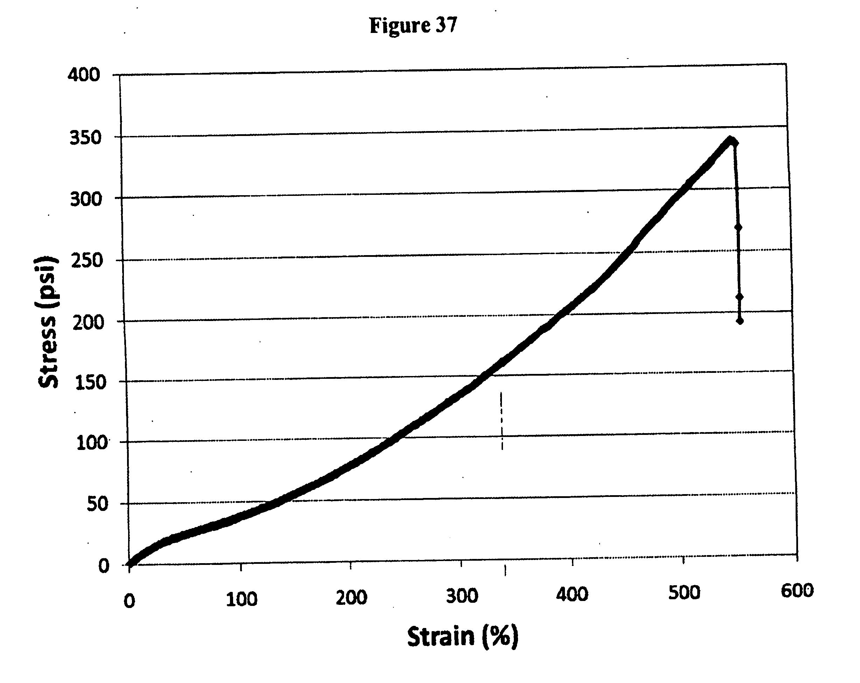

The tensile test results of the purified Example 5 are shown in Figure 37. The tensile strength of the purified Example 5 was measured by a tensile test. Example 5 was soft and readily yielded. As shown in Figure 37, the elongation at break of the purified Example 5 was found to be about 550% with a maximum tensile strength of about 340 psi. The modulus of the purified Example 5 was calculated to be about 65.9 kpsi. Stress at 300% elongation of the purified Example 5 was found to be about 57.1 psi.

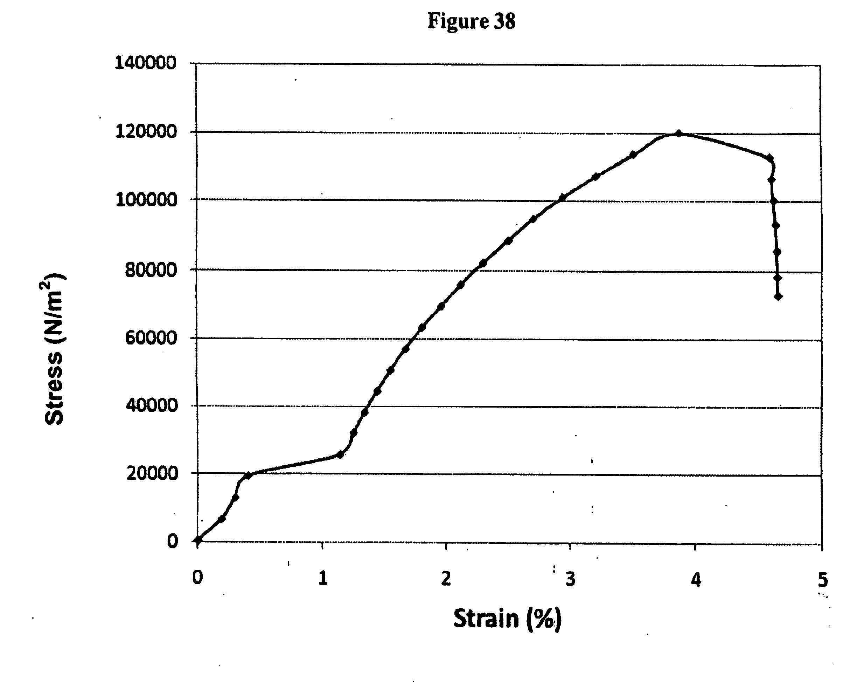

The purified Example 5 was observed to be highly tacky. The lap test results of the purified Example 5, due to an adhesive failure, are shown in Figure 38. The adhesive capability of the purified Example 5 was measured by a lap test. The adhesive energy of the purified Example 5 was found to be about 1,787,000 J/m² with a peak stress of about 120,000 N/m².

### Example 6

Example 6 was formed by the vulcanization of Example 1. To formulate the reaction mixture, 62.7 g of Example 1 was mixed with 3.20 g zinc oxide, 1.25 g stearic acid, 0.94 g Rubbermakers Sulfur MC-98, 0.13 g Accelerator TMTD (tetramethylthiuram disulfide), and 0.63 g Accelerator OBTS (N-oxydiethylene-2-benzothiazole sulfenamide). Zinc oxide, stearic acid, Rubbermakers Sulfur MC-98, Accelerator TMTD, and Accelerator OBTS were obtained from Akrochem Corporation, Akron, OH. The mixture was then placed in a vulcanization mold and degassed at about 140 °C for about 30 minutes. After degassing, the mixture was cured at about 170 °C for about 15 minutes. After de-molding, Example 6, an elastic solid, was collected at 70.4 g (yield 81%).

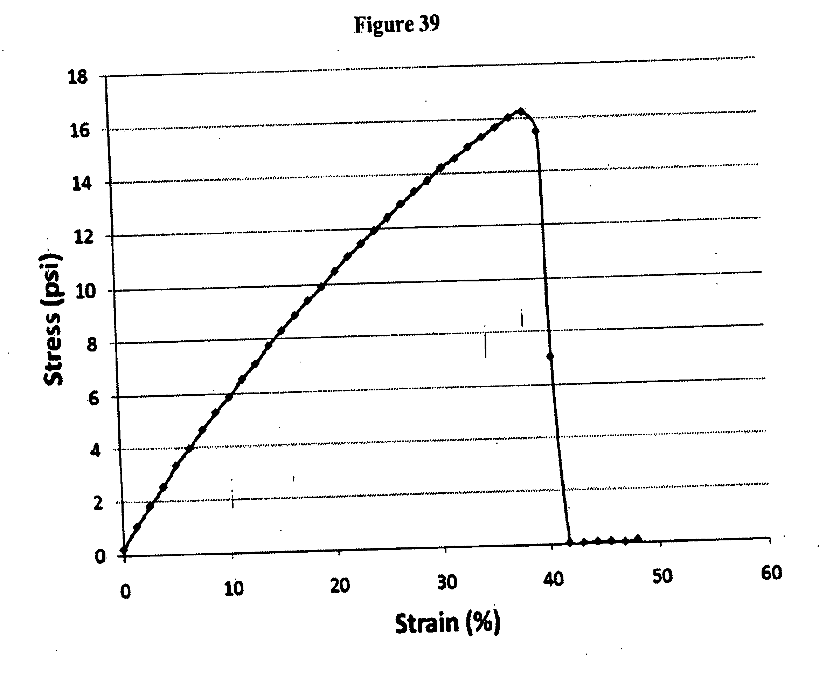

The tensile test results of Example 6 are shown in Figure 39. The tensile strength of Example 6 was measured by a tensile test. As shown in Figure 39, the elongation at break of Example 6 was about 38% with a maximum tensile strength of about 16 psi. The modulus of Example 6 was calculated to be about 58 psi.

### Example 7

Example 7 was formed by the vulcanization of Example 2. Example 7 was synthesized similarly according to the procedure for Example 6 except that Example 1 was replaced by 60.3 g Example 2. The net weight of Example 7 was found to be 68.1 g (yield 78%).

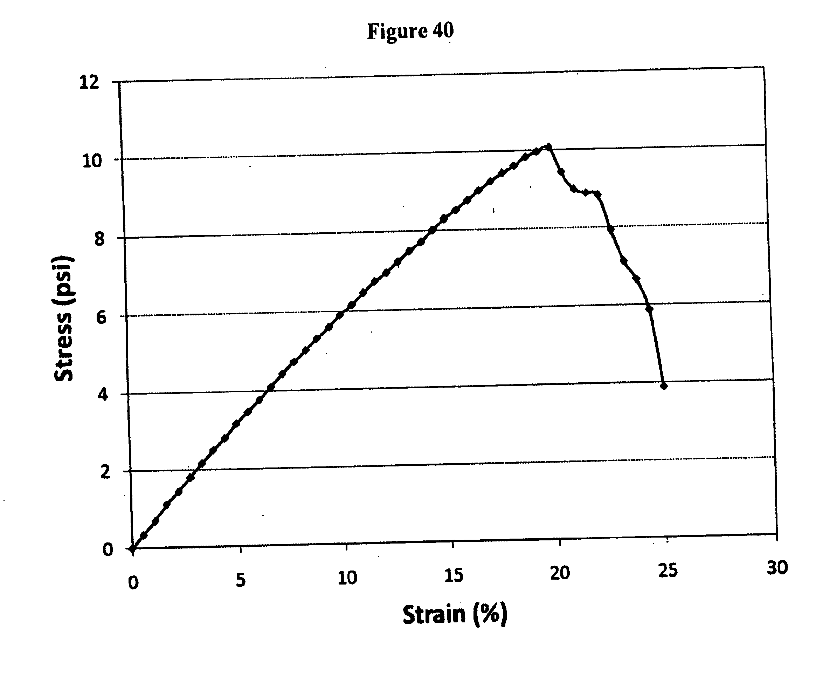

The tensile test results of Example 7 are shown in Figure 40. The tensile strength of Example 7 was measured by a tensile test. As shown in Figure 40, the elongation at break of Example 7 was found to be about 25% with a maximum tensile strength of about 10 psi. The modulus of Example 7 was calculated to be about 66 psi.

While the invention has been described with respect to a limited number of embodiments, the specific features of one embodiment should not be attributed to other embodiments of the invention. No single embodiment is representative of all aspects of the invention. In some embodiments, the compositions or methods may include numerous compounds or steps not mentioned herein. In other embodiments, the compositions or methods do not include, or are substantially free of, any compounds or steps not enumerated herein. Variations and modifications from the described embodiments exist. Finally, any number disclosed herein should be construed to mean approximate, regardless of whether the word "about" or "approximately" is used in describing the number. The appended claims intend to cover all those modifications and variations as falling within the scope of the invention.

## Claims

1. A composition comprising a polyfarnesene and a tackifier, wherein the polyfarnesene has formula (X'): 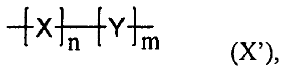 wherein n is an integer from 1 to 100,000; m is an integer from 0 to 100,000; X is derived from a farnesene; and Y is derived from a vinyl monomer, with the proviso that (i) when m is 1 or greater, the mole percent ratio of X to Y is from 1:4 to 100:1, and (ii) when m is 0, the polyfarnesene is a farnesene homopolymer.

2. The composition of claim 1, wherein X has one or more of formulae (I')-(VIII') and stereoisomers thereof: 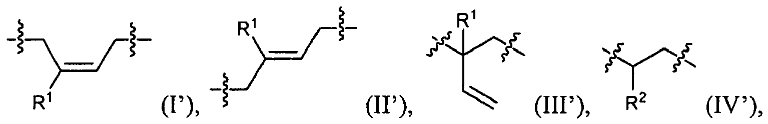 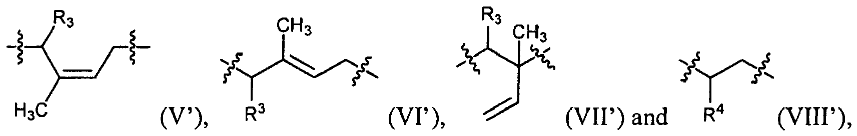 wherein R¹ has formula (XI): 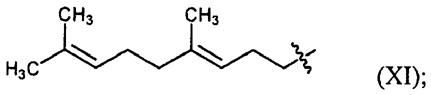 R² has formula (XII): 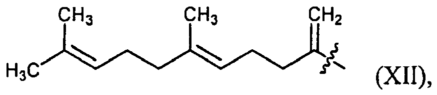 R³ has formula (XIII): 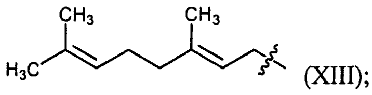 and
R⁴ has formula (XIV):

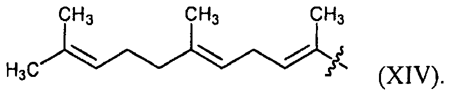

3. The composition of claim 2, wherein the amount of formula (I') is at most 80 wt.%, based on the total weight of the polyfarnesene; and/or
wherein the amount of formula (II') is from 5 wt.% to 99 wt.%, based on the total weight of the polyfarnesene; and/or
wherein the amount of formula (III') is at least 70 wt.%, based on the total weight of the polyfarnesene; and/or
wherein at least a portion of the double bonds in one or more of formulae (I')-(III'), (V')-(VII'), and (XI)-(XIV) and stereoisomers thereof is hydrogenated.

4. The composition of any of claims 1-3, wherein Y has formula (IX') or a stereoisomer thereof: 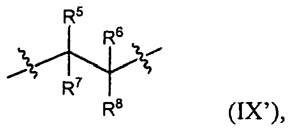 wherein each of R⁵, R⁶, R⁴ and R⁸ is independently H, alkyl, cycloalkyl, aryl, cycloalkenyl, alkynyl, heterocyclyl, alkoxy, aryloxy, carboxy, alkoxycarbonyl, aminocarbonyl, alkylaminocarbonyl, dialkylaminocarbonyl, acyloxy, nitrile or halo; or
wherein R⁵ is aryl, preferably phenyl, and each of R⁶, R⁷ and R⁸ is H.

5. The composition of any of claims 1-4, wherein the sum of m and n is greater than 300; and/or wherein
m is 0 and the polyfarnesene is a farnesene homopolymer, or
m is from 1 to 100,000 and the polyfarnesene is a random farnesene interpolymer, or
m is from 1 to 100,000 and the polyfarnesene is a block farnesene interpolymer, optionally wherein the block farnesene interpolymer comprises one block comprising X and two blocks comprising Y and wherein the block comprising X is between the two blocks comprising Y.

6. The composition of any of claims 1-5, wherein the M_{w} of the polyfarnesene is greater than 60,000 daltons; or
wherein the T_{g} of the polyfarnesene is less than -60 °C.

7. The composition of any of claims 1-6, wherein the tackifier is present in the range from 5 wt.% to 70 wt.%, based on the total weight of the composition; and/or
wherein the composition further comprises an additive selected from the group consisting of plasticizers, oils, waxes, antioxidants, UV stabilizers, colorants or pigments, fillers, flow aids, coupling agents, crosslinking agents, surfactants, solvents and combinations thereof; and/or
wherein the composition further comprises a second polymer, optionally wherein the second polymer is a natural rubber, synthetic rubber, polyacrylate, polymethacrylate, poly-alpha-olefin, ethylene homopolymer, ethylene copolymer, styrene block copolymer or a combination thereof.

8. A composition comprising a polyfarnesene and a tackifier, wherein the polyfarnesene is obtainable by polymerizing a farnesene and an optional vinyl monomer in the presence of a catalyst, with the proviso that when the vinyl monomer is present, the mole percent ratio of the farnesene to the vinyl monomer is from 1:4 to 100:1.

9. The composition of claim 8, wherein the farnesene is an α-farnesene, β-farnesene or a combination thereof; or
wherein the amount of cis-1,4-microstructure in the polyfarnesene is at most 80 wt.%, based on the total weight of the polyfarnesene; or
wherein the vinyl monomer is present and the polyfarnesene is a farnesene interpolymer.

10. The composition of claim 8 or 9, wherein the vinyl monomer is styrene; and/or
wherein the farnesene interpolymer is a block interpolymer; and/or
wherein the farnesene is prepared by a microorganism; and/or
wherein the farnesene is derived from a monosaccharide or a disaccharide; and/or
wherein the catalyst comprises an organolithium reagent, optionally wherein the catalyst further comprises 1,2-bis(dimethylamino)ethane; and/or
wherein the composition is a hot melt adhesive composition or a pressure sensitive adhesive composition.

11. An article comprising a substrate partially or fully coated with the composition of any of claims 1-10.

12. The article of claim 11, wherein the article is a paper product, packaging material, laminated wood panel, kitchen countertop, vehicle, label, disposable diaper, hospital pad, feminine sanitary napkin, surgical drape, tape, case, carton, tray, medical device or bandage, optionally wherein the article is a tape, case, carton, tray, medical device or bandage.

13. A method of adhering a first adherend to a second adherend by using an adhesive comprising the composition of any of claims 1-10.

14. The method of claim 13, wherein each of the first adherend and the second adherend independently comprises metal, wood, paper, plastic, rubber, glass, stone, granite, marble, masonry, porcelain, ceramic, tile, china, concrete, clay, sand, chalk, textile, cloth, non-woven fabric, leather or a combination thereof; and/or
wherein each of the first adherend and the second adherend is independently in the form of sheet, strip, tape, label, tag, web, disk, plate, film or any molded shape.

15. An adhesive composition comprising a rubber and a tackifier, wherein the tackifier comprises a polyfarnesene having formula (X'): 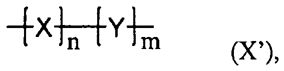 wherein n is an integer from 1 to 100,000; m is an integer from 0 to 100,000; X is derived from a famesene; and Y is derived from a vinyl monomer, with the proviso that (i) when m is 1 or greater, the mole percent ratio of X and Y is from 1:4 to 100:1, and (ii) when m is 0, the polyfarnesene is a farnesene homopolymer.

16. The adhesive composition of claim 15, wherein the polyfarnesene is present in the range from 5 wt.% to 70 wt.%, based on the total weight of the adhesive composition; and/or
wherein the polyfarnesene has a R&B softening point equal to or greater than 80°C, as measured in accordance with ASTM 28-67; and/or
wherein the rubber has a T_{g} less than 20 °C.

## Patentansprüche

1. Zusammensetzung umfassend ein Polyfarnesen und einen Klebrigmacher, wobei das Polyfarnesen Formel (X') aufweist: 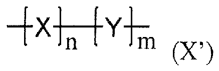 wobei n eine ganze Zahl von größer als 1 bis 100.000 ist; m eine ganze Zahl von 0 bis 100.000 ist; X von einem Farnesen abstammt; und Y von einem Vinylmonomer abstammt, mit der Maßgabe, dass wenn m 1 oder größer ist, das Molprozentverhältnis von X zu Y 1:4 bis 100:1 1 beträgt.

2. Zusammensetzung gemäß Anspruch 1, wobei X eine oder mehrere der Formeln (I')-(VIII') und Stereoisomere davon aufweist: 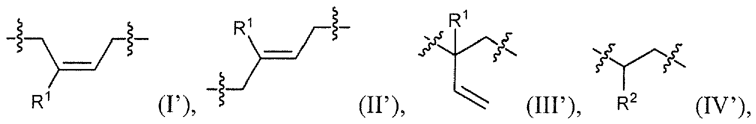 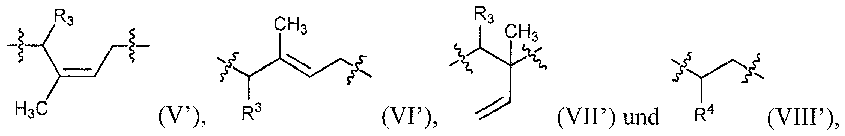 wobei R¹ Formel (XI) aufweist: 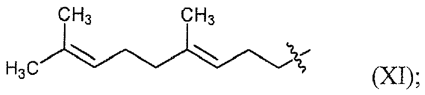 R² Formel (XII) aufweist: 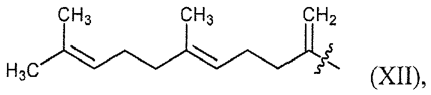 R³ Formel (XIII) aufweist: 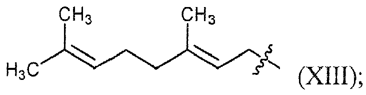 und
R⁴ Formel (XIV) aufweist:

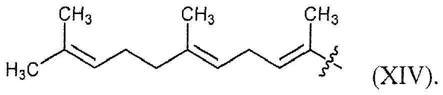

3. Zusammensetzung gemäß Anspruch 2, wobei die Menge der Formel (I') höchstens 80 Gew.% basierend auf dem Gesamtgewicht des Polyfarnesens beträgt; und/oder
wobei die Menge der Formel (II') 5 Gew.% bis 99 Gew.% basierend auf dem Gesamtgewicht des Polyfarnesens beträgt; und/oder
wobei die Menge der Formel (III') mindestens 70 Gew.% basierend auf dem Gesamtgewicht des Polyfarnesens beträgt; und/oder
wobei mindestens ein Teil der Doppelbindungen in einer oder mehreren der Formeln (I')-(III'), (V')-(VII') und (XI)-(XIV) und Stereoisomeren davon hydriert ist.

4. Zusammensetzung gemäß einem der Ansprüche 1-3, wobei Y Formel (IX') oder ein Stereoisomer davon aufweist: 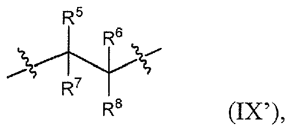 wobei jedes R⁵, R⁶, R⁷ und R⁸ unabhängig voneinander Wasserstoff, Alkyl, Cycloalkyl, Aryl, Cycloalkenyl, Alkinyl, Heterocyclyl, Alkoxy, Aryloxy, Carboxy, Alkoxycarbonyl, Aminocarbonyl, Alkylaminocarbonyl, Dialkylaminocarbonyl, Acyloxy, Nitril oder Halogen ist; oder
wobei R⁵ Aryl, vorzugsweise Phenyl ist, und jedes R⁶, R⁷ und R⁸ Wasserstoff ist.

5. Zusammensetzung gemäß einem der Ansprüche 1-4, wobei die Summe aus m und n größer 300 ist; und/oder wobei
m 0 ist und das Polyfarnesen ein Farnesen-Homopolymer ist, oder
m 1 bis 100.000 ist und das Polyfarnesen ein ungeordnetes Farnesen-Mischpolymer ist, oder
m 1 bis 100.000 ist und das Polyfarnesen ein Farnesen-Blockmischpolymer ist, gegebenenfalls wobei das Farnesen-Blockmischpolymer einen X umfassenden Block und zwei Y umfassende Blöcke umfasst und wobei der X umfassende Block zwischen den beiden Y umfassenden Blöcken liegt.

6. Zusammensetzung gemäß einem der Ansprüche 1-5, wobei das M_{w} des Polyfarnesens größer als 60.000 Dalton ist; oder
wobei die T_{g} des Polyfarnesens kleiner als -60 °C ist.

7. Zusammensetzung gemäß einem der Ansprüche 1-6, wobei der Klebrigmacher in einem Bereich von 5 Gew.% bis 70 Gew.% basierend auf dem Gesamtgewicht der Zusammensetzung vorliegt; und/oder
wobei die Zusammensetzung ferner ein Additiv umfasst, ausgewählt aus Weichmachern, Ölen, Wachsen, Antioxidanzien, UV-Stabilisatoren, Farbstoffen oder Pigmenten, Füllstoffen, Fließmitteln, Kopplungsmitteln, Vernetzungsmitteln, Tensiden, Lösungsmitteln und Kombinationen davon; und/oder
wobei die Zusammensetzung ferner ein zweites Polymer umfasst, gegebenenfalls wobei das zweite Polymer ein Naturkautschuk, Synthesekautschuk, Polyacrylat, Polymethacrylat, Poly-alpha-olefin, Ethylen-Homopolymer, Ethylen-Copolymer, Styrol-Blockcopolymer oder eine Kombination davon ist.

8. Zusammensetzung umfassend ein Polyfarnesen und einen Klebrigmacher, wobei das Polyfarnesen durch Polymerisieren eines Farnesens und eines optionalen Vinylmonomers in Anwesenheit eines Katalysators erhältlich ist, mit der Maßgabe, dass wenn das Vinylmonomer vorhanden ist, das Molprozentverhältnis des Farnesens zu dem Vinylmonomer 1:4 bis 100:1 1 beträgt.

9. Zusammensetzung gemäß Anspruch 8, wobei das Farnesen ein α-Farnesen, β-Farnesen oder eine Kombination davon ist; oder
wobei die Menge der cis-1,4-Mikrostruktur in dem Polyfarnesen höchstens 80 Gew.% basierend auf dem Gesamtgewicht des Polyfarnesens beträgt; oder
wobei das Vinylmonomer vorhanden ist und das Polyfarnesen ein Farnesen-Mischpolymer ist.

10. Zusammensetzung gemäß Anspruch 8 oder 9, wobei das Vinylmonomer Stryol ist; und/oder
wobei das Farnesen-Mischpolymer ein Blockmischpolymer ist; und/oder
wobei das Farnesen durch einen Mikroorganismus hergestellt wird; und/oder
wobei das Farnesen von einem Monosaccharid oder einem Disaccharid abstammt; und/oder
wobei der Katalysator ein Organolithium-Reagenz umfasst, gegebenenfalls wobei der Katalysator ferner 1,2-Bis(dimethylamino)ethan umfasst; und/oder
wobei die Zusammensetzung eine Schmelzklebstoffzusammensetzung oder Haftklebstoffzusammensetzung ist.

11. Gegenstand umfassend einen mit der Zusammensetzung gemäß einem der Ansprüche 1-10 teil- oder vollbeschichteten Träger.

12. Gegenstand gemäß Anspruch 11, wobei der Gegenstand ein Papierprodukt, ein Verpackungsmaterial, eine laminierte Holzplatte, eine Küchenarbeitsplatte, ein Träger, ein Aufkleber, eine Einwegwindel, eine Krankenhaus-Matte, eine Damenbinde, eine OP-Abdeckung, ein Klebeband, ein Behälter, ein Karton, eine Schale, ein Medizinprodukt oder Verband ist, gegebenenfalls wobei der Gegenstand ein Klebeband, ein Behälter, ein Karton, eine Schale, ein Medizinprodukt oder Verband ist.

13. Verfahren zum Verkleben eines ersten zu verklebenden Teils mit einem zweiten zu verklebenden Teil mittels eines Klebstoffs umfassend die Zusammensetzung gemäß einem der Ansprüche 1-10.

14. Verfahren gemäß Anspruch 13, wobei jedes erste zu verklebende Teil und jedes zweite zu verklebende Teil unabhängig voneinander Metall, Holz, Papier, Kunststoff, Gummi, Glas, Stein, Granit, Marmor, Mauerwerk, Porzellan, Keramik, Fliese, Porzellan, Beton, Lehm, Sand, Kalk, Gewebe, Stoff, Vlies, Leder oder eine Kombination davon ist; und/oder
wobei jedes erste zu verklebende Teil und zweite zu verklebende Teil unabhängig voneinander in Form einer Bahn, einer Leiste, eines Bands, eines Etiketts, eines Schilds, einer Fläche, einer Scheibe, einer Platte, eines Films oder jedes anderen Formteils vorliegt.

15. Klebstoffzusammensetzung umfassend einen Kautschuk und einen Klebrigmacher, wobei der Klebrigmacher ein Polyfarnesen der Formel (X') aufweist: 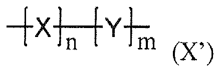 wobei n eine ganze Zahl von größer als 1 bis 100.000 ist; m eine ganze Zahl von 0 bis 100.000 ist; X von einem Farnesen abstammt; und Y von einem Vinylmonomer abstammt, mit der Maßgabe, dass wenn m 1 oder größer ist, das Molprozentverhältnis von X zu Y 1:4 bis 100:1 beträgt.

16. Klebstoffzusammensetzung gemäß Anspruch 15, wobei das Polyfarnesen in einem Bereich von 5 Gew.% bis 70 Gew.% basierend auf dem Gesamtgewicht der Klebstoffzusammensetzung vorliegt; und/oder
wobei das Polyfarnesen einen R&B Schmelzpunkt von 80 °C oder höher gemessen nach ASTM 28-67 aufweist; und/oder
wobei der Kautschuk eine T_{g} von weniger als 20 °C aufweist.

## Revendications

1. Composition comprenant un polyfarnesène et un agent poisseux, où le polyfarneséne a la formule (X'): 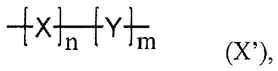 où n est un entier de supérieur à 1 à 100 000; m est un entier de 0 à 100 000; X est dérivé d'un farnesène; et Y est dérivé d'un monomère vinylique, à condition que si m est 1 ou supérieur, le rapport de pour cent molaire entre X et Y est de 1:4 à 100:1.

2. Composition selon la revendication 1, où X a une ou plusieurs des formules (I')-(VIII') et stéréoisomères de celles-ci: 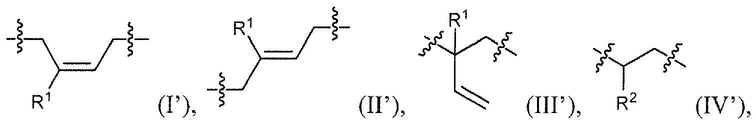 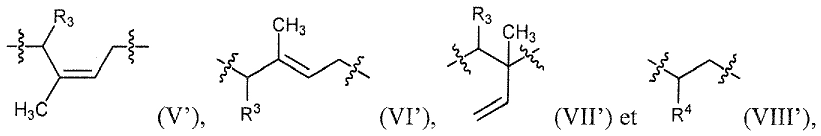 où R¹ a la formule (XI): 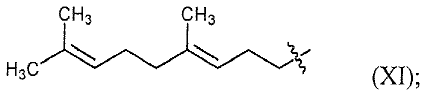 R² a la formule (XII): 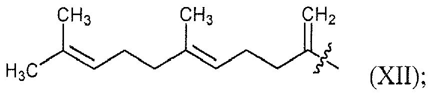 R³ a la formule (XIII): 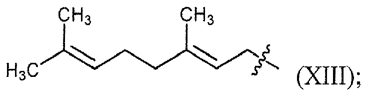 et
R⁴ a la formule (XIV):

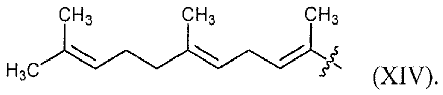

3. Composition selon la revendication 2, où la quantité de la formule (I') est au maximum 80 % en poids, à base du poids total du polyfarnesène; et/ou
où la quantité de la formule (II') est de 5 % en poids à 99 % en poids, à base du poids total du polyfarnesène; et/ou
où la quantité de la formule (III') est au moins 70 % en poids, à base du poids total du polyfarnesène; et/ou
où au moins une partie des liaisons doubles dans une ou plusieurs des formules (I')-(III'), (V')-(VII') et (XI)-(XIV) et stéréoisomères de celles-ci est hydrogénée.

4. Composition selon l'une quelconque des revendications 1-3, où Y a la formule (IX') ou un stéréroisomère de celle-ci: 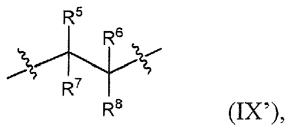 où chacun de R⁵, R⁶, R⁷ et R⁸ est indépendamment H, alkyle, cycloalkyle, aryle, cycloalcényle, alcynyle, hétérocyclyle, alkoxy, aryloxy, carboxy, alkoxycarbonyle, aminocarbonyle, alkylaminocarbonyle, dialkylaminocarbonyle, acyloxy, nitrile ou halo; ou
où R⁵ est aryle, de préférence phényle, et chacun de R⁶, R⁷ et R⁸ est H.

5. Composition selon l'une quelconque des revendications 1-4, où la somme de m et n est supérieure à 300; et/ou où
m est 0 et le polyfarnesène est un homopolymère farnesène, ou
m est de 1 à 100 000 et le polyfarnesène est un interpolymère farnesène aléatoire, ou
m est de 1 à 100 000 et le polyfarnesène est un interpolymère farnesène bloc, éventuellement où l'interpolymère farnesène bloc comprend un bloc comprenant X et deux blocs comprenant Y et où le bloc comprenant X est entre les deux blocs comprenant Y.

6. Composition selon l'une quelconque des revendications 1-5, où la M_{w} du polyfarnesène est supérieure à 60 000 daltons; ou
où la Tᵥ du polyfarnesène est moins de -60 °C.

7. Composition selon l'une quelconque des revendications 1-6, où l'agent poisseux est présent dans la gamme de 5 % en poids à 70 % en poids, à base du poids total de la composition; et/ou
où la composition comprend en outre un additif choisi parmi le groupe consistant en plastifiants, huiles, cires, antioxydants, stabilisateurs UV, colorants ou pigments, charges, adjuvants fluidifiants, agents de couplage, agents de réticulation, surfactants, solvants et des combinaisons de ceux-ci; et/ou
où la composition comprend en outre un deuxième polymère, éventuellement où le deuxième polymère est un caoutchouc naturel, un caoutchouc synthétique, un polyacrylate, un polyméthacrylate, une poly-alpha-oléfine, un homopolymère d'éthylène, un copolymère d'éthylène, un copolymère bloc styrène ou une combinaison de ceux-ci.

8. Composition comprenant un polyfarnesène et un agent poisseux, où le polyfarnesène est susceptible d'être obtenu par la polymérisation d'un farnesène et d'un monomère vinylique optionnel en présence d'un catalyseur, à condition que si le monomère vinylique est présent, le rapport de pour cent molaire entre le farnesène et le monomère vinylique est de 1:4 à 100:1.

9. Composition selon la revendication 8, où le farnesène est un α-farnesène, un β-farnesène ou une combinaison de ceux-ci; ou
où la quantité de cis-1,4-microstructure dans le polyfarnesène est au maximum 80 % en poids, à base du poids total du polyfarnesène; ou
où le monomère vinylique est présent et le polyfarnesène est un interpolymère famesène.

10. Composition selon la revendication 8 ou 9, où le monomère vinylique est styrène; et/ou
où l'interpolymère farnesène est un interpolymère bloc; et/ou
où le farnesène est préparé par un microorganisme; et/ou
où le farnesène est dérivé d'un monosaccharide ou d'un disaccharide; et/ou
où le catalyseur comprend un réactif d'organolithium, éventuellement où le catalyseur comprend en outre 1,2-bis(diméthylamino)éthane; et/ou
où la composition est une composition adhésive thermofusible ou une composition adhésive sensible à la pression.

11. Article comprenant un substrat partiellement ou entièrement recouvert par la composition selon l'une quelconque des revendications 1-10.

12. Article selon la revendication 11, où l'article est un produit de papier, un matériau d'emballage, un panneau en bois laminé, un plan de travail de cuisine, un véhicule, un label, une couche jetable, un surmatelas d'hôpital, une serviette hygiénique pour femme, un drap chirurgical, un ruban, un récipient, un carton, un plateau, un dispositif médical ou bandage, éventuellement où l'article est un ruban, un récipient, un carton, un plateau, un dispositif médical ou bandage.

13. Procédé d'adhérer une première partie adhérée à une deuxième partie adhérée par l'utilisation d'un adhésif comprenant la composition selon l'une quelconque des revendications 1-10.

14. Procédé selon la revendication 13, où chacune de la première partie adhérée et de la deuxième partie adhérée indépendamment comprend métal, bois, papier, plastique, caoutchouc, verre, pierre, granite, marbre, maçonnerie, porcelaine, céramique, tuile, chine, béton, argile, sable, craie, textile, tissu, tissu non-tissé, cuir ou une combinaison de ceux-ci; et/ou
où chacune de la première partie adhérée et de la deuxième partie adhérée est indépendamment sous forme de feuille, bande, ruban, label, étiquette, surface, disque, plaque, film ou une forme moulée quelconque.

15. Composition adhésive comprenant un caoutchouc et un agent poisseux, où l'agent poisseux comprend un polyfarnesène ayant la formule (X'): 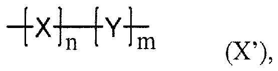 où n est un entier de supérieur à 1 à 100 000; m est un entier de 0 à 100 000; X est dérivé d'un farnesène; et Y est dérivé d'un monomère vinylique, à condition que si m est 1 ou supérieur, le rapport de pour cent molaire entre X et Y est de 1:4 à 100:1.

16. Composition adhésive selon la revendication 15, où le polyfarnesène est présent dans la gamme de 5 % en poids à 70 % en poids, à base du poids total de la composition adhésive; et/ou
où le polyfarnesène a un point de ramollissement B&A égal à ou supérieur à 80 °C, tel que mesuré conformément à ASTM 28-67; et/ou
où le caoutchouc a une Tᵥ de moins de 20 °C.
